# EUROPEAN PATENT APPLICATION

(11) **EP 1 987 777 A2**
(43) Date of publication of application: **05.11.2008**
(21) Application number: 08251072.8
(22) Date of filing: 26.03.2008
(51) Int. Cl.: A61B 17/00

(54) **Systems and methods for treating septal defects**

(30) Priority: 04.05.2007 US 744784
(71) Applicant: Ovalis, Inc., Mountain View, CA 94043 (US)
(72) Inventor: Abbott, Ryan, San Jose, California 95128 (US); Belef, W. Martin, San Jose, California 95125 (US); Doshi, Rajiv, Stanford, California 94309 (US); Ginn, Richard S., Gilroy, California 95020 (US); Jabba, Ronald J., Redwood City, California 94062 (US); Gray, William, Mercer Island, Washington 98040 (US)
(74) Representative: McLeish, Nicholas Alistair Maxwell

(57) **Abstract**

A system for treating a septal defect having an implantable treatment apparatus and devices for delivering the implantable treatment apparatus, devices for controlling delivery of the treatment apparatus and methods for treating a septal defect are provided. The implantable treatment apparatus is preferably implantable through a septal wall or portion thereof. The treatment system can include a flexible elongate body member, a delivery device configured to deliver the implantable apparatus, and a proximal control device for controlling delivery of the implantable apparatus, among others.

## Description

### FIELD OF THE INVENTION

The present invention relates generally to systems and methods for treating internal tissue defects, such as septal defects.

### BACKGROUND OF THE INVENTION

By nature of their location, the treatment of internal tissue defects is inherently difficult. Access to a defect through invasive surgery introduces a high level of risk that can result in serious complications for the subject. Access to the defect remotely with a catheter or equivalent device is less risky, but treatment of the defect itself is made more difficult given the limited physical abilities of the catheter. The difficulty in accessing and treating tissue defects is compounded when the defect is found in or near a vital organ. For instance, a patent foramen ovale ("PFO") is a serious septal defect that can occur between the left and right atria of the heart and a patent ductus arteriosus ("PDA") is an abnormal shunt between the aorta and pulmonary artery.

During development of a fetus in utero, oxygen is transferred from maternal blood to fetal blood through complex interactions between the developing fetal vasculature and the mother's placenta. During this process, blood is not oxygenated within the fetal lungs. In fact, most of the fetus' circulation is shunted away from the lungs through specialized vessels and foramens that are open during fetal life, but typically will close shortly after birth. Occasionally, however, these foramen fail to close and create hemodynamic problems, which, in extreme cases, can prove fatal. During fetal life, an opening called the foramen ovale allows blood to bypass the lungs and pass directly from the right atrium to the left atrium. Thus, blood that is oxygenated via gas exchange with the placenta may travel through the vena cava into the right atrium, through the foramen ovale into the left atrium, and from there into the left ventricle for delivery to the fetal systemic circulation. After birth, with pulmonary circulation established, the increased left atrial blood flow and pressure causes the functional closure of the foramen ovale and, as the heart continues to develop, this closure allows the foramen ovale to grow completely sealed.

In some cases, however, the foramen ovale fails to close entirely. This condition, known as a PFO, can allow blood to continue to shunt between the left and right atria of the heart throughout the adult life of the individual. A PFO can pose serious health risks for the individual, including strokes and migraines. The presence of PFO's have been implicated as a possible contributing factor in the pathogenesis of migraines. Two current hypothesis that link PFO's with migraine include the transit of vasoactive substances or thrombus/emboli from the venous circulation directly into the left atrium without passing through the lungs where they would normally be deactivated or filtered respectively. Other diseases that have been associated with PFO's (and which could benefit from PFO closure) include but are not limited to depression and affective disorders, personality and anxiety disorders, pain, stroke, TIA, dementia, epilepsy, and sleep disorders.

Still other septal defects can occur between the various chambers of the heart, such as atrial-septal defects (ASD's), ventricular-septal defects (VSD's), and the like. To treat these defects as well as PFO's, open heart surgery can be performed to ligate or patch the defect closed. Alternatively, catheter-based procedures have been developed that require introducing umbrella or disc-like devices into the heart. These devices include opposing expandable structures connected by a hub or waist. Generally, in an attempt to close the defect, the device is inserted through the natural opening of the defect and the expandable structures are deployed on either side of the septum to secure the tissue surrounding the defect between the umbrella or disc-like structure.

These devices suffer from numerous shortcomings. For instance, these devices typically involve frame structures that often support membranes, either of which may fail during the life of the subject, thereby introducing the risk that the defect may reopen or that portions of the device could be released within the subject's heart. These devices can fail to form a perfect seal of the septal defect, allowing blood to continue to shunt through the defect. Also, the size and expansive nature of these devices makes safe withdrawal from the subject difficult in instances where withdrawal becomes necessary. The presence of these devices within the heart typically requires the subject to use anti-coagulant drugs for prolonged periods of time, thereby introducing additional health risks to the subject. Furthermore, these devices can come into contact with other portions of the heart tissue and cause undesirable side effects such as an arrhythmia, local tissue damage, and perforation.

Accordingly, improved devices, systems and methods for treating and closing internal tissue defects within the heart are needed.

### SUMMARY

Improved devices and systems for treating internal tissue defects, such as septal defects and the like, are provided herein by the way of exemplary embodiments. These embodiments are examples only and are not intended to limit the invention. Generally, these embodiments include devices for controlling a medical system remotely, devices for improved interaction with the septal wall and improved operation while within a patient.

Other systems, methods, features and advantages of the invention will be or will become apparent to one with skill in the art upon examination of the following figures and detailed description. It is intended that all such additional systems, methods, features and advantages be included within this description, be within the scope of the invention, and be protected by the accompanying claims. It is also intended that the invention is not limited to require the details of the example embodiments.

### BRIEF DESCRIPTION OF THE FIGURES

The details of the invention, both as to its structure and operation, may be gleaned in part by study of the accompanying figures, in which like reference numerals refer to like parts. The components in the figures are not necessarily to scale, emphasis instead being placed upon illustrating the principles of the invention. Moreover, all illustrations are intended to convey concepts, where relative sizes, shapes and other detailed attributes may be illustrated schematically rather than literally or precisely.
**FIG. 1** is a block diagram depicting an exemplary embodiment of a treatment system.
**FIG. 2A** is an exterior/interior view of the right atrium depicting an example human heart.
**FIGs. 2B-2C** are enlarged views of an example atrial septal wall.
**FIG. 2D** is a cross-sectional view taken along line 2D-2D of FIGs. 2B-2C depicting another example septal wall.
**FIG. 3** is a block diagram depicting an exemplary embodiment of an implantable treatment device.
**FIG. 4A** is a perspective view depicting another exemplary embodiment of an implantable treatment device.
**FIG. 4B** is a perspective view depicting an exemplary embodiment of several coiled segments of an implantable treatment device.
**FIG. 4C** depicts a side view of the embodiment of the implantable treatment device taken along direction 330 of FIG. 4A.
**FIG. 4D** is a schematic view depicting another exemplary embodiment of the implantable treatment device as viewed from direction 329 of FIG. 4C.
**FIG. 4E** is cross-sectional view depicting the exemplary embodiment of the implantable treatment device depicted in FIG. 4A implanted within an example heart.
**FIGs. 4F-G** are cross-sectional views of additional exemplary embodiments of the treatment system with a delivery device.
**FIGs. 5A-E** are perspective views depicting additional exemplary embodiments of the central portion the implantable treatment device.
**FIGs. 6A-I** are perspective views depicting additional exemplary embodiments of either the first and/or the second end portions of the implantable treatment device.
**FIG. 7A-C**, **8** and **9A-C** are perspective views depicting additional exemplary embodiments of the implantable treatment device.
**FIG. 10A** is a flow diagram depicting one exemplary method of manufacturing another exemplary embodiment of the implantable treatment device.
**FIG. 10B** is a perspective view of an exemplary embodiment of a body shaping device.
**FIGs. 11A-C** are perspective views depicting additional exemplary embodiments of an implantable treatment device.
**FIG. 12** depicts another exemplary embodiment of the treatment system within a heart.
**FIG. 13** is a block diagram depicting an exemplary embodiment of a delivery device.
**FIG. 14A** is a perspective view depicting another exemplary embodiment of the treatment system.
**FIG. 14B** is a cross-sectional view depicting another exemplary embodiment of the delivery device.
**FIGs. 14C-F** are perspective views depicting a portion of the septal wall and an additional exemplary embodiment of the treatment system.
**FIGs. 15A-D** are perspective views depicting additional exemplary embodiments of the delivery device.
**FIGs. 16A-B** are cross-sectional views depicting additional exemplary embodiments of the treatment system.
**FIG. 16C** is a perspective view depicting the embodiment described with respect to **FIGs. 16A-B** during delivery.
**FIG. 17** is a cross-sectional view depicting an exemplary embodiment of the delivery device taken along line 17-17 of FIG. 14A.
**FIG. 18A** is a cross-sectional view of an exemplary embodiment of a needle member.
**FIGs. 18B-C** are cross-sectional views depicting additional exemplary embodiments of a delivery device.
**FIGs. 19A-B** are cross-sectional views depicting exemplary embodiments of a delivery device and an implantable treatment device.
**FIGs. 20A-B** are schematic views depicting additional exemplary embodiments of a delivery device and an implantable treatment device.
**FIG. 21** is a cross-sectional view depicting another exemplary embodiment of a delivery device taken along lines 21-21 of FIG. 14A.
**FIG. 22** is a block diagram depicting an exemplary embodiment of a stabilization device.
**FIGs. 23A-C** are cross-sectional views depicting additional exemplary embodiments of a stabilization device.
**FIGs. 24A-B** are perspective views depicting additional exemplary embodiments of a stabilization device.
**FIGs. 25A-D** are cross-sectional views depicting additional exemplary embodiments of a stabilization device.
**FIGs. 26A-C** are cross-sectional views depicting additional exemplary embodiments of a stabilization device.
**FIG. 27A** is a perspective view depicting an additional exemplary embodiment of a stabilization device.
**FIG. 27B** is a cross-sectional view depicting another exemplary embodiment of a stabilization device.
**FIGs. 28A-C** are cross-sectional views depicting additional exemplary embodiments of a centering device.
**FIG. 28D** is a schematic view depicting another exemplary embodiment of a centering device within a septal wall.
**FIGs. 29A-C**, **30** and **31** are schematic views depicting additional exemplary embodiments of a centering device.
**FIGs. 32A-B** are cross-sectional views depicting additional exemplary embodiments of a centering device.
**FIG. 32C** is a cross-sectional view depicting another exemplary embodiment of a centering device with an exemplary embodiment of a stabilization device.
**FIG. 32D** is a schematic view depicting another exemplary embodiment of a centering device with an exemplary embodiment of a stabilization device.
**FIG. 33A** is a longitudinal cross-sectional view of an exemplary embodiment of a treatment system.
**FIG. 33B** is a radial cross-sectional view of another exemplary embodiment of a treatment system taken along line 33B-33B of **FIG. 33A****.**
**FIG. 34A** is a longitudinal cross-sectional view of an exemplary embodiment of a treatment system.
**FIG. 34B** is a radial cross-sectional view of another exemplary embodiment of a treatment system taken along line 34B-34B of **FIG. 34A****.**
**FIG. 34C** is a longitudinal cross-sectional view of another exemplary embodiment of a treatment system taken along line 34C-34C of **FIG. 34A****.**
**FIG. 35A** is a longitudinal cross-sectional view of an exemplary embodiment of a treatment system.
FIG. 35B is a radial cross-sectional view of another exemplary embodiment of a treatment system taken along line 35B-35B of FIG. 35A.
FIG. 36A is a longitudinal cross-sectional view of an exemplary embodiment of a treatment system.
FIG. 36B is a radial cross-sectional view of another exemplary embodiment of a treatment system taken along line 36B-36B of FIG. 36A.
FIG. 37A is a longitudinal cross-sectional view of an exemplary embodiment of a treatment system.
**FIG. 37B** is a radial cross-sectional view of an exemplary embodiment of a treatment system taken along line 37B-37B of FIG. 37A.
**FIGs. 38A-E** are cross-sectional views of a septal wall depicting exemplary embodiments of the implantable treatment device.
**FIGs. 39A-B** are flow diagrams depicting an example of a method of treating a septal defect.
**FIG. 40** is a flow diagram depicting another exemplary method of treating a septal defect.
**FIG. 41A** is an exploded perspective view depicting an exemplary embodiment of a proximal control device.
**FIG. 41B** is a top down view depicting another exemplary embodiment of a proximal control device.
FIG. 41C is a cross-sectional view taken along line 41C-41C of FIG. 41B depicting another exemplary embodiment of a proximal control device.
**FIGs. 42A-I** are perspective views depicting additional exemplary embodiments of a proximal control device.
FIG. 43A is a perspective view depicting another exemplary embodiment of a proximal control device.
FIG. 43B is an internal perspective view depicting the exemplary embodiment of a proximal control device depicted in FIG. 43A.
**FIGs. 43C-M** are assorted views depicting additional exemplary embodiments of a proximal control device.
**FIG. 44A** is a perspective view depicting another exemplary embodiment of a treatment system.
**FIG. 44B** is an internal perspective view depicting the exemplary embodiment of a treatment system depicted in **FIG. 44A****.**
**FIG. 44C** is a cross-sectional view depicting another exemplary embodiment of a needle member.
**FIG. 44D** is an internal perspective view depicting the exemplary embodiment of a treatment system depicted in **FIGs. 44A-B.**
**FIGs. 44E-F** are perspective views depicting additional exemplary embodiments of a pusher member.
**FIGs. 45A-B** are a perspective view depicting additional exemplary embodiments of a treatment system.
**FIG. 45C-D** are perspective views depicting additional exemplary embodiments of a lower jaw-like portion of the treatment system.
**FIGs. 45E-G** are top down views depicting additional exemplary embodiments of a treatment system.
**FIG. 45H-I** are radial cross-sectional views taken along lines 45H-45H of **FIG. 45A** depicting additional exemplary embodiments of a delivery device.
**FIG. 46A** is a side view depicting another exemplary embodiment of a treatment system.
**FIGs. 46B-C** are perspective views depicting additional exemplary embodiments of a treatment system.

### DETAILED DESCRIPTION

Described herein are improved devices and methods for treating septal defects. For ease of discussion, the devices and methods will be described with reference to treatment of a PFO. However, it should be understood that the devices and methods can be used in treatment of any type of septal defect including ASD's, VSD's and the like, as well as PDA's or other structural cardiac or vascular defects.

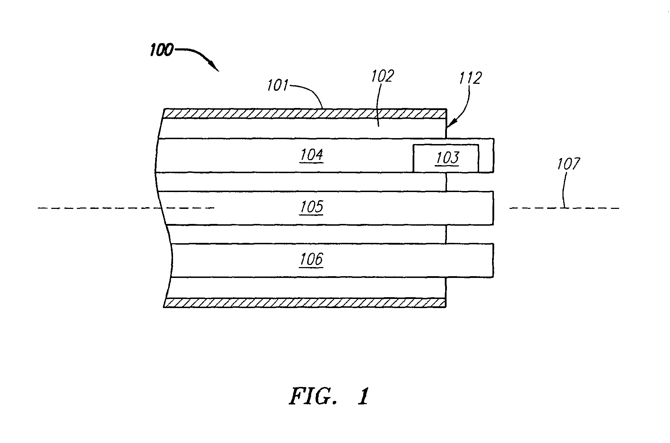

**FIG. 1** is a block diagram depicting a distal portion of an exemplary embodiment of a septal defect treatment system 100 configured to treat, and, preferably close, a PFO. In this embodiment, treatment system 100 includes an elongate body member 101 configured for insertion into the vasculature of a patient (human or animal) having a septal defect. Body member 101 has a longitudinal axis 107, distal end 112 and can include one or more lumens 102, each of which can be configured for achieving multiple functions. Preferably, treatment system 100 includes an implantable device 103 (referred to herein as an "implant") configured to at least partially close a septal defect. Treatment system 100 can include a flexible elongate delivery device 104 configured to house and deliver implant 103. To minimize the width of body member 101, implant 103 can be deformable from the configuration desired after implantation to a configuration having a smaller cross-section for storage and housing within delivery device 104 prior to implantation.

Treatment system 100 can also optionally include a stabilization device 105 for stabilization of body member 101 during delivery of implant 103 and a centering device 106 for facilitating the centering or the otherwise desired positioning of implant 103 for delivery. Although shown here as four separate components, any combination of body member 101, delivery device 104, stabilization device 105 and centering device 106 can be integrated together to reduce the number of components to three, two or one total components in treatment system 100.

The use of a similar treatment systems 100, capable of having body members 101, implants 103, delivery devices 104, stabilization devices 105 and positioning devices 106, are described in detail in co-pending U.S. patent application serial nos. 11/218,794, filed September 1, 2005 and entitled "Suture-based Systems and Methods for Treating Septal Defects" and 11/295,338, filed December 5, 2005 and entitled "Clip-based Systems and Methods for Treating Septal Defects," both of which are fully incorporated by reference herein. It should be noted that any of the types of implantable closure devices, systems for delivering the closure devices and methods for using the same that are described in these incorporated applications can be used with the systems and methods described herein.

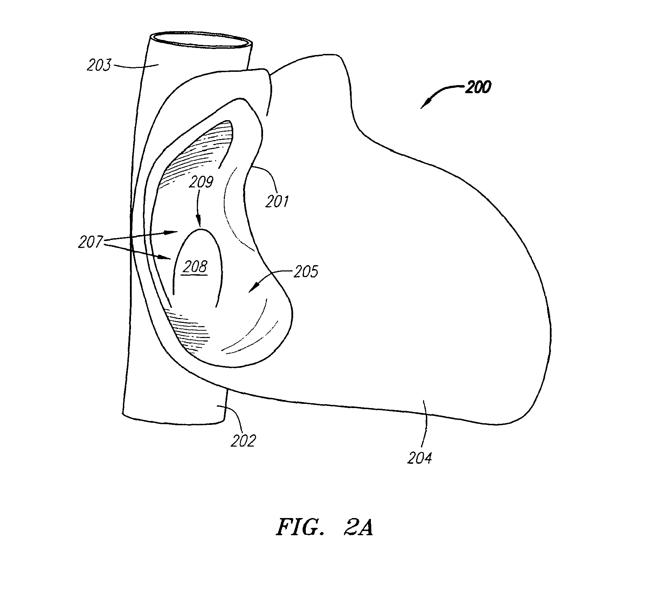

To better understand the many alternative embodiments of treatment system 100, the anatomical structure of an example human heart having a PFO will be described in brief. **FIG. 2A** is an exterior/interior view depicting an example human heart 200 with a portion of the inferior vena cava 202 and the superior vena cava 203 connected thereto. Outer tissue surface 204 of heart 200 is shown along with the interior of right atrium 205 via cutaway portion 201. Depicted within right atrium 205 is septal wall 207, which is placed between right atrium 205 and the left atrium located on the opposite side (not shown). Also depicted is fossa ovalis 208, which is a region of septal wall 207 where the tissue is relatively thinner than the surrounding tissue. PFO region 209 is located near the upper portion beyond the fossa ovalis 208.

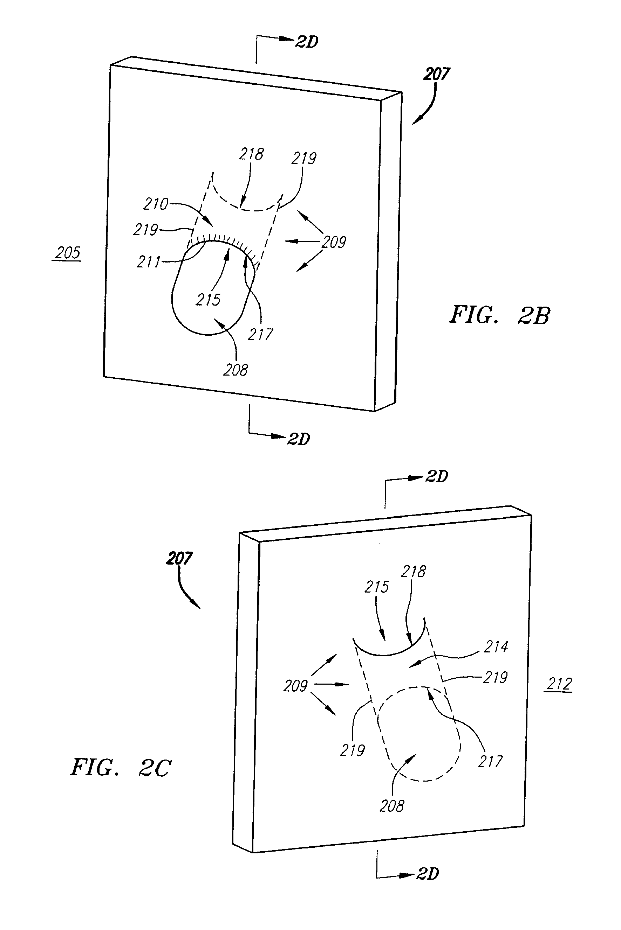

**FIG. 2B** is an enlarged view of septal wall 207 depicting PFO region 209 in more detail as viewed from right atrium 205. PFO region 209 includes septum secundum 210, which is a first flap-like portion of septal wall 207. The edge of this flap above fossa ovalis 208 is referred to as the limbus 211. **FIG. 2C** is also an enlarged perspective view of septal wall 207, instead depicting septal wall 207 as viewed from left atrium 212. Here, PFO region 209 is seen to include septum primum 214, which is a second flap-like portion of septal wall 207. Septum primum 214 and septum secundum 210 partially overlap each other and define a tunnel-like opening 215 between sidewalls 219 (indicated as dashed lines in **FIGs. 2B-C)** that can allow blood to shunt between right atrium 205 and left atrium 212 and is commonly referred to as a PFO.

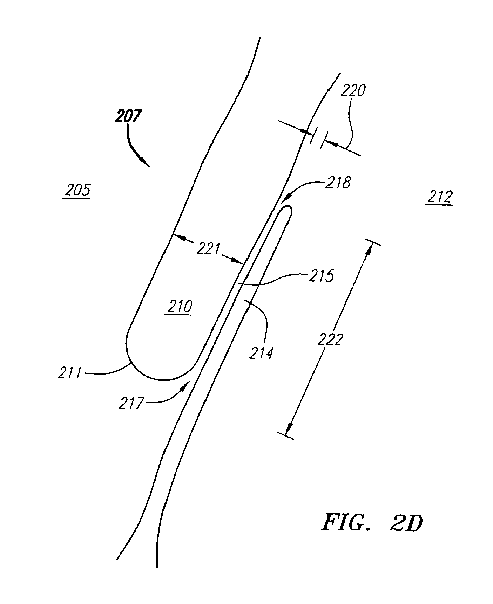

**FIG. 2D** is a cross-sectional view depicting an example PFO region 209 taken along line 2D-2D of **FIGs. 2B-C.** Here, it can be seen that septum secundum 210 is thicker than septum primum 214. Typically, the blood pressure within left atrium 212 is higher than that within right atrium 205 and tunnel 215 remains sealed. However, under some circumstances a valsalva condition can occur where the blood pressure within right atrium 205 becomes higher than the blood pressure within left atrium 212 and blood shunts from right atrium 205 to left atrium 212. Because most typical shunts occur in this manner and for purposes of facilitating the discussion herein, region 217 in **FIG. 2D** will be referred to as PFO entrance 217, and region 218 will be referred to as PFO exit 218.

Many different variations of PFO's can occur. For instance, thickness 220 of septum primum 214, thickness 221 of septum secundum 210, overlap distance 222 and the flexibility and distensibility of both septum primum 214 and septum secundum 210 can all vary. In **FIGs. 2B-C**, PFO entrance 217 and PFO exit 218 are depicted as being relatively the same size with the width of tunnel 215, or the distance between sidewalls 219, remaining relatively constant. However, in some cases PFO entrance 217 can be larger than PFO exit 218, resulting in an tunnel 215 that converges as blood passes through. Conversely, PFO entrance 217 can be smaller than PFO exit 218, resulting in an opening that diverges as blood passes through. Furthermore, multiple PFO exits 218 can be present, with one or more individual tunnels 215 therebetween. Also, in **FIGs. 2B-D,** both septum primum 214 and septum secundum 210 are depicted as relatively planar tissue flaps, but in some cases one or both of septum primum 214 and septum secundum 210 can have folded, non-planar, highly irregular shapes.

As will be described in more detail below, treatment of a PFO preferably includes inserting treatment system 100 into the vasculature of a patient and advancing body member 101 through the vasculature to inferior vena cava 202, from which access to right atrium 205 can be obtained. Once properly positioned within right atrium 205, delivery device 104 can be used to deliver implant 103 to PFO region 209, preferably by inserting implant 103 through septum secundum 210 and primum 214 such that implant 103 lies transverse to tunnel 215 and can at least partially close tunnel 215.

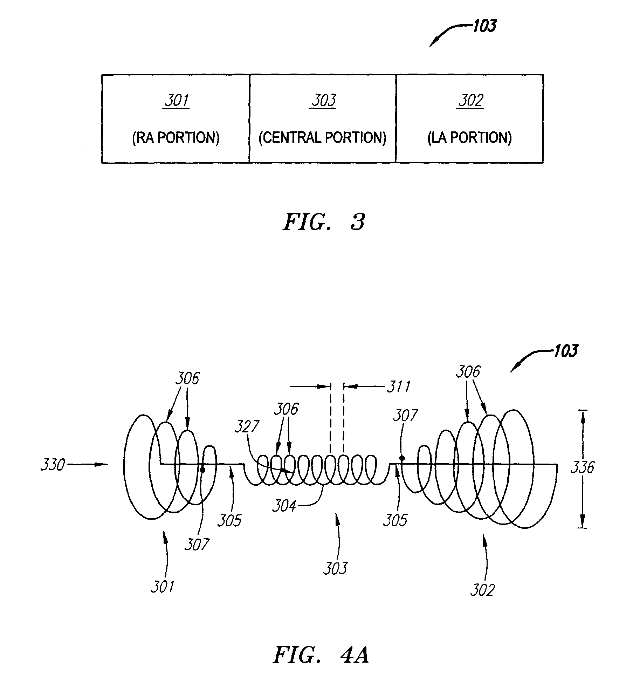

FIG. 3 is a block diagram depicting one exemplary embodiment of implant 103. Implant 103 can be configured in an almost limitless number of different ways, as this block diagram shows. Here, implant 103 includes a first end portion 301, a second end portion 302 and a central portion 303 preferably coupled therebetween. First and second end portions 301-302 are each preferably configured to engage opposing surfaces of septal wall 207. First end portion 301 can be configured to engage the surface of septal wall 207 on the right atrium (RA) side, while second end portion can be configured to engage the surface of septal wall 207 on the left atrium (LA) side. Although end portions 301-302 can be placed anywhere within heart 200 as desired, in order to facilitate the description of implant 103 herein, first end portion 301 will be referred to as RA portion 301 and second end portion will be referred to as LA portion 302.

Central portion 303 is preferably configured to fit within a manmade or surgically created opening in either septum primum 214, septum secundum 210 or both. Central portion 303 is also preferably configured to apply a force adequate to bring end portions 301-302 towards one another when implanted, to be implantable into septal walls 207 of varying thickness and to fit within elongate body member 101, the diameter of which is preferably minimized for ease of insertion within the patient's vasculature.

Implant 103 can be configured in any manner desired to fit the needs of the application. Implant 103 can have any size and shape and can include additional portions not shown in FIG. 3 to achieve a different set of functions. Implant 103 can also be fabricated in any desired manner and from any materials suitable for implantation within the patient including, but not limited to, elastic materials, superelastic materials, shape-memory materials, composite materials, polymeric materials, coatings, drug containing materials, blends with radio-opaque materials and biodegradable materials.

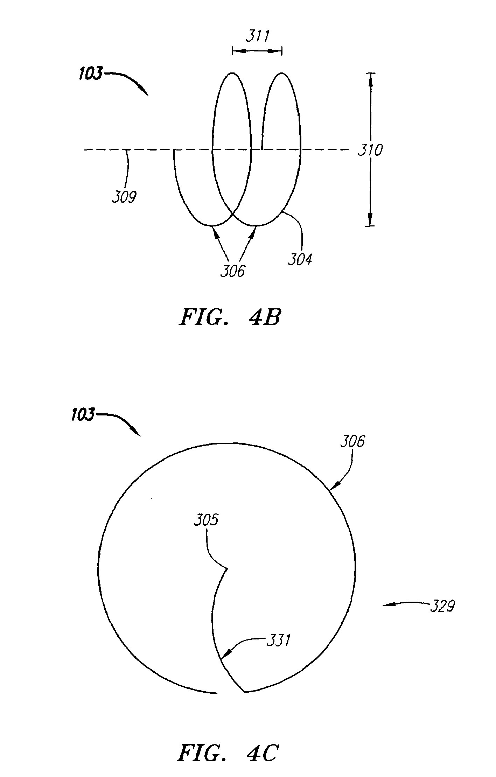

FIG. 4A is a perspective view depicting another exemplary embodiment of implant 103 shown in an "at rest" configuration. In this embodiment, implant 103 is configured in a coil-shaped manner with a wire-like body 304 composed of an elastic material. Wire-like body 304 can have any wire-like cross-sectional shape including, but not limited to circular, elliptical, oval, rounded, arcuate, polygonal and any combination thereof. Each portion 301-303 can be composed of one or more coiled segments 306, with a coiled segment 306 being defined herein as a segment that is curved or otherwise shaped in any manner about one or more axes. Thus, rounded, straight, irregular and polygonal segments are all considered to be coiled. A coiled segment 306 can be curved or otherwise shaped less than 360 degrees about the one or more axes. **FIG. 4B** is a perspective view depicting an exemplary embodiment of several coiled segments 306, which could be used in any of portions 301-303. In this embodiment, each coiled segment 306 is coiled with a constant rate of curvature about the same axis 309. Coiled segments 306 have approximately the same width 310 and are stacked and separated by a distance 311, which will be referred to herein as stacking distance 311.

Referring back to FIG. 4A, implant 103 has an overall width 336. Central portion 303 includes a plurality of coiled segments 306 having substantially the same width 310. Each end portion 301-302 includes a plurality of coiled segments having varied widths or diameters 310. In this case, the width 310 of the outermost coiled segment 306 is the greatest and the widths 310 of each successive coiled segment 306 decreases as one approaches the innermost coiled segment 306. Each end portion 301-302 is coupled with central portion 303 via optional generally straight sections 305. Generally straight sections 305 can prevent blood from shunting between the right and left atria through open interior region 327 of coiled central portion 303, by allowing the adjacent tissue to encroach upon and surround straight section 305. Plugs of bioabsorbable or hydrophilic material may also be provided to minimize such shunting. Generally straight sections 305 can also prevent tissue from getting caught, or hung up, between central portion 303 and RA/LA portions 301/302. Each generally straight sections 305 is not required to be straight and, in fact, can have any non-coiled shape. Central portion 303 can be placed approximately equidistant from end portions 301-302, as depicted here, or central portion 303 can be placed closer to one of end portions 301-302 than the other. Generally straight sections 305 are optional and can be included on only one side of central portion 303 or omitted altogether, in which case the coiled segments 306 of central portion 303 extend directly up to a coiled segment 306 of each end portion 301-302.

The end tips 307 of body 304 are preferably atraumatic so as to minimize injury to cardiac tissue. In this embodiment, end tips 307 are rounded and have a larger diameter than body 304. End tips 307 can also be configured as floppy tips that are curled or coiled and can be flexible or non-flexible. Also, it should be noted that any part of implant 103 can be modified for imaging purposes. For instance, in this embodiment end tips 307 are radio-opaque to increase visibility of implant 103 during imaging. Also, end tips 307 can be configured to facilitate delivery. For instance, in one embodiment end tips 307 can be shaped to minimize the risk of becoming caught on any portion of the delivery device 104. In another embodiment, end tips 307 are configured to interface with the delivery device 104 to allow manipulation of implant 103 before, during or after delivery.

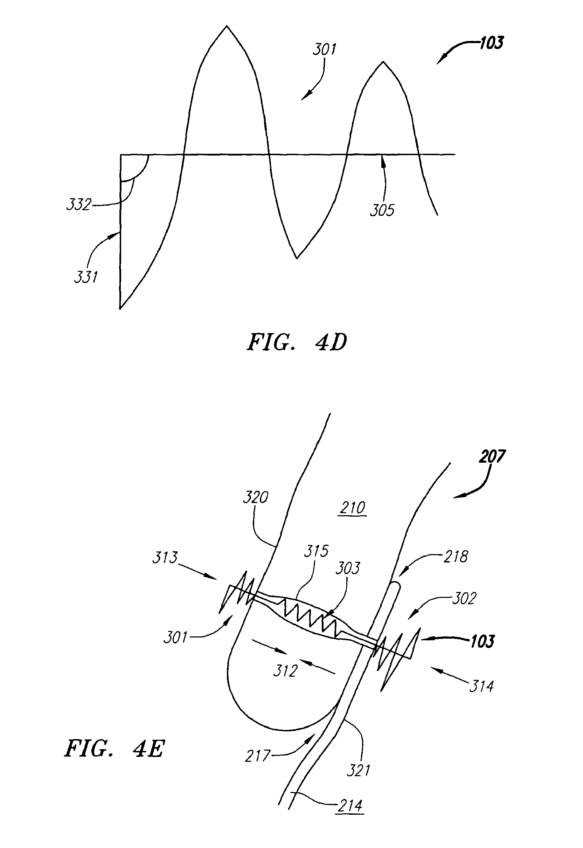

FIG. 4C depicts a side view of the embodiment of implant 303 taken along direction 330 of FIG. 4A. For ease of illustration, FIG. 4C depicts only the outermost coiled segment 306 of RA portion 301, transition section 331 and the generally straight section 305 located between RA portion 301 and central portion 303. Transition section 331 is an optional section of implant 103 that can be straight, curved or any other shape. FIG. 4D depicts RA portion 301, transition section 331 and the generally straight section 305 located between RA portion 301 and central portion 303 as viewed from direction 329 of FIG. 4C. Here, it can be seen that transition section 331 connects to generally straight section 305 at 90 degree angle 332. Angle 332 can be varied as desired, but values of angle 332 approaching 0 degrees or 180 degrees are less preferable due to the increased risk of RA portion 301 (or LA portion 302) being drawn into manmade opening 315, which is described in more detail below.

FIG. 4E is cross-sectional view depicting the exemplary embodiment of implant 103 depicted in FIG. 4A implanted within heart 200 using one exemplary method of implantation. Here, an opening 315 has been surgically created in septum primum 214 and septum secundum 210 and implant 103 has been positioned such that central portion 303 resides within the opening 315. RA portion 301 and LA portion 302 are positioned on opposite sides of septal wall 207 to engage surface 320 of septum secundum 210 and surface 321 of septum primum 214, respectively. Central portion 303 preferably exerts a contractile force 312 to bring portions 301-302 towards one another, which in turn preferably draws septum primum 214 and septum secundum 210 together to at least partially close PFO tunnel 215. Typically, portions 301 and 302 will lie flat against the septa, but are illustrated as compressed conical coils for purposes of clarity. As mentioned above, the widths 310 of coiled segments 306 of RA and LA portions 301-302 get progressively larger from the innermost to the outermost segment 306. If the rate of change of width 310 is large enough to allow coiled segments 306 to pass through each other, then portions 301 and 302 can exert additional closure forces 313 and 314, respectively, which oppose each other and assist central portion 303 in closing PFO tunnel 215.

LA portion 302 and RA portion 301 can each be sized in any manner desired. Preferably, LA portion 302 is configured to have relatively larger coiled segment widths 310, include relatively more coiled segments 306 and exert a closure force over a relatively larger area 314 than RA portion 301. This can be for one of at least two reasons. As will be described in more detail below, preferably, LA portion 302 is deployed in PFO region 209 first and, once in contact with septal wall 207, LA portion 302 is used to help deploy, or pull, portions 303 and 301 from delivery device 104. Also, septum primum 214 is typically thinner than septum secundum 210 and more likely to tear or deform to the extent that LA portion 302 can be pulled though septum primum 214.

Preferably, implant 103 is configured to adjust to septal walls 207 having varying degrees of thickness. Accordingly, central portion 303 preferably has a compressibility sufficient to apply a closure force 312 to thinner septal walls 207 while at the same time having an expandability sufficient to accommodate thicker septal walls 207 without excessive permanent deformation. In one exemplary embodiment, which is for purposes of illustration only and should not be used to limit the scope of the invention in any way, central portion 303 is expandable from 3 to 8 millimeters (mm) without excessive permanent deformation.

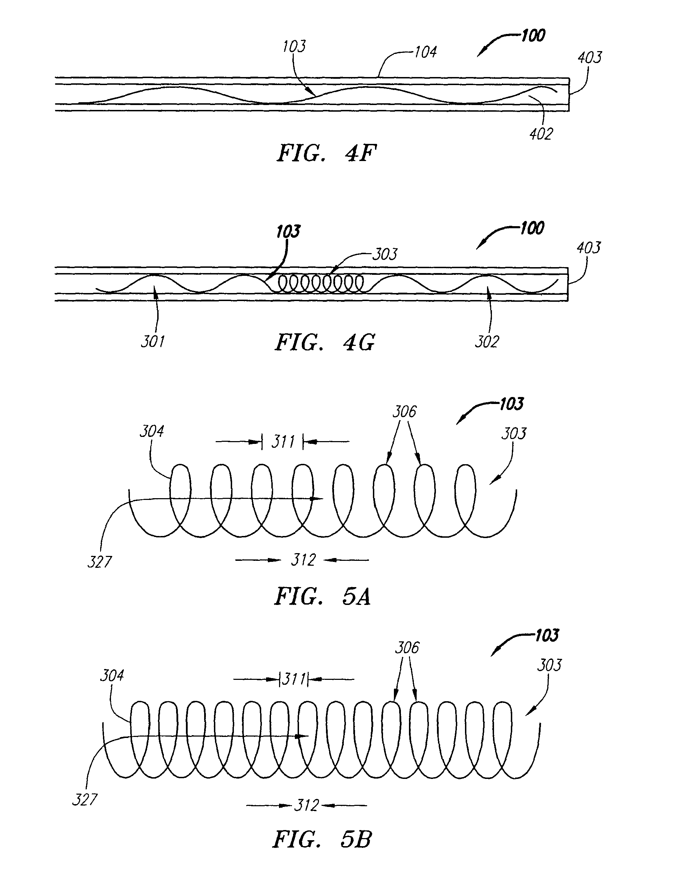

As mentioned above, implant 103 can be deformable between a configuration suited for housing within delivery device 104 and the implanted configuration depicted in **FIG. 4E****.** **FIG. 4F** is a cross-sectional view of an exemplary embodiment of treatment system 100 depicting delivery device 104 having an inner lumen 402 with implant 103 housed therein. Implant 103 is preferably housed within lumen 402 until body member 101 is advanced within the patient into the desired position within heart 200 for implantation, at which time implant 103 is delivered to PFO region 209 through open distal end 403. Here, implant 103 is deformed from the at rest, i.e., unbiased, configuration depicted in **FIG. 4A** into a generally straight configuration where coiled portions 301-303 are mostly unwound into a relatively straight state. This housed configuration significantly reduces the overall anchor width 336 of implant 103 and allows the size of delivery device 104 and, in turn, body member 101 to be minimized.

**FIG. 4G** is a cross-sectional view of another exemplary embodiment of treatment system 100 depicting delivery device 104 with implant 103 in the housed configuration. Here, central portion 303 of implant 103 remains coiled in a state similar to the resting state of **FIG. 4A****,** while RA/LA portions 301/302 are partially unwound into a relatively straight state from the coiled rest state. Preferably, coiled segments 306 of central portion 303 generally have smaller widths 310 than most of the coiled segments 306 of RA/LA portions 301/302. Coiled segments 306 having a smaller width, i.e., more tightly wound coils, can be permanently deformed more easily when unwound and, therefore, by maintaining central portion 303 in the coiled state, the risk of permanent deformation to central portion 303 is reduced. Implant 103 can be deformed in any manner when housed within delivery device 104. For coil-like embodiments of implant 103, this can include deforming any or all of coiled segments 306, to any degree, in any portion 301-303.

To facilitate the deformation of implant 103 between the housed configuration and the implanted configuration depicted in **FIG. 4E****,** implant 103 is preferably composed of an elastic material. Preferably, body 304 is composed of a titanium-nickel alloy such as NITINOL, although any elastic material can be used, including polymers, rubber-like materials, stainless steel, other metal alloys and the like. As one of skill in the art will recognize, the amount of closure force 312-314, the degree of allowable deformation and the like will depend, in part, on the type of material used to form body 304.

**FIGs. 5A-E** are perspective views depicting additional exemplary embodiments of central portion 303 of implant 103. Each of these embodiments can be used with any RA portion 301 and LA portion 302. In **FIG. 5A****,** central portion 303 includes a plurality of coiled segments 306 where the stacking distance 311 between each segment 306 is relatively greater than the embodiment of central portion 303 depicted in **FIG. 5B****.** Generally, a smaller stacking distance 311 will provide a greater closure force 312, if all other implant parameters remain the same. Any stacking distance 311 can be used in central portion 303 as desired, including configurations where there is no gap between each coiled segment 306, i.e., each coiled segment 306 lies flush with any adjacent coiled segment 306. Use of a larger stacking distance 311 that provides for gaps between adjacent coiled segments 306 allows the adjacent septal tissue to grow into the open interior region 327 of the coiled central portion 303, which can provide positional stability to the device and reduce any risk of blood shunting through open region 327.

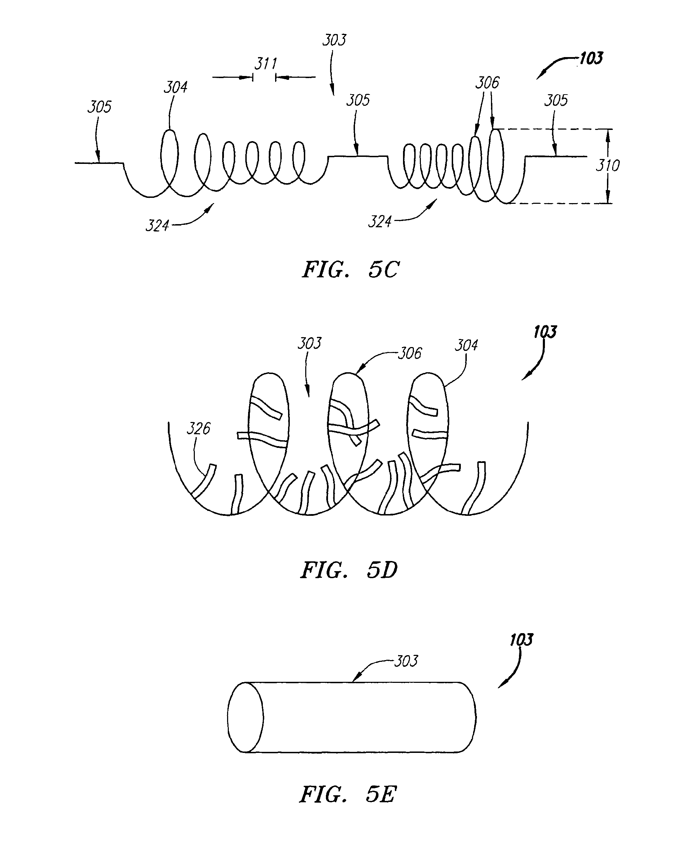

In **FIG. 5C****,** central portion 303 includes a combination of coiled sections 324 and generally straight sections 305. It should be noted that central portion 303 can include any number of one or more coiled sections 324 in any combination with any number of one or more generally straight sections 305. As can be seen here, each coiled section 324 can be configured differently from any other coiled section 324, i.e., each coiled portion can include a different number of coiled segments 306, with different stacking distances 311 and different widths 310, etc.

**FIG. 5D** depicts another exemplary embodiment where blocking material 326 has been coupled with coil body 304. Blocking material 326 preferably reduces any risk of blood shunting through the interior of coiled segments 306, either by blocking blood flow directly or by facilitating the formation of blood clots within open interior region 327. In one exemplary embodiment, blocking material 326 can include multiple DACRON fibers adhesively or mechanically coupled to the outer surface of body 304. In another exemplary embodiment, a polymer or metal plug is placed in open interior region 327 to prevent blood flow. As one of skill in the art will readily recognize, any type of plug, device, material or coating can be used and attached to body 304 in any manner, the numerous combinations of which will not be listed here.

Central portion 303 is not required to include a coiled section 324 and can, in fact, be only a generally straight section 305. Furthermore, central portion 304 is not required to be formed from a wire-like body 304 and can be configured in any manner desired as depicted in the block diagram of **FIG. 3****.** For instance, central portion 303 can be formed from an elastomeric or rubber-like stretchable member, as depicted in **FIG. 5E****.**

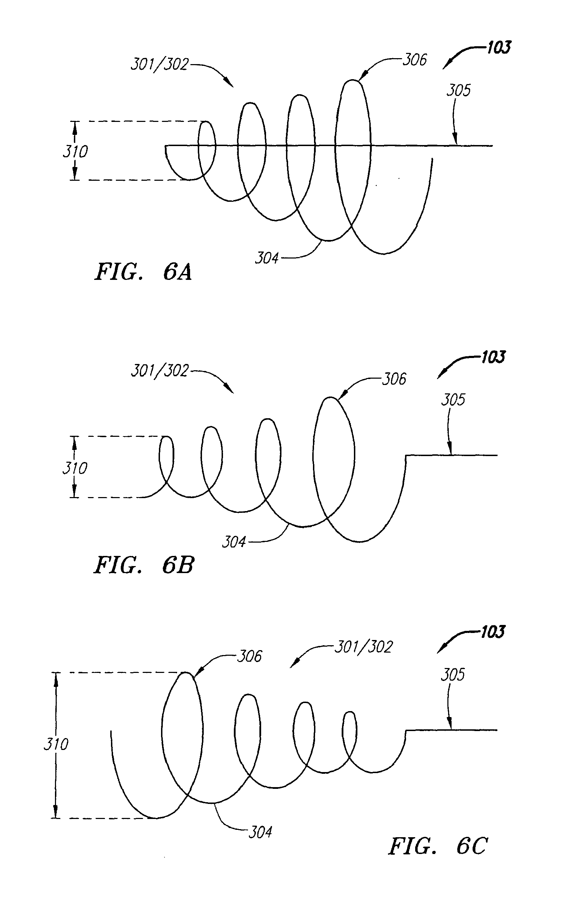

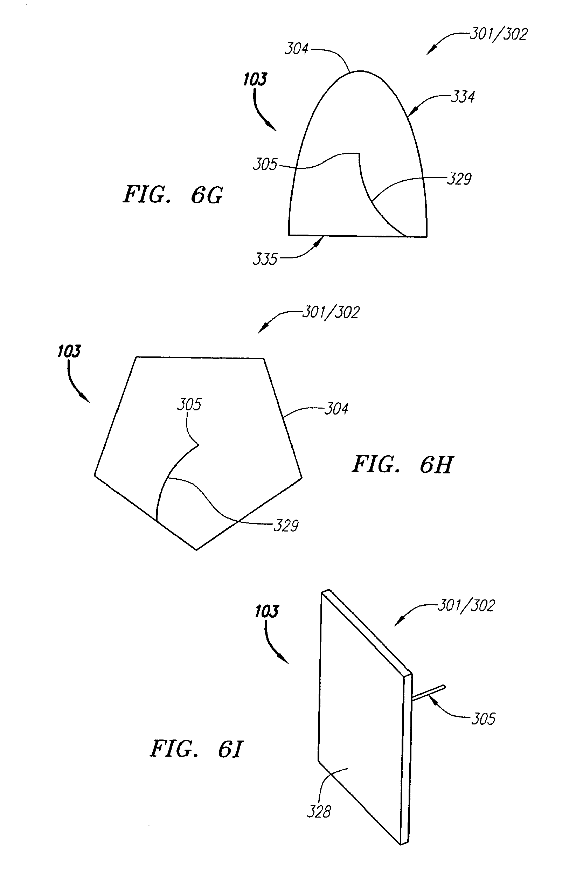

Referring in more detail to RA portion 301 and LA portion 302, **FIGs. 6A-I** are perspective views depicting multiple embodiments exemplary of either RA portion 301 or LA portion 302. Any of the RA/LA portions 301/302 depicted here can be used with any embodiment of central portion 303 described with respect to **FIGs. 5A-E**. For instance, an exemplary embodiment of implant 103 can have RA portion 301 configured in a manner similar to that described with respect to **FIG. 6A****,** central portion 303 configured in a manner similar to that described with respect to **FIG. 5A****,** and LA portion 302 configured in a manner similar to that described with respect to **FIG. 6B****.**

In **FIG. 4A****,** RA/LA portions 301/302 include multiple stacked coiled segments 306 having gradually decreasing widths 310 from the outermost to the innermost segment 306 (outermost being used to reference the segments 306 on the far left and right of **FIG. 4A****).** In **FIG. 6A****,** RA/LA portions 301/302 include multiple coiled segments 306 having gradually increasing widths 310 from the outermost to the innermost segment 306. The embodiment of portions 301-302 described with respect to **FIG. 4A** can be less susceptible to entering opening 315, due to the presence of a relatively larger coiled segment 306 coupled with transition region 305.

In both **FIGs. 4A** and **6A****,** coiled segments 306 of RA/LA portions 301/302 are stacked in an inwards manner, i.e., the outermost segment 306 is coupled with central portion 303 or generally straight section 305, if present (as shown here) and RA/LA portion 301/302 overlaps central portion 303. In **FIGs. 6B-C,** RA/LA portions 301/302 include multiple coiled segments 306 stacked in an outwards manner, i.e., the innermost segment 306 is coupled with central portion 303 or generally straight section 305, if present (as shown here). Generally, stacking segments 306 in an inwards manner will provide greater closure forces than stacking in an outwards manner. In **FIG. 6B****,** RA/LA portions 301/302 include multiple coiled segments 306 having gradually increasing widths 310 from the outermost to the innermost segment 306, while in **FIG. 6C****,** RA/LA portions 301/302 include multiple coiled segments 306 having gradually decreasing widths 310 from the outermost to the innermost segment 306.

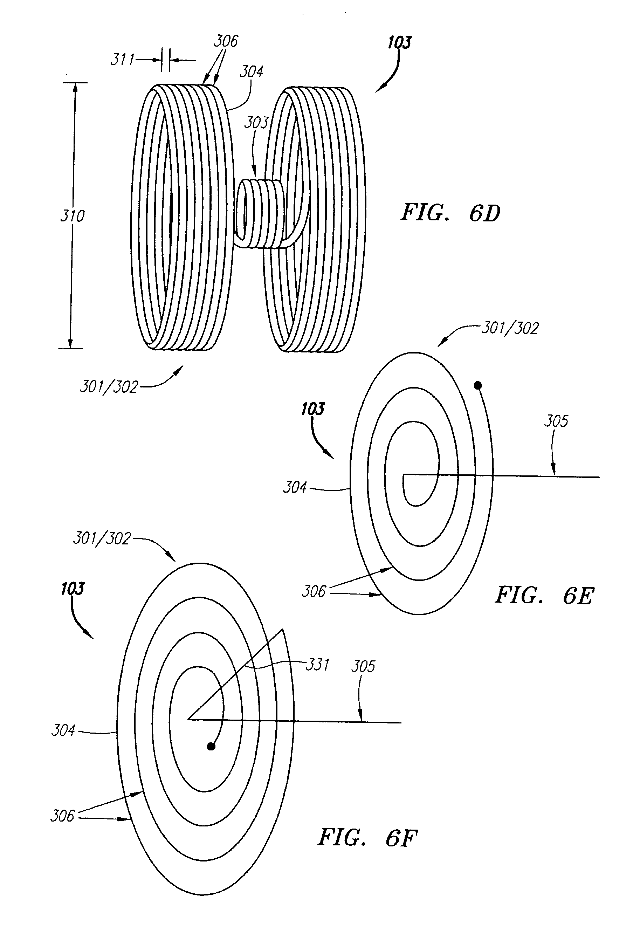

In **FIG. 6D****,** RA/LA portions 301/302 are tightly stacked with a constant width 310 such that no gap exists between adjacent coiled segments 306. This embodiment of RA/LA portions 301/302 exhibits a high resistance to the potential for being pulled into opening 315.

RA/LA portions 301/302 are not required to be implemented in a stacked configuration. For instance, in **FIGs. 6E-F,** RA/LA portions 301/302 each include multiple coiled segments 306 having varying widths 310 arranged in a generally co-planar fashion, i.e., for all segments 306 the stacking distance 311 is close to or equal to zero. In **FIG. 6E****,** the smallest coiled segment 306 is coupled with generally straight section 305, while in **FIG. 6F****,** the largest coiled segment 306 is coupled with generally straight section 305. To lessen the risk of RA/LA portions 301/302 being pulled into opening 315 in the embodiment depicted in **FIG. 6F****,** transition section 331 is preferably positioned on the outside of coiled segments 306 such that, when implanted, coiled segments 306 are located between transition section 331 and septal wall 207.

In the embodiments discussed above, the radius of curvature of the coiled segments 306, present in either RA/LA portions 301/302 or central portion 303, is generally constant or varies at a constant rate, resulting in a circular, spiral or helical appearance when viewed from the side (e.g., direction 330 of **FIG. 4A****).** It should be understood that the radius of curvature can vary at any rate, abruptly or gradual, allowing coiled segments 306 to take any shape or form desired, whether in RA/LA portions 301/302 or central portion 303. For instance, **FIGs. 6G-H** are schematic views depicting additional exemplary embodiments of RA/LA portions 301/302 as viewed from the side. **FIG. 6G** depicts RA/LA portion 301/302 having an elliptical D shape. Here, RA/LA portion 301/302 has an elliptical portion 334 and a generally straight portion 335, which can be placed adjacent to fossa ovalis 208 to lessen the extent to which RA/LA portion 301/302 overlaps fossa ovalis 208 and minimize the risk of piercing or rupturing fossa ovalis 208. **FIG. 6G** depicts another exemplary embodiment of RA/LA portion 301/302 having a generally pentagonal shape.

RA/LA portions 301/302 are not required to include coiled segments 306 and are not required to be formed from a wire-like body 304. As mentioned above, RA/LA portions 301/302 can be configured in any manner desired as depicted in the block diagram of **FIG. 3****.** For instance, RA/LA portions 301/302 can be formed from an elastomeric or rubber-like membrane 328 in an umbrella-like fashion, or a sheet-like fashion as depicted in the exemplary embodiment of **FIG. 6I****.**

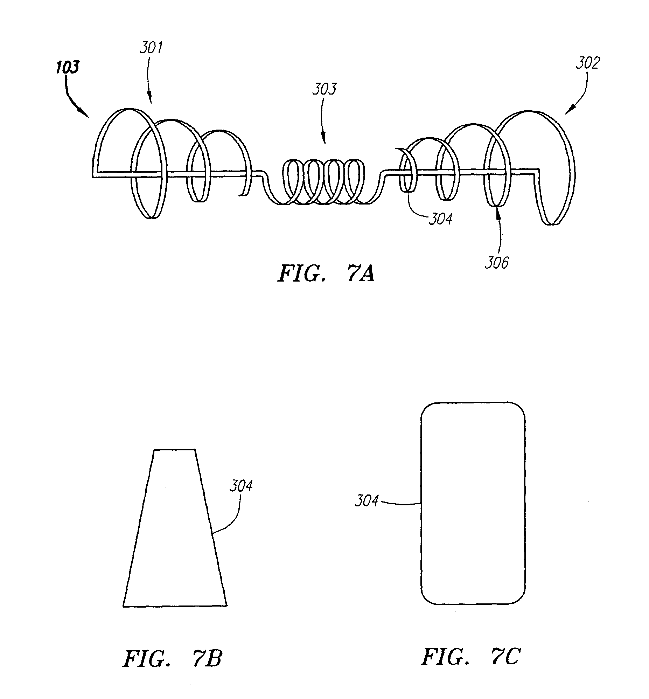

**FIG. 7A-C** are perspective views depicting additional exemplary embodiments of implant 103 having a ribbon-like body 304. Ribbon-like bodies 304 can have a generally polygonal cross-section and can be differentiated from the wire-like bodies 304 depicted in **FIGs. 4A-5E****,** which can have generally circular, rounded etc. cross-sections as described above. **FIG.** 7A is an embodiment of implant 103 having a ribbon-like body 304 configured similar to that of the embodiment depicted in **FIG. 4A****.** Generally, any of the embodiments described with respect to wire-like bodies 304 can also be implemented with ribbon-like bodies 304. Ribbon-like bodies 304 can have any ribbon-like cross-sectional shape desired. **FIGs. 7B-C** are cross-sectional views depicting ribbon-like body 304 having generally polygonal shapes. **FIG. 7B** is a cross-sectional view depicting ribbon-like body 304 having a generally tapered trapezoidal shape. **FIG. 7C** is a cross-sectional view depicting ribbon-like body 304 having a generally rectangular shape with rounded corners.

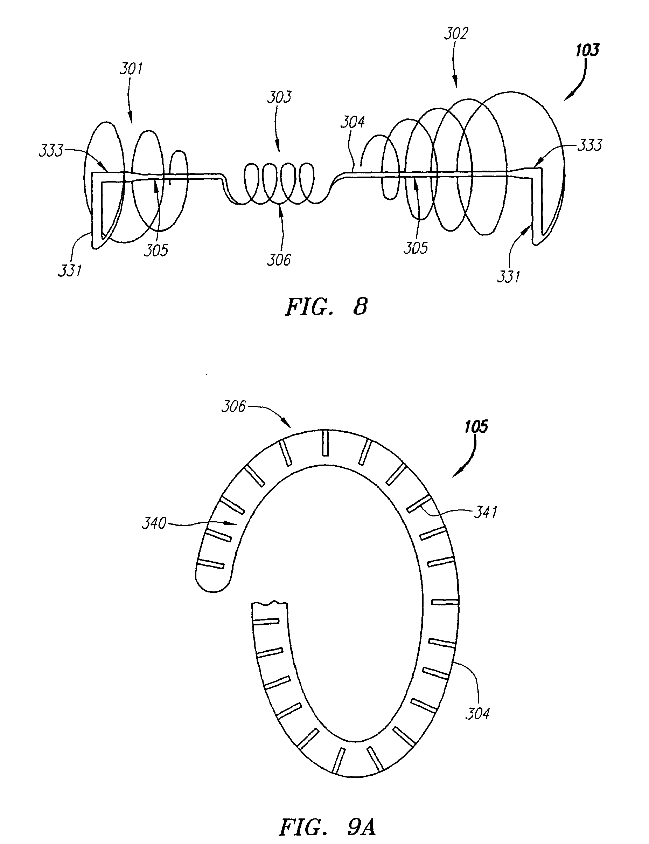

In addition to other parameters, the thickness of implant body 304 can vary as desired. For instance, **FIG. 8** is a perspective view depicting another exemplary embodiment of implant 103 having a wire-like body 304 with varying thicknesses. Here, it can be seen that generally straight section 305 is relatively thicker than the coiled segments 306 of central portion 303, while interface 333 between generally straight sections 305 and transition sections 329 is relatively thicker still. Relatively thicker regions of body 304, whether formed from a wire, ribbon or other structure, generally have greater strength and less flexibility than relatively thinner regions of body 304. Thus, relatively thicker regions can be used to add strength while relatively thinner regions can be used where added flexibility is desired.

Like the thickness, the surface of body 304 can also be varied as desired. The surface can be modified directly or through etching, grinding, additional coatings or add-ons, which are applied to the underlying body 304. The surface can be modified for any purpose including, but not limited to increasing surface friction with tissue, increasing the ability to engage tissue, allowing tissue in-growth, promoting healing, promoting scarring, promoting thrombogencity, preventing blood passage or shunting around or through implant 103, minimizing thrombus formation, promoting anti-coagulation (e.g., with drugs such as heparin and the like), modifying imaging characteristics (e.g., radio-opacity and the like) and decreasing body surface friction (e.g., with a hydrophilic coating and the like).

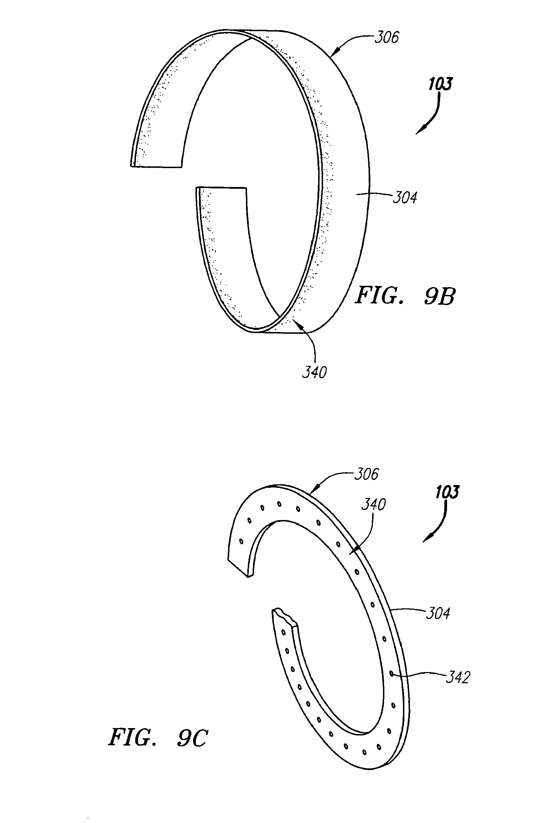

**FIGs. 9A-C** are perspective views depicting just several additional exemplary embodiments of implant 103 having a modified surface region 340. The surface of implant 103 can be modified in any location and in any manner desired, including, but not limited to, etching, grinding, coating, drilling, and cutting. For instance, **FIGs. 9A-C** depict the innermost coiled segment 306 of exemplary embodiments of RA/LA portion 301/302. **In** **FIG. 9A****,** wire-like body 304 has been etched or otherwise treated such that modified surface region 340 is a textured surface including multiple recesses 341 for increasing surface friction and allowing coiled segment 306 to more easily grasp septal wall 207. It should be noted that any surface texture pattern can be used. In **FIG. 9B****,** a coating has been applied to ribbon-like body 304 to create an abrasive surface region 340, also to increase surface friction. In **FIG. 9C****,** apertures 342 in ribbon-like body 304 are present to facilitate tissue in-growth on and around modified surface region 340. Also, in this embodiment the orientation of ribbon-like body 340 has been rotated 90 degrees so that the widest surface is adjacent to the septal tissue.

As stated above, implant 103 can be configured in any manner desired in accordance with the needs of the application. The following is a non-exhaustive list of just some exemplary factors one of skill in the art may consider in designing, configuring, manufacturing and/or otherwise implementing implant 103.

LA portion 302 can be configured to use compressive force 312 from center portion 303 to hold septum primum 214 against septum secundum 210 and at least partially close or seal PFO tunnel 215. LA portion 302 can also be configured to maintain a stable position as central portion 303 and RA portion 301 are deployed without being pulled through septum primum 210. LA portion 302 can be configured to lie flush against septum primum 214 when deployed and not to distort the native geometry of tunnel 215 to create residual shunts. LA portion 302 can be sized to provide adequate coverage over PFO tunnel 215. (In one exemplary embodiment, which is included as an example only and should not be used to limit the invention, LA portion 302 has a maximum width 310 of 1.2 centimeters to accommodate most large PFO tunnels 215.) LA portion 302, in combination with central portion 303 and RA portion 301, can be configured to exert enough closure force 314 to seal PFO tunnel 215 and prevent shunting during normal and valsalva atrial blood pressures. LA portion 302 can also be configured: to be deployable with minimal and consistent push force (e.g., push force on pusher member 406, which will be described in more detail below); so that the shape before and after deployment is predictable; to be devoid of characteristics that cause chronic or excessive tissue irritation, inflammation, etc.; and/or for visibility during imaging procedures.

Central portion 303 can be configured to maintain LA portion 302 and RA portion 301 in a state of contact with septal wall 207 with enough closure force 312 to at least partially close and seal PFO tunnel 215. Central portion 303 can also be configured: with an adequate spring constant (k) to prevent tunnel 215 from opening during normal and valsalva atrial blood pressures; not to distort the native geometry of tunnel 215 and create residual shunts; to be deployable with minimal and consistent push force (e.g., push force on pusher member 406, which will be described in more detail below); for visibility during imaging procedures; to expand or stretch to accommodate variable septal wall thicknesses without excessive permanent deformation; with adequate strength to withstand any motion it may experience in vivo; to allow LA portion 302 or RA portion 301 to tilt, for instance, if the area of delivery is wedge shaped; so that central portion 303 does not pinch or sever any tissue that could embolize, for instance, with a spring constant low enough to prevent severing tissue; to exert adequate closure force 312 to close any residual shunts that exist; and/or with maximized width 310 and minimized strains to optimize fatigue performance.

RA portion 301 can be configured to hold septum secundum 210 against septum primum 214 and at least partially close or seal PFO tunnel 215. RA portion 301 can also be configured: to lie flush against septum secundum 210 when deployed and not to distort the native geometry of tunnel 215 to create residual shunts; to be deployable with minimal and consistent push force (e.g., push force on pusher member 406, which will be described in more detail below); so that the shape before and after deployment is predictable; to be devoid of characteristics that cause chronic or excessive tissue irritation, inflammation, etc.; for visibility during imaging procedures; and/or to resist being pulled through septal wall 207.

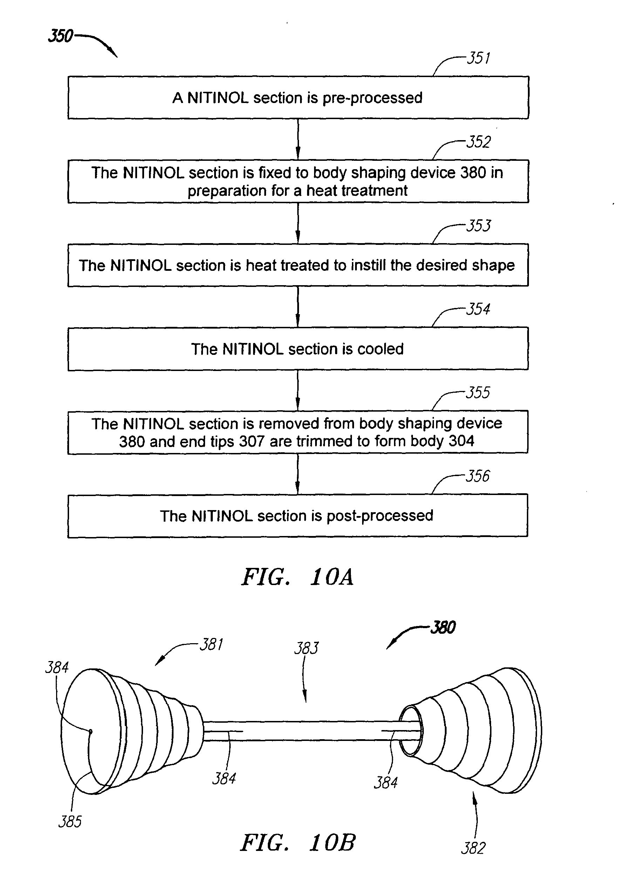

Also provided herein are methods of manufacturing implant 103. **FIG. 10A** is a flow diagram depicting one exemplary method 350 of manufacturing an exemplary embodiment of a coil-like implant 103 having body 304, which can be wire, ribbon or the like, composed of NITINOL. First, at 351, a section of NITINOL, from which body 304 can be formed, is preprocessed. Pre-processing 351 can include adding a modified surface region 340 having a desired texture, adjusting body thickness, adjusting the cross-sectional shape of body 304 and the like.

With a ribbon-like implant 103, pre-processing can include etching of the NITINOL section. Methods of etching NITINOL materials are readily understood to one skilled in the art. For instance, a sheet of NITINOL is first etched or grinded or otherwise altered to vary the cross-sectional shape, thickness, surface texture and the like of one or more sections present on the sheet. Etching of the NITINOL sheet can allow for the implementation of numerous different cross-sectional shapes, thicknesses, surface textures and combinations thereof. Afterwards, each section of NITINOL can be cut from the sheet and trimmed as desired.

At 352, the NITINOL section is fixed to body shaping device 380 in preparation for heat treatment. Heat treatment of NITINOL can instill the desired at rest configuration to body 304 and is well known to those of skill in the art. Accordingly, body shaping device 380 is preferably shaped such that when the NITINOL section is coiled around body shaping device 380, it is in the final desired at rest configuration. One exemplary embodiment of body shaping device 380 is depicted in **FIG. 10B****.** Here, body shaping device 380 is shaped for the exemplary embodiment of implant 103 depicted in **FIG. 4A****.** Body shaping device 380 includes a central body shaping portion 383 corresponding to the shape of central portion 303, and two end body shaping portions 381 and 382 corresponding to the shape of RA portion 301 and LA portion 302, respectively. End body shaping portions 381 and 382 are preferably configured to telescope over central body shaping portion 383 to allow for the inwards manner of coiling of RA/LA portions 301/302 over central portion 303. Central portion 303 includes recesses 384 into which the NITINOL section can be placed to form generally straight sections 305. End body shaping portions 381 and 382 also preferably include recess 385 that can allow for each transition section 331.

Once wrapped around and fixed to body shaping device 380, at 353, the NITINOL section is then preferably heat treated to instill the desired shape. Heat treating can occur at any time and temperature sufficient to instill the desired at rest shape and level of elasticity in implant 103. In one embodiment, which is included as an example only and should in no way be used to limit the invention, heat treating can occur at a temperature range of 500-550 degrees Celsius for approximately five minutes.

At 354, the NITINOL section is preferably cooled, e.g., by rapid quenching in room temperature water, then at 355, the NITINOL section is preferably removed from body shaping device 380 and end tips 307 are trimmed, if necessary, to the desired length to form body 304. Finally, at 356, any post-processing is performed, such as the addition of radio-opaque markers, the shaping of end tips 307 and the addition of any desired coatings or blocking material 326.

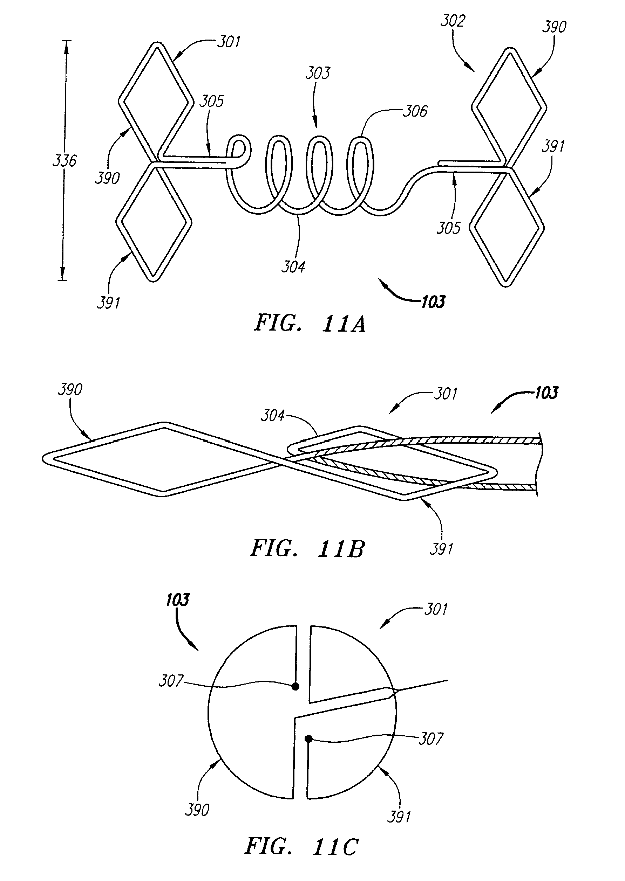

**FIGs. 11A-C** depict additional exemplary embodiments of implant 103. Specifically, **FIG. 11A** is a perspective view depicting an exemplary embodiment of implant 103 formed from multiple bodies 304. More specifically, from central portion 303 to RA portion 301 and LA portion 302, body 304 splits into separate wires which are then configured as shaped portions 390 and 391, which in this embodiment have substantially polygonal shapes. The shape and size of polygonal shaped portions 390 and 391 can be configured as desired to facilitate PFO closure. Here, portions 390 and 391 are entirely connected such that implant 103 does not have discrete end tips 307. Polygonal shaped portions 390 and 391 operate similar to coiled segments 306 and are deformable between a housed configuration and an "at rest" deployed configuration as shown here in **FIG. 11A. FIG. 11B** depicts RA portion 301 in the housed configuration. **FIG. 11C** depicts another exemplary embodiment where portions 390 and 391 have "D" shapes. Each portion 390 and 391 is not entirely connected and each portion 390 and 391 has an atraumatic end tip 307. It should be noted that body 304 can split into any number of separate portions having any number of configurations. Also, although not shown, implant 103 can include any number of separate bodies 304.

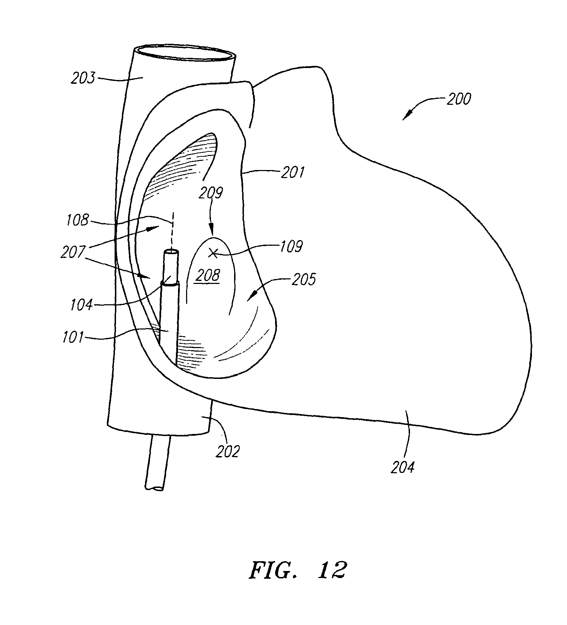

Turning now to the devices and methods for delivering implant 103, **FIG. 12** depicts another exemplary embodiment of treatment system 100 within heart 200. Implant 103 is preferably delivered from right atrium 205, although delivery from left atrium 212 is also possible. Right atrium 205 is preferably accessed via inferior vena cava 202. In this embodiment, implant 103 is delivered from within delivery device 104. To facilitate delivery in this manner, longitudinal axis 108 of delivery device 104 is preferably substantially parallel, i.e., at least close to parallel but not necessarily parallel, to the normal axis 109 of the surface of septal wall 207 into which implant 103 is to be delivered. However, as shown in **FIG. 12****,** longitudinal axis 108 of delivery device 104 is close to perpendicular to this normal axis 109 (shown here extending into the page). To accommodate for this, treatment system 100 is preferably configured for off-axis delivery, which allows the orientation of delivery device 104 to be changed so that the longitudinal axis 108 of delivery device 104 is transverse to the longitudinal axis 107 (not shown) of body member 101.

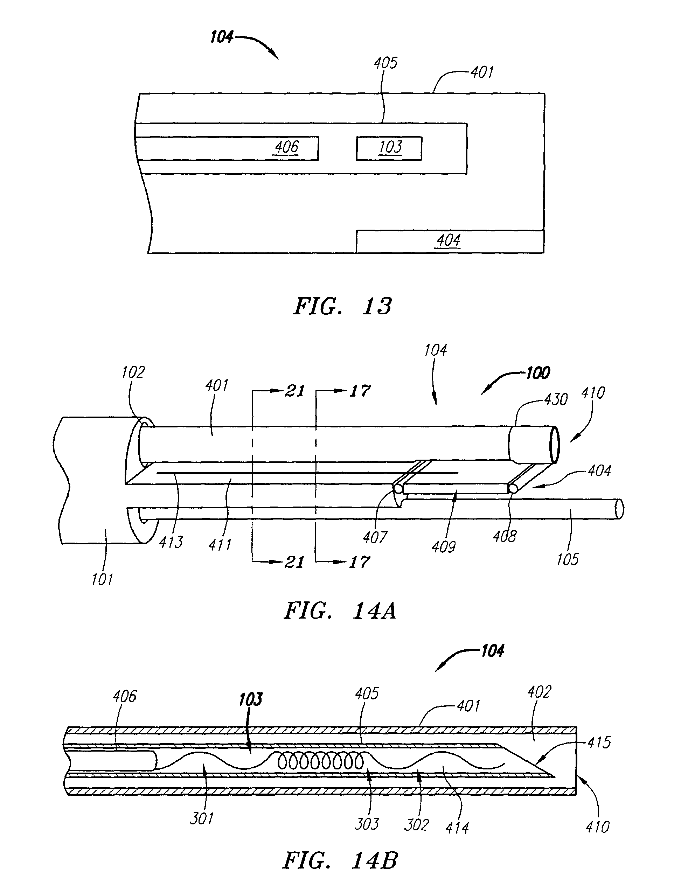

**FIG. 13** is a block diagram depicting one exemplary embodiment of delivery device 104 configured for off-axis delivery. Here, delivery device 104 includes an off-axis (OA) delivery member 401. Delivery device 104 is preferably configured to grasp or engage cardiac tissue to support and/or facilitate orientation of delivery member 401. Accordingly, an optional tissue engagement device 404 is included within delivery device 104. Delivery device 104 can also include a needle member 405 for puncturing septal wall 207 and a pusher member 406 for pushing implant 103 from within delivery device 104.

**FIG. 14A** is a perspective view depicting another exemplary embodiment of treatment system 100, including body member 101, delivery device 104 and stabilization device 105. Here, OA delivery member 401 is an elongate flexible tubular member having open distal end 410. Inner lumen 102 of body member 101 is preferably configured to slidably receive OA delivery member 401, such that OA delivery member 401 can be advanced both proximally and distally. Distal end 410 of OA delivery member 401 is coupled with an elongate support structure 411 of body member 101 via optional grasping device 404. In this embodiment, grasping device 404 includes an arm member 409 coupled with support structure 411 and OA delivery member 401 with hinges 407 and 408, respectively. A biasing element 413 can also be optionally included, to apply a bias force to maintain arm member 409 in the position shown here. Stabilization device 105 is also an elongate member preferably placed in a location to oppose arm member 401.

**FIG. 14B** is a cross-sectional view depicting another exemplary embodiment of OA delivery member 401 with embodiments of needle member 405, pusher member 406 and implant 103 located within lumen 414. Needle member 405 has an open distal end 415 and an inner lumen 414 in which pusher member 406 and implant 103 are slidably received and housed. In this embodiment, implant 103 is deformed to the housed configuration where RA/LA portions 301/302 are relatively straightened but central portion 303 remains in the coiled at rest configuration. As will be discussed in more detail below, delivery of implant 103 is accomplished by first orienting delivery device 104 in the desired orientation transverse to longitudinal axis 107 such that distal end 410 is in proximity with septal wall 207, then advancing needle member 405 through septal wall 207 to create opening 315. After needle member 405 has advanced through septal wall 207 into left atrium 212, pusher member 406 is advanced distally to push LA portion 302 of implant 103 from within lumen 414. Once LA portion 302 is outside lumen 414, LA portion 302 returns to the coiled at rest configuration. Needle member 405 can then be retracted proximally such that LA portion 302 engages septal wall 207 and remains in left atrium 212. As needle member 405 is retracted through septal wall 207, central portion 303 deploys within opening 315. Once needle member 405 is retracted back into lumen 402, OA delivery member 401 can be retracted from septal wall 207, for instance by pulling body member 101 proximally back, thereby allowing RA portion 301 to deploy and engage septal wall 207 in a coiled configuration.

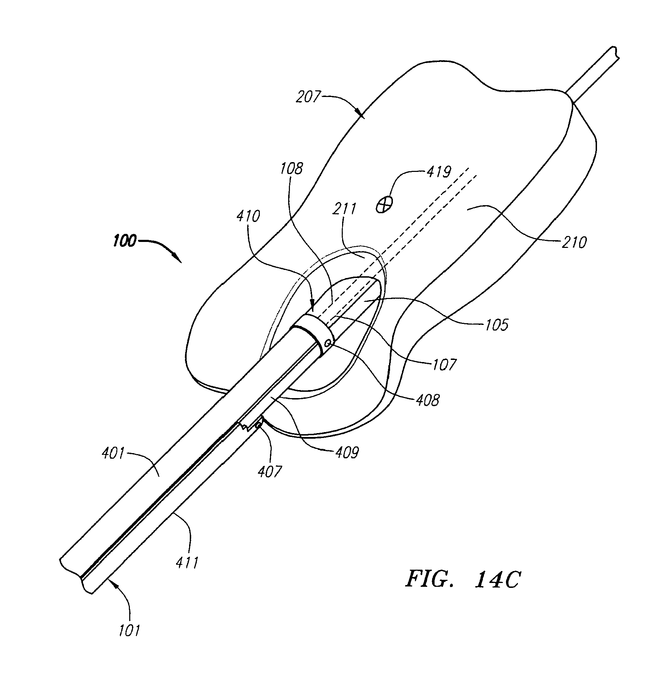

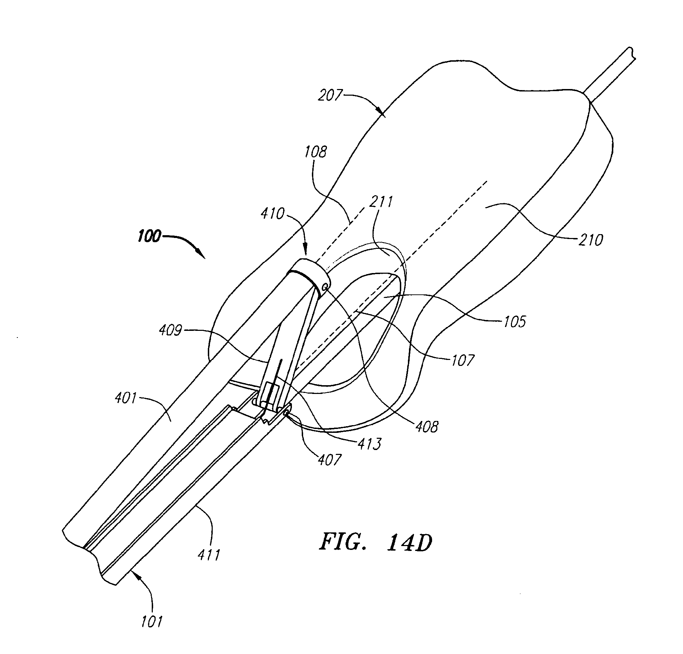

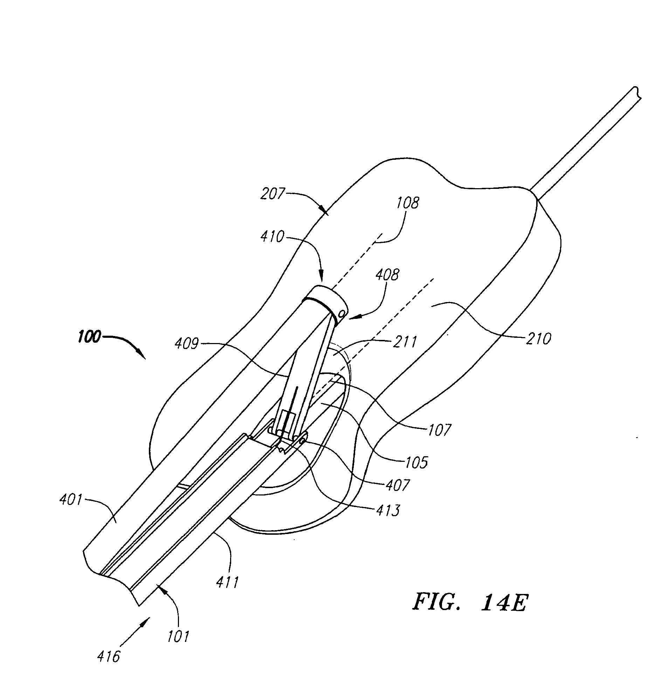

**FIGs. 14C-F** are perspective views depicting a portion of septal wall 207 and an additional exemplary embodiment of treatment system 100 during use of delivery device 104 prior to insertion of needle member 405. Here, the preferred location for insertion of needle member 405 is indicated by location 419. **FIG. 14C** depicts treatment system 100 with delivery device 401 in the on-axis position, where the longitudinal axes 107-108 are generally or substantially parallel. Stabilization device 105, the use and structure of which will be described in more detail below, is shown positioned within PFO tunnel 215. In **FIG. 14D****,** OA delivery member 401 has been retracted proximally with respect to body member 101 and in opposition to bias member 413, causing distal end 410 to move away from stabilization device 105 by way of arm member 409 and hinges 407-408. In **FIG. 14E****,** treatment system 100 is advanced distally in direction 416 until the underside surface 417 of arm member 409 abuts limbus 211, at which point OA delivery member 401 can be advanced distally with respect to body member 101 to force arm member 409 back towards stabilization device 105 to clamp, or grasp limbus 211 between arm member 409 and stabilization device 105, which is preferably in a substantially fixed position with respect to arm member 409. By grasping limbus 211 in this manner, treatment system is effectively anchored to septal wall 207.

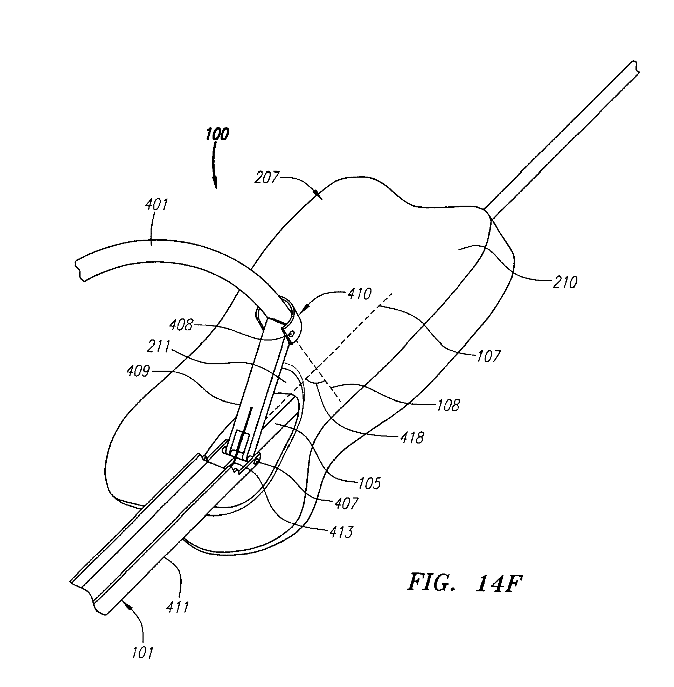

In **FIG. 14F****,** OA delivery member 401 is further advanced distally with respect to body member 101, which causes OA delivery member to deflect, or arc outwards, in order to rotate distal end 410 about hinge 408 into the desired orientation with respect to septal wall 207. Distal end 410 is now preferably in contact with septal wall 207 at the desired needle insertion location 419. As shown here, OA delivery member 401 is in an outwardly arced state. The degree to which OA delivery member 401 arcs outwards can be adjusted by altering the length of OA delivery member 401 present outside of body member 101. Because needle member 405, pusher member 406 and implant 103 all preferably move within OA delivery member 401, the radius of curvature of the arc is preferably kept large enough to allow movement within OA delivery member 401. A very large radius of curvature can result in sharp angles or kinking in OA delivery member 401 that can make movement difficult.

As shown in **FIG. 14F****,** longitudinal axis 108, as measured at distal end 410, is now transverse to longitudinal axis 107. Preferably, the delivery angle 418, which is the angle between longitudinal axis 107 and longitudinal axis 108 as measured at distal end 410, is approximately 90 degrees. Once distal end 410 is in the desired orientation, needle member 405 can be advanced into septal wall 207.

The needle insertion location 419 can be placed in any desired location, but should be chosen based in part on the configuration and size of implant 103 and the degree of overlap between septum primum 214 and septum secundum 210. For instance, in one exemplary embodiment, which is included for illustration only and in no way should be used to limit the invention, needle insertion location 419 is placed between 3 and 7 mm from limbus 211. The position of needle insertion location 419 can be determined by the length of arm member 409, which in turn can position distal end 410 using limbus 211 as a point of reference. To allow for added flexibility, the length of arm member 409 can be configured to be adjustable during the implantation procedure. Thus, arm member 409 is preferably configured for at least two functions: (1) to stop travel of body member 101 at limbus 211 by abutting limbus 211 and (2) to position distal end 410 in the desired needle insertion location 419.

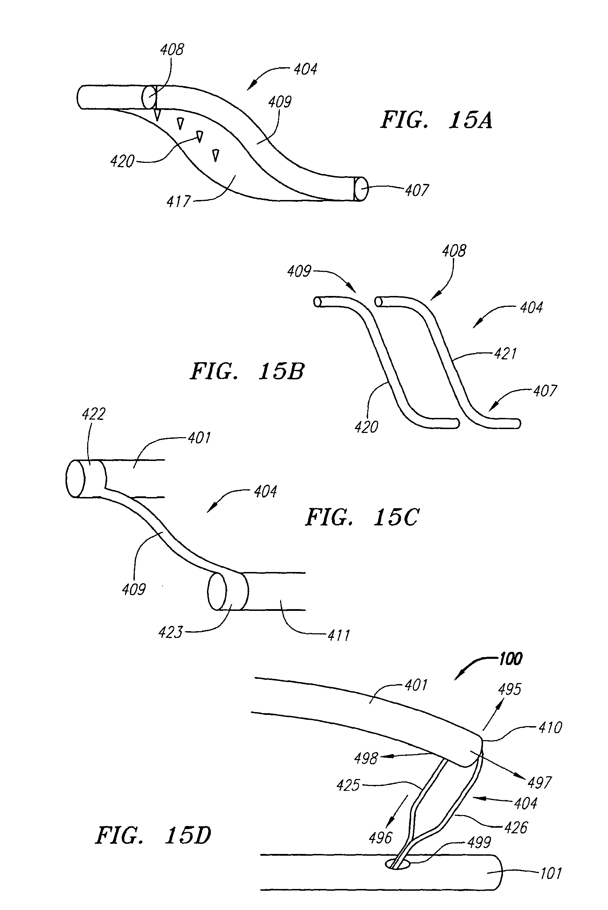

**FIGs. 15A-D** are perspective views depicting additional exemplary embodiments of grasping device 404 in a pulled back position. In **FIG. 15A****,** arm member 409 is configured to engage limbus 211 with a contoured undersurface 417 that accommodates the shape of limbus 211 in order to facilitate grasping or engagement. Undersurface 417 can also be textured as desired to increase surface friction, or made lubricious to assist in friction-free centering, and, as shown here, undersurface can include abutments 420 configured to fixably grasp limbus 211. Also, it should be noted that any type of hinges 407-408 can be used including, but not limited to, the swivel-type hinges depicted here.

**FIGs. 15B-C** depict exemplary embodiments of grasping device 404 where hinges 407 and 408 are integrated into arm member 409. In **FIG. 15B****,** arm member 409 includes two elastic wires 420 and 421 each configured to flex at hinge positions 407 and 408, e.g., by reducing the thickness of the material at the hinge positions. Arm member 409 is preferably biased towards a downwards position, which can allow elimination of any additional biasing element 413. In **FIG. 15C****,** arm member 409 is configured to be both flexible and stretchable and can be composed of an elastomeric or rubber-like material or thin or slotted metal or polymeric material with the appropriate modulus. This flexibility and stretchability facilitates the conformance of arm member 409 to limbus 211. Here, arm member 409 includes tubular portions 422 and 423 for coupling arm member 409 with OA delivery member 401 and support structure 411, respectively.

**FIG. 15D** is a perspective view depicting yet another exemplary embodiment of grasping device 404. Here, arm member 409 again includes two flexible wires 420 and 421 that can be coupled with OA delivery member 401. Like the embodiment described with respect to **FIG. 15B****,** hinges 407 and 408 can be integrated into wires 420 and 421, which can be biased towards a downwards position. As shown in Fig. **15D****,** wires 425 and 426 are preferably routed through aperture 499 into a lumen 102 within body member 101 and to the proximal end of body member 101, where they can be independently adjusted to control, or steer, OA delivery member 401. For instance, distal movement of both wires 425 and 426 moves distal end 410 of OA delivery member 401 in direction 495 and proximal movement of both wires 425 and 426 moves distal end 410 of OA delivery member 401 in direction 496, as OA delivery member 401 permits. Distal advancement of wire 425 with respect to wire 426, alone or in combination with proximal movement of wire 426 with respect to wire 425, moves distal end 410 in lateral direction 497, while reverse movement moves distal end 410 in lateral direction 498, as OA delivery member 401 permits.

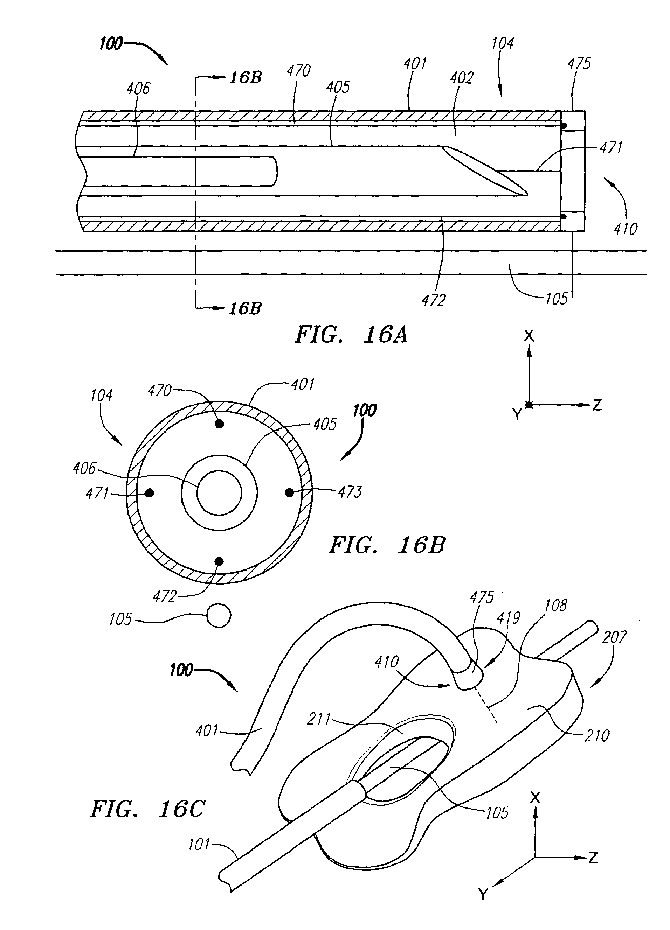

**FIGs. 16A-B** are cross-sectional views depicting additional exemplary embodiments of treatment system 100 with delivery device 104. **FIG. 16A** depicts a longitudinal cross-sectional view of treatment system 100 and **FIG. 16B** depicts a radial cross-sectional view of treatment system 100 taken along line 16B-16B of **FIG. 16A****.** Here, delivery device 104 includes a steerable OA delivery member 401, which is configured to be freely steerable to position distal end 410 in the desired orientation at needle insertion location 419. Accordingly, distal end 410 is preferably left unconnected with any grasping device 404 (not shown). Preferably, steerability is provided through the use of one or more pull wires 424 coupled with distal end cap 475. In this embodiment, four pull wires 470-473 are equally spaced apart from each other within lumen 402. This configuration allows for manipulation of distal end 410 to any three-dimensional (X, Y, Z) orientation. For instance, pulling wire 470 back proximally with respect to wires 471-473, or pulling wire 472 back proximally with respect to wires 470-471 and 473 allows movement of distal end 410 in the X-Z plane. Pulling wire 471 back proximally with respect to wires 470 and 472-473, or pulling wire 473 back proximally with respect to wires 470-472 allows movement of distal end 410 in the Y-Z plane.

**FIG. 16C** is a perspective view depicting the embodiment described with respect to **FIGs. 16A-B** during delivery. Here, distal end 410 has been oriented in its needle insertion location 419 and longitudinal axis 108 lies within both the X-Z and Y-Z planes. The degree of steerability can be altered as desired for each individual application. For instance, the inclusion of additional pull back wires can provide for more finely controllable steerability, while the deletion of any of pull wires 470-473 can eliminate freedom of steerability, but can simplify the overall design of device 104. The design and use of steerable devices is also discussed in parent U.S. Patent Application 10/847,747, filed on May 7, 2004.

As mentioned above, OA delivery member 401 is preferably configured to allow slidable movement of needle member 405, pusher member 406 and implant 103 within inner lumen 402. Preferably, OA delivery member 401 is configured so as to maintain a sufficient degree of structural integrity and kink resistance, while at the same time providing adequate torque or twist control. In one exemplary embodiment, OA delivery member 401 is composed of a flexible braided metal reinforced polymeric tube configured to provide the desired amount of kink resistance and torque control. In other exemplary embodiments, OA delivery member 401 can be composed of a braided or unbraided polymeric tube. In yet another exemplary embodiment, OA delivery member 401 is composed of a metal tube having apertures located therein to provide added flexibility. For instance, OA delivery member 401 can be a NITINOL slotted tube, with the size and spacing of each slot configured for optimal flexibility, kink resistance and torque control. The apertures are preferably placed in a location corresponding to the portion of OA delivery member 401 that extends or arcs out, while the portion of OA delivery member 401 proximal to this can be left solid without apertures to maintain resilience in OA delivery member 401 and provide resistance to push back from needle member 405 as it penetrates septal wall 207.

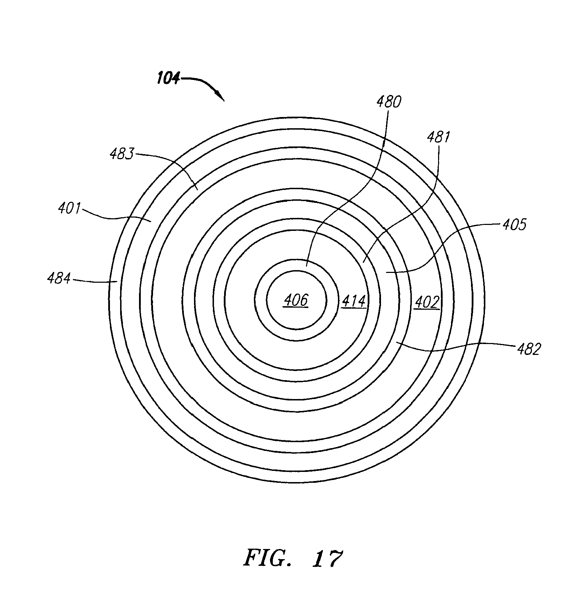

Furthermore, OA delivery member 401 can be coated to provide low friction surfaces to facilitate advancement of OA delivery member 401 within body member 101 and the patient's body, as well as to facilitate movement of needle member 405 within lumen 402. Pusher member 406 and needle member 405 can be coated as well. For instance, **FIG. 17** is a cross-sectional view depicting an exemplary embodiment of OA delivery member 401 taken along line 17-17 of **FIG. 14A****.** Here, pusher member 406 includes an outer coating 480, needle member 405 includes both an inner coating 481 and an outer coating 482 and OA delivery member 401 includes both an inner coating 483 and an outer coating 484. Coatings 480-484 can be implemented for any purpose desired. For instance, in one embodiment, coatings 480-484 are composed of any material used to lower surface friction, including, but not limited to polymers such as polyethylene (PE), polytetrafluoroethylene, fluorinated ethylene/propylene copolymers, silicones, hydrogels, hydrophilic coatings or polyurethane (PU) and the like. Preferably, a high density PE material is used that is thin enough to provide the desired degree of flexibility while at the same time providing a low friction surface.

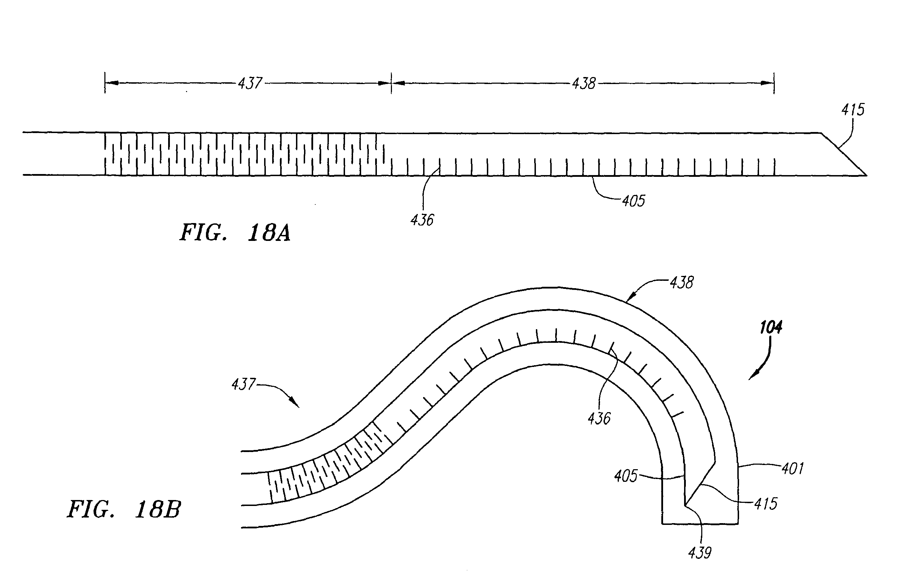

Like OA delivery member 401, needle member 405 and pusher member 406 are also preferably flexible elongate members. **FIG. 18A** is a cross-sectional view of an exemplary embodiment of needle member 405. Distal end 415 of needle member 405 is preferably substantially sharp enough to penetrate the desired portion of septal wall 207. In this embodiment, distal end 415 is tapered similar to a conventional needle. Also, needle member 405 is preferably flexible enough to move within OA delivery member 401 when deflected for off-axis delivery.

For instance, needle member 405 can include one or more openings, or apertures 436, to increase flexibility. Here, needle member 405 includes multiple apertures 436 in various arrangements. Needle member 405 can be fabricated from any desired material including, but not limited to, NITINOL and stainless steel, and apertures 436 can be formed in any manner including, but not limited to, molding, milling, grinding, laser cutting, EDM, chemical etching, punching and drilling. The design and use of flexible needles is also discussed in parent U.S. Patent Application 10/847,747, filed on May 7, 2004.

A first region 437 of needle member 405 includes apertures 436 located at various intervals around the circumference of needle member 405. A second region 438, located distal to the first region 437, includes apertures 436 on the lower portion of needle member 405. **FIG. 18B** is a cross-sectional view depicting an exemplary embodiment of needle member 405 in a deflected state within an exemplary embodiment of OA delivery member 401. Because apertures 436 in region 437 are located around the circumference of needle member 405, region 437 is relatively more flexible than region 438. In region 438, placement of apertures 436 on the lower surface, reduces the possibility that implant 103 will catch or snag an aperture 436 during advancement of needle member 405 from OA delivery member 401. In addition, distal tip 439 of needle member 405 is also preferably aligned on the lower portion of needle member 405 to reduce the possibility that distal tip 439 will impact, catch, snag, or damage OA delivery member 401.

Treatment system 100 can be configured to apply a suction-type force to any surface of septal wall 207 to allow needle member 405 to more easily penetrate the septal tissue without excessive "tenting" of septal wall 207 in response to the pressure applied by needle member 405. For instance, the proximal end of OA delivery member 401 can be coupled with a vacuum or pressure adjustment device configured to lower the air or fluid pressure within OA delivery member 401. The pressure is preferably lowered to a degree sufficient to create a suction-type force between OA delivery member 401 and septal wall 207 thereby keeping septal wall 207 in contact or in proximity with OA delivery member 401 while needle member 405 is advanced into septal wall 207. Also, the suction-type force can be applied through needle member 405 instead of, or in addition to OA delivery member 401.

Treatment system 100 preferably includes one or more sensors to facilitate determination of when needle member 405 has entered left atrium 212. For instance, in one exemplary embodiment, needle member 405 includes a sensor at or near distal end 415. The sensor can be any type of applicable sensor, such as a pressure sensor, thermal sensor, imaging device, acoustic device and the like. In one exemplary embodiment, a pressure sensor is included that is configured to sense the blood pressure change between right atrium 205 and left atrium 212. The pressure sensor can be any type of pressure sensor including, but not limited to, an electrical sensor and a fluid feedback sensor such as a lumen within needle member 405 having an open distal end in fluid communication with the exterior environment. In an alternative exemplary embodiment, distal end 415 of needle member 405 is configured to be visible by an external or internal imaging device, which can then be used to track the position of distal end 415 with respect to septal wall 207.

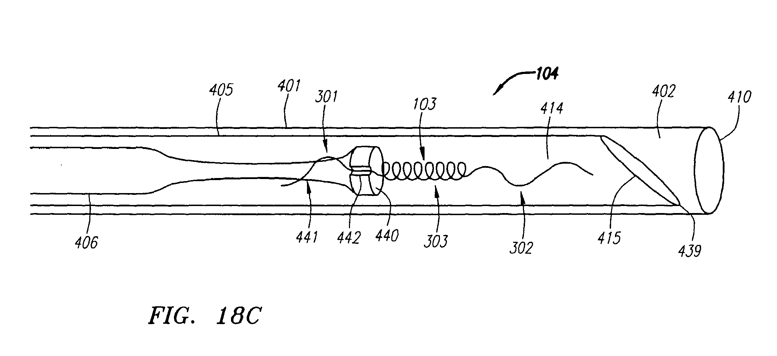

**FIG. 18C** is a cross-sectional view of another exemplary embodiment of delivery device 104. Here, distal end 440 of pusher member 406 is configured to push against central portion 303 of implant 103 as opposed to end tip 307 ofRA portion 301. This reduces the likelihood that RA portion 301 will coil when pushed within lumen 414, which could result in bunching of implant 103 within lumen 414 making delivery more difficult. Because distal end 440 of pusher member 406 is located distal to RA portion 301, pusher member 406 includes a relatively thinner portion 441 that can provide additional room for RA portion 301 within lumen 414 as well as provide added flexibility to pusher member 406. Relatively thinner portion 441 is relatively thinner than distal end 440, which is preferably thick enough to adequately engage central portion 303. Distal end 440 can include a recess 442 to provide enough room for RA portion 301. Recess 442 can also be used to help position implant 103 during delivery. For instance, rotation of pusher member 406 can cause implant 103 to rotate if implant 103 is still routed through recess 442. This can allow the proper rotational orientation of implant 103 before or during delivery into septal wall 207. Distal end surface 443 can be configured in any manner desired to facilitate proper contact and engagement of implant 103.

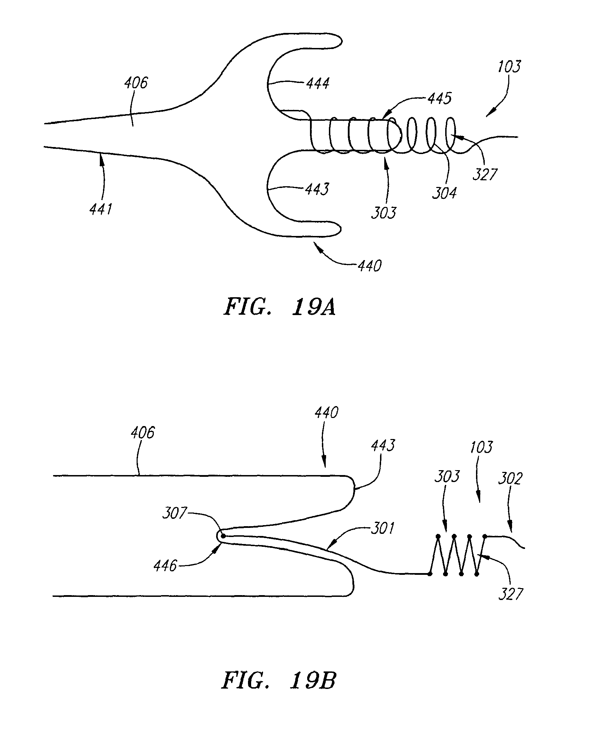

For instance, **FIGs. 19A-B** are cross-sectional views depicting exemplary embodiments of pusher member 406 and implant 103. In **FIG. 19A****,** distal end surface 443 is contoured with a rounded recessed portion 444 into which a coiled central portion 303 can rest and an elevated portion 445 configured to fit within open interior region 327. As one of skill in the art will readily recognize, the contours of distal end surface 443 are dependent on the type and housed configuration of implant 103, as well as the desired point of contact on implant 103. In **FIG. 19B****,** distal end surface 443 is contoured with a narrow recessed portion 446 into which end tip 307 of RA portion 301 can rest.

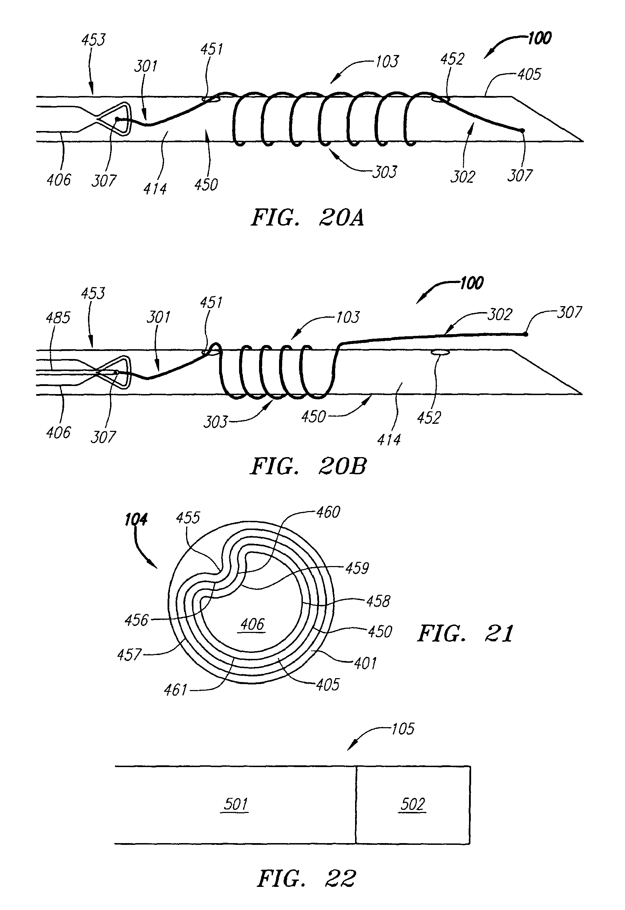

Pusher member 406 can also be configured to releasably couple with implant 103. For instance, in one exemplary embodiment, pusher member 406 is tethered to implant 103 with a tether 485 in order to allow implant 103 to be drawn back into needle member 405 if needed, such as in a case of improper deployment. If implant 103 is properly deployed, tether 485 can be released from pusher member 406. In another exemplary embodiment, pusher member 406 can be configured to both push and pull implant 103 while within needle member 405, as depicted in **FIGs. 20A-B.**

**FIGs. 20A-B** are schematic views depicting additional exemplary embodiments of needle member 405, pusher member 406 and implant 103. In **FIG. 20A****,** implant 103 is placed over outer surface 450 of needle member 405 and end tips 307 of RA portion 301 and LA portion 302 can be routed through apertures 451 and 452, respectively, and housed within lumen 414. To deliver implant 103, after needle member 405 has traversed septal wall 207 into left atrium 212, pusher member 406 is used to pull implant 103 back proximally to expose end tip 307 of LA portion 302 as depicted in **FIG. 20B****.** To grasp end tip 307, pusher member 406 can include any type of grasping device desired. Here, pusher member 406 includes a clamp-type device 453. Once removed from aperture 452, LA portion 302 can enter the coiled state. As needle member 405 is withdrawn back through septal wall 207, LA portion 302 engages septal wall 207 and cause implant 103 to slide off needle member 405. Pusher member 406 can also be used to push end tip 307 of RA portion 301 to facilitate deployment. In this embodiment, proximally located end tip 307 includes an aperture through which a tether 485 is routed for use as described above.

Delivery device 104 can be configured to maintain the proper orientation of OA delivery member 401, needle member 405, pusher member 406 and implant 103 during delivery. **FIG. 21** is a cross-sectional view depicting another exemplary embodiment of delivery device 104 taken along lines 21-21 of **FIG. 14A** where delivery device 104 is configured to use a lock and key technique to maintain proper orientation. Here, the lock and keys are implemented with a combination of abutments and corresponding recesses. For instance, outer surface 450 of needle member 405 includes a recess 456 configured to receive an abutment 455 located on inner surface 457 of OA delivery member 401. Recess 456 can extend longitudinally along needle member 405 for any desired distance to ensure proper orientation even when needle member 405 is advanced and retracted within OA delivery member 401. Similarly, outer surface 458 of pusher member 406 includes a recess 459 configured to receive an abutment 460 located on inner surface 461 of needle member 405. Like recess 456, recess 459 can extend longitudinally along pusher member 406 for any desired distance to ensure proper orientation when pusher member 406 is advanced and retracted. As discussed above with respect to **FIGs. 18A-B,** pusher member 406 can include recess 442 to accommodate for the presence of RA portion 301. This recess 442 can also maintain implant 103 in the proper orientation with respect to pusher member 406.

The distances that OA delivery member 401, needle member 405 and pusher member 406 are moved proximally and distally with respect to body member 101, can be relatively small. Manual movement of these components, while possible, can be difficult. Treatment system 100 can include one or more automated systems or devices at the proximal end of body member 101 to facilitate movement of these components and lessen the risk that each component is inadvertently advanced too far or not enough. The automated systems or devices can also be configured to apply the desired amount of force to move each component and sense if too much force is being used, which could be indicative of an error in the delivery process.

To further facilitate movement of OA delivery member 401, needle member 405 and pusher member 406, each can be optionally pre-shaped. For instance, in one exemplary embodiment, one or more of OA delivery member 401, needle member 405 and pusher member 406 can include a curved section that corresponds to the desired deflected arc shape of OA delivery member 401 depicted in **FIG. 14F****.**

It should also be noted that needle member 405 can be excluded from system 100 altogether. Pusher member 406 can deploy implant 103 through a pre-existing hole, or implant 103 can be configured with a substantially sharp end tip 307 for creation of a hole while being deployed by pusher member 406.

As described with respect to **FIG. 1****,** treatment system 100 can optionally include stabilization device 105. **FIG. 22** is a block diagram depicting an exemplary embodiment of stabilization device 105 within treatment system 100. Here, stabilization device 105 is preferably configured to stabilize treatment system 100 during delivery of implant 103. Stabilization device 105 can have any configuration desired in accordance with the needs of the application. For instance, stabilization device 105 can be configured as a body routed through PFO tunnel 215 or any portion of the patient's vasculature, such as superior vena cava 203. Stabilization device 105 preferably includes an elongate stabilization member 501 and can optionally include grasping device 502, which is preferably configured to grasp nearby tissue in order to facilitate stabilization.

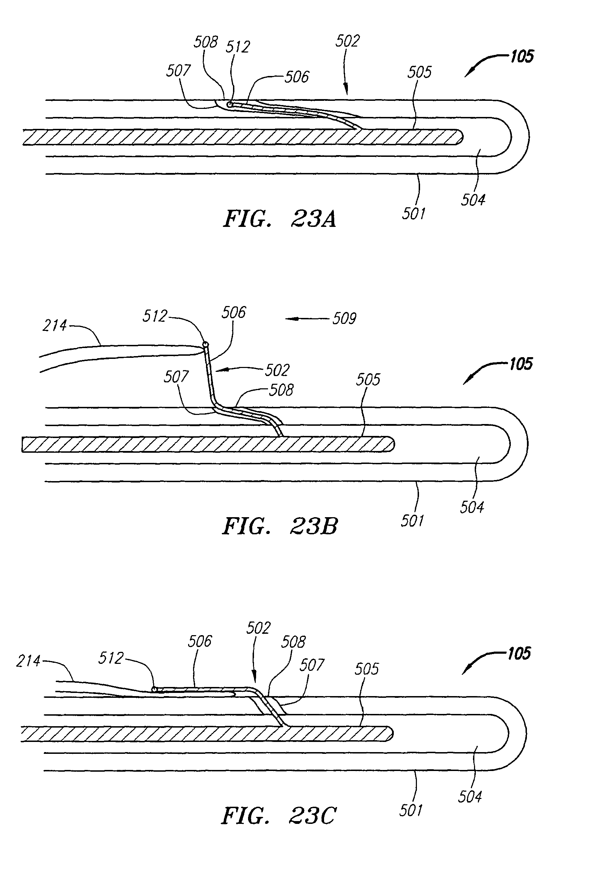

**FIGs. 23A-C** are cross-sectional views depicting additional exemplary embodiments of stabilization device 105 being used to in an exemplary method of stabilizing treatment system 100. Here, stabilization member 105 is configured as an elongate member including an outer tubular sheath 501 having an inner lumen 504 configured to slidably receive inner elongate pull member 505. Outer tubular sheath 501 and inner pull member 505 are preferably semi-rigid, having enough rigidity to stabilize treatment system 100 while at the same time having enough flexibility to allow movement and manipulation within the patient's vasculature and heart 200. In these embodiments, stabilization device 105 is preferably configured to be routed from right atrium 205 through PFO tunnel 215 into left atrium 212, where grasping device 502 can be used to cover a portion of septum primum 214 and anchor stabilization device 105 thereto.

The nature of the tissue forming septum primum 214 can be irregular, for instance including overlapping folds, variations in tissue thickness and variations in distensibility, each of which can cause septum primum 214 to move, or tent, when needle member 405 is advanced through. The inclusion of grasping device 502 can also provide the additional advantage of holding septum primum 214 in place and reducing the risk of tenting.

Grasping device 502 preferably includes a flexible grasping element 506 coupled with inner pull member 505. Here, grasping element 506 is configured as a rectangular element. Outer tubular sheath 501 preferably includes lumen 507 having open distal end 508, from which grasping element 506 can be deployed. Lumen 507 can be configured with contoured sidewalls to facilitate deployment of grasping element 506. To deploy grasping element 506, inner member 505 can be pulled in a proximal direction with respect to outer sheath 501, causing grasping element 506 to advance through lumen 507 and out of distal end 508. Grasping element 506 can optionally include an atraumatic end 512, which in this embodiment is a radio-opaque element, which may be gold or platinum. In this embodiment, grasping element 506 is configured as a deformable, pre-shaped element having three main configurations.

**FIG. 23A** depicts grasping element 506 in a first configuration housed within lumen 507. This configuration is preferably used while treatment system 100 is moved through the patient's vasculature and as well as when stabilization device 105 traverses PFO tunnel 215, as depicted here. **FIG. 23B** depicts grasping element 506 in a second configuration partially deployed from within lumen 507. Once stabilization device 105 is advanced through PFO tunnel 215 and out of PFO exit 218, grasping element 506 is preferably deployed to this configuration by pulling inner member 505 proximally with respect to outer sheath 501. In this configuration, grasping element 506 can be used to catch the edge of septum primum 214 as stabilization device 105 is pulled slightly back in proximal direction 509. **FIG. 23C** depicts grasping element 506 in a third, fully deployed configuration, after inner member 505 has been pulled back further. Grasping element 506 can optionally include a recess configured to engage an abutment on outer sheath 541 in this configuration, which is preferably used to more fully grasp or engage septum primum 214 to anchor stabilization device 105 thereto.

Once the delivery procedure is complete, inner member 505 can be advanced distally with respect to outer sheath 501 to draw grasping element 506 back within lumen 507. Any component of treatment system 100 adequately coupled with stabilization device 105 is thereby also anchored to septum primum 214. One of skill in the art will readily recognize that this and similar embodiments of stabilization device 105 can be used to engage any tissue flap or edge desired, not solely septum primum 214.

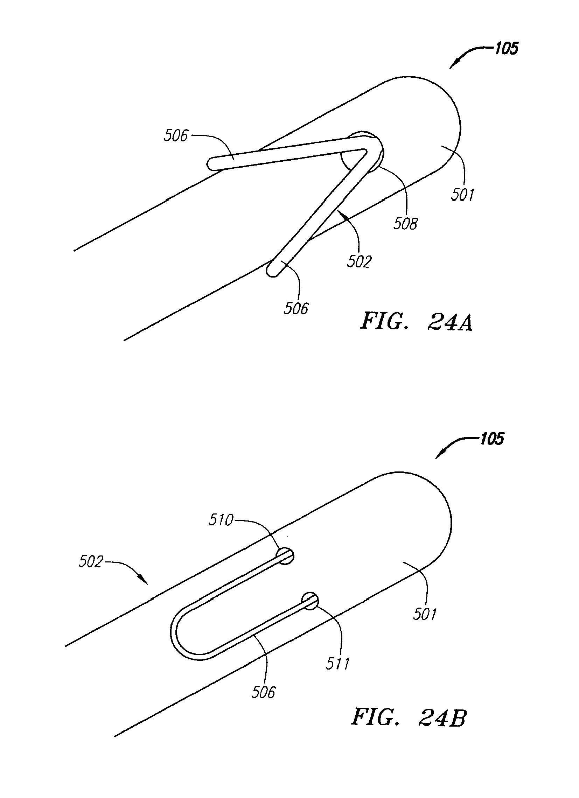

Grasping device 502 can be configured in any manner desired in accordance with the needs of the application. **FIGs. 24A-B** are perspective views depicting additional exemplary embodiments of stabilization device 105 with grasping device 502. In **FIG. 24A****,** grasping device 502 includes multiple grasping elements 506 for grasping over a wider area. In **FIG. 24B****,** grasping device 502 includes a wire-like grasping element 506. Here, grasping element 506 is looped into lumen 507 (not shown) via apertures 510 and 511, which communicate with lumen 507.

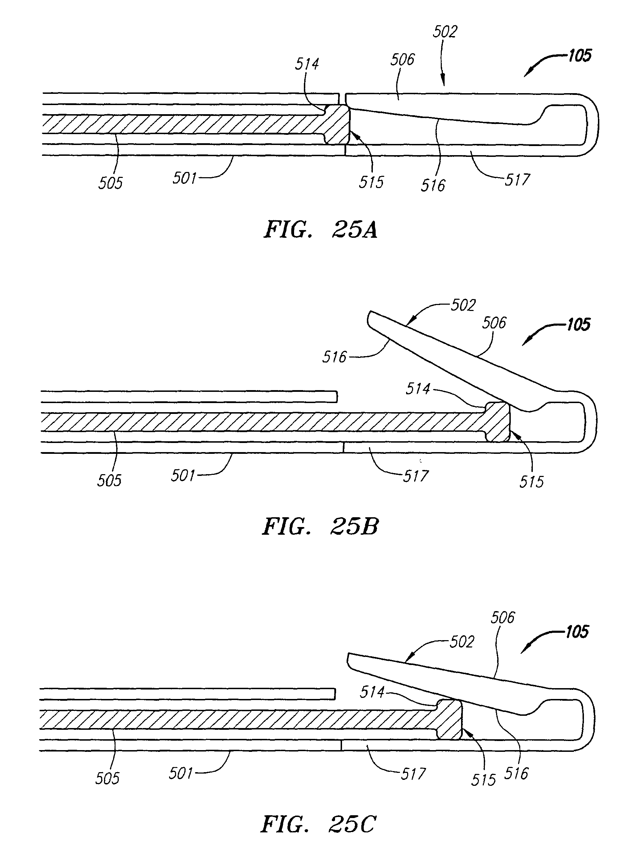

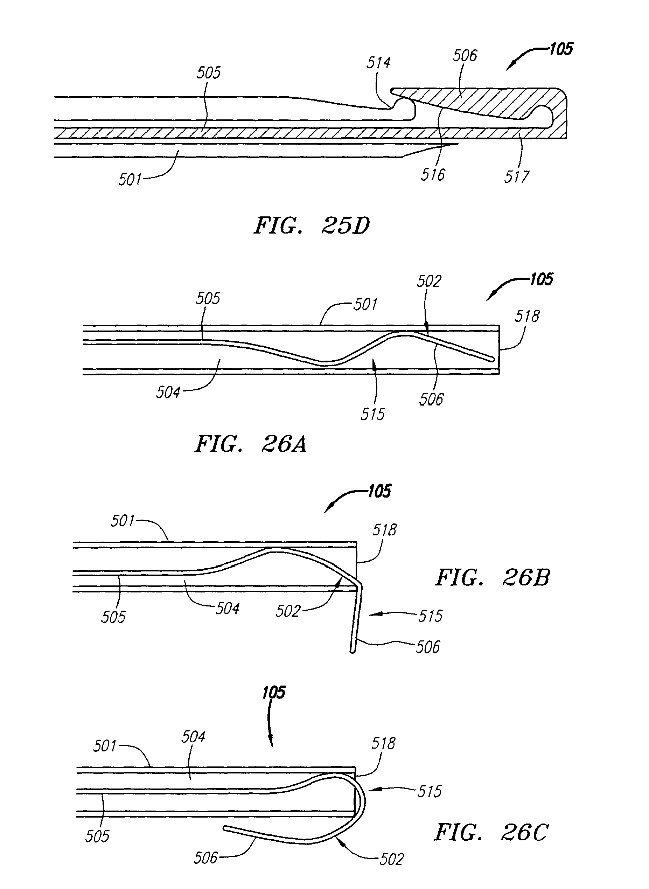

**FIGs. 25A-D** are cross-sectional views depicting additional exemplary embodiments of stabilization device 105. Here, grasping element 506 has a flap-like shape with tapered inner surface 516 and is located on distal end member 517 of outer sheath 501. Inner member 505 includes an abutment 514 on distal end portion 515 and is configured to push against and apply a force to grasping element 506. **FIG. 25A** depicts grasping element 506 in the first, housed configuration. To deploy grasping element 506 to the second configuration for catching septum primum 214, inner member 505 is advanced distally with respect to outer sheath 501 as depicted in **FIG. 25B****.** Because of tapered inner surface 516, the more inner member 505 is advanced distally, the more outwards deflection of element 506 will occur. To more fully grasp septum primum 214, inner member 505 (and body member 101, if necessary) is retracted proximally by the desired amount, as depicted in **FIG. 25C****.** Manufacture of this embodiment can be made relatively simple. For instance, distal end member 517 and grasping element 506 can be formed by laser or EDM cutting a NITINOL tube. In **FIG. 25D****,** distal end member 517 is located on distal end of inner member 505 and abutment 514 is located on sheath 501.

**FIGs. 26A-C** are cross-sectional views of additional exemplary embodiments of stabilization device 105. Here, outer sheath 501 preferably includes an open distal end 518, from which grasping device 502 can be deployed. Grasping element 506 is preferably located on distal end portion 515 of inner member 505 and can be formed of a deformable elastic material such as stainless steel, NITINOL, shape memory polymers and the like. Grasping element 506 is preferably configured to be slidable within inner lumen 504 and is preferably pre-shaped, such as by heat-treating NITINOL, so that grasping element 506 can assume a desired shape when advanced from inner lumen 504. In **FIG. 26A****,** grasping element 506 is depicted in the first, housed configuration within inner lumen 504. In **FIG. 26B****,** inner member 505 has been advanced distally to deploy grasping element 506 in the second configuration for catching septum primum 214. In **FIG. 26C****,** inner member 505 has been advanced further distally to place grasping element 506 in the third configuration for grasping septum primum 214. Embodiments of stabilization device 105 where grasping device 502 can be deployed by pushing grasping device 502 out from within inner lumen 504, such as that described with respect to **FIGs. 26A-C,** will be referred to herein as "push out" embodiments.

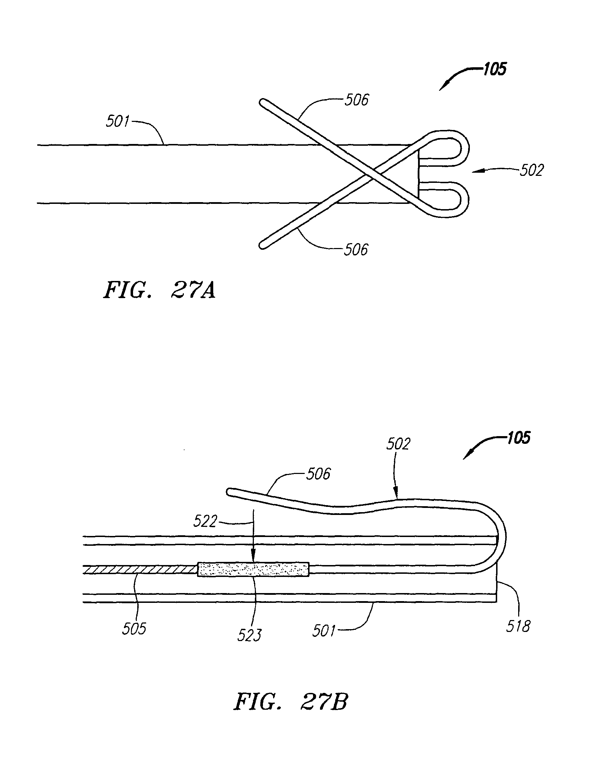

**FIG. 27A** is a perspective view depicting an additional exemplary embodiment of stabilization device 105 having a "push-out" grasping device 502. Here, grasping device 502 is shown in the fully deployed third configuration having two grasping elements 506. It should be noted that grasping device 502 can include any number of grasping elements 506. Here, each grasping element 506 overlaps so as to provide additional grasping force at location 419 where needle member 405 insertion occurs. **FIG. 27B** is a cross-sectional view depicting another exemplary embodiment where grasping element 506 is configured to attract to a magnetic force 522 provided by magnet 523 coupled with inner member 505. Once deployed, the magnetic force is preferably great enough to penetrate outer sheath 501 and septum primum 214 and attract elements 506 to provide additional grasping force. Of course, magnet 523 can be placed in any desired location, for instance, on outer sheath 501 at distal end 518 or on grasping element 506, in which case inner member 505 could be configured to attract to the magnetic force, or any combination thereof.

It should be noted that, in order to provide additional surface friction, additional abutments can be included on grasping element 506 and/or the surface of grasping element 506 can be etched or coated or otherwise textured.

As discussed with respect to **FIG. 1****,** treatment system 100 can include centering device 106 to facilitate proper placement of implant 103. Centering device 106 can be configured to align delivery device 104 in the desired location with respect to the center of PFO tunnel 215. Although the term "centering" is used, it should be understood that centering device 106 can be configured to align delivery device 104 in any location, not necessarily the center of PFO tunnel 215.

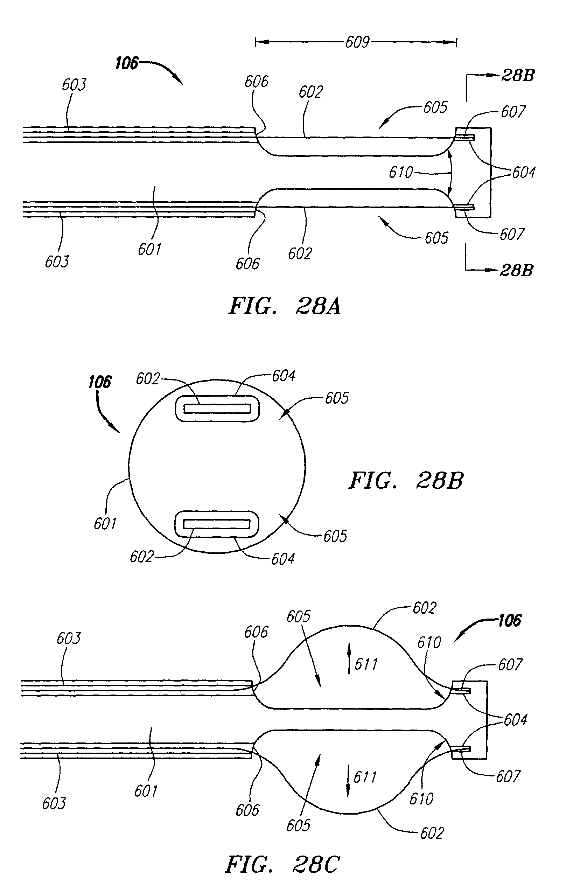

**FIGs. 28A-C** are cross-sectional views depicting additional exemplary embodiments of centering device 106. In this embodiment, centering device 106 includes an elongate centering support member 601 having two elongate flexible positioning members 602, referred to herein as centering arms 602, located on opposite sides of and extending along the length of support member 601. Support member 601 can include two lumens 603, each configured to slidably receive a centering arm 602. Each lumen 603 preferably has an open distal end 606 which opens to an open or recessed portion 605 of support member 601. Each centering arm 602 preferably extends through this recessed portion 605 and into seat 604 preferably configured to receive distal end 607 of each centering arm 602. Seat 604 is preferably located in recessed portion 605 in a position opposite to lumen 603.

**FIG. 28A** depicts centering arms 602 at rest within recessed portion 605 along the sides of support member 601. **FIG. 28B** is a cross-sectional view of centering device 106 taken along line 28B-28B of **FIG. 28A****.** As depicted here, centering arms 602 are preferably configured as rectangular wire bands, although any configuration can be used as desired. Advancement of centering arms 602 in a distal direction causes distal end 607 to contact seat 604 and forces centering arms 602 to extend outwards from recessed portion 605 as depicted in **FIG. 28C****.** Configuration of centering arms 602 as bands helps ensure that arms 602 extend directly away from support member 601 in direction 611.

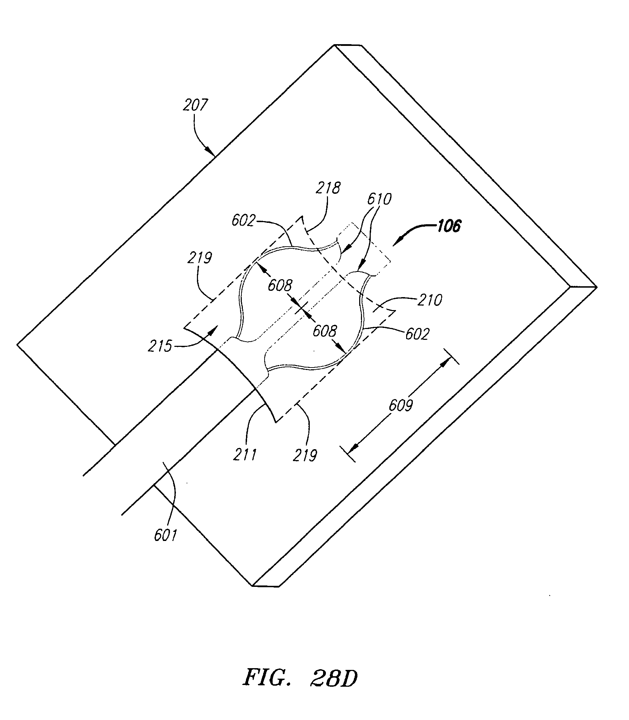

When centering device 106 is placed within PFO tunnel 215, centering arms 602 can be extended until coming into contact with sidewalls 219, as depicted in **FIG. 28D****,** which is a perspective view of centering device 106 within PFO tunnel 215. Here, sidewalls 219 and PFO exit 218 are shown as dashed lines to indicate their presence underneath septum secundum 210. When centering arms 602 are each advanced the same amount until contact with both sidewalls 219 is made, the extension distance 608 of each arm 602 will likewise be the same amount and support member 601 will be forced into a centered position within PFO tunnel 215.

In this manner, centering device 106 can be centered within PFO tunnel 215 and can be used as a reference point for delivering implant 103. Preferably, centering device 106 is coupled with delivery device 104, so that centering of centering device 106 will also cause centering of delivery device 104. Preferably, once implant 103 is delivered, centering arms 602 are retracted proximally into lumens 603 and centering device can then be retracted through PFO tunnel 215. Surface 610 of recessed portion 605 is preferably curved, or tapered, to reduce the risk that support member 601 will catch or become hung up on any tissue in or around PFO tunnel 215.

Here, the extended portions of centering arms 602 are shown as being located entirely within PFO tunnel 215. One of skill in the art will readily recognize that variation of length 609 of recessed portion 605 will cause the extended portion of centering arms 602 to vary accordingly.

Support member 601 and centering arm 602 can each be composed of any desired material in accordance with the needs of the application. Preferably, support member 601 is composed of a flexible polymer, such as polyimides, polyamides, polyproylene and the like. Preferably, centering arms 602 are composed of a flexible polymer or metal, such as NITINOL, stainless steel and the like.

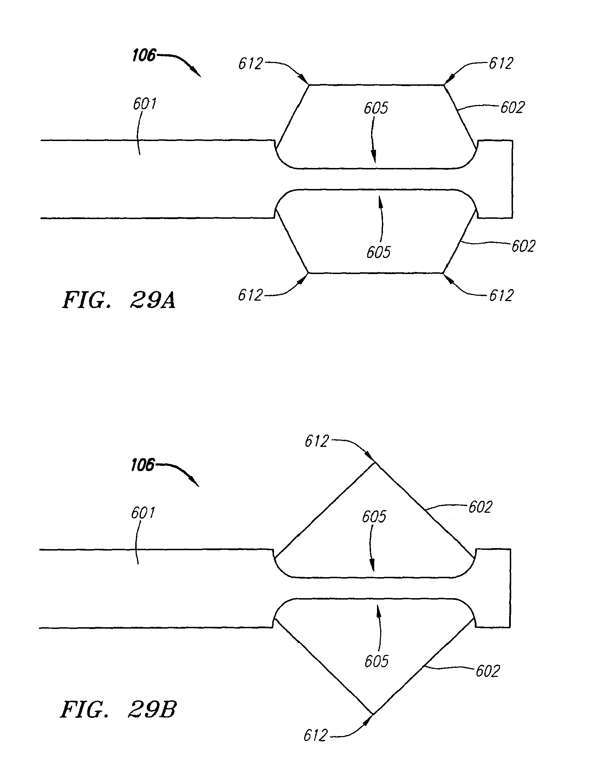

In the embodiment described with respect to **FIGs. 28A-D,** centering arms 602 have a curved or arcuate shape when extended from support member 601. As the **FIGs. 29A-C** will show, centering arms 602 can be configured to have any desired shape when extended. **FIGs. 29A-B** are schematic views depicting additional exemplary embodiments of centering device 106 with centering arms 602 extended in a three-sided and two-sided shapes, respectively. Preferably, portions 612 of centering arms 602 are made thinner than the surrounding portions, so that centering arms 602 have a tendency to flex first in portions 612, allowing these polygonal shapes to be achieved.

Also, arms 602 can be pre-shaped to be biased to assume a desired shape when allowed to expand from recessed portion 605. For instance, in one exemplary embodiment, arms 602 are composed of NITINOL and are heat-treated for pre-shaping. One of skill in the art will readily recognize, in light of this disclosure, that variation of the thickness of arms 602 and pre-shaping can allow an almost limitless number of shapes to be achieved, having curved portions, straight portions and any combination thereof which can be symmetric or asymmetric.

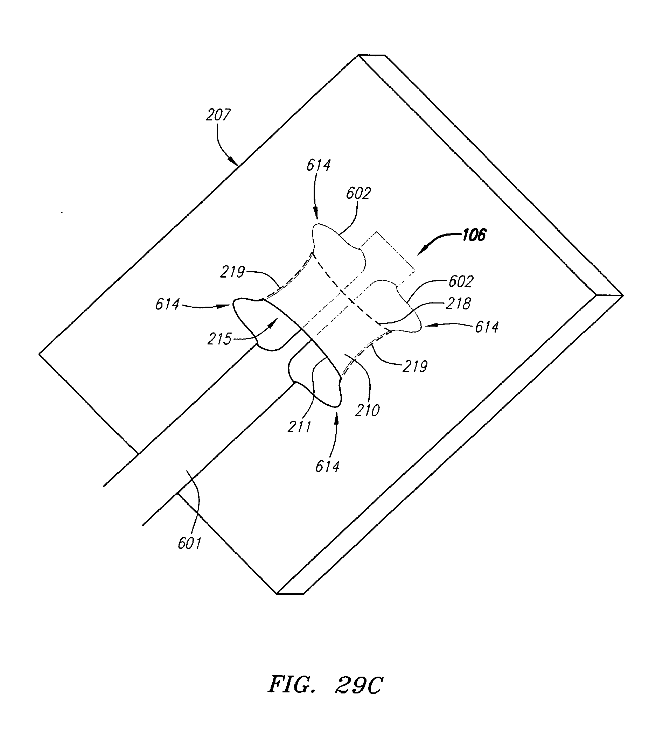

As mentioned above, in some cases, sidewalls 219 of PFO tunnel 215 are not equidistant along the length of PFO tunnel 215, causing PFO tunnel 215 to diverge or converge from PFO entrance 217 to PFO exit 218. Divergence or convergence of PFO tunnel 215 can cause centering device 106 to slip out from PFO tunnel 215 when arms 602 are extended. **FIG. 29C** is a schematic view depicting another exemplary embodiment of centering device 106 where each centering arm 602 is configured to extend with two outcroppings 614. These outcroppings 614 can be placed outside PFO tunnel 215 to prevent centering device 106 from slipping out of PFO tunnel 215. Outcroppings 614 can be formed by making that portion of centering arm 602 relatively thicker than the surrounding portions, making outcropping 614 less likely to flex. A desired radius of curvature in centering arms 602 can be implemented by pre-shaping, or by gradually varying the thickness and/or width of centering arms 602, where a relatively thinner portion will correspond to a relatively larger rate of curvature.

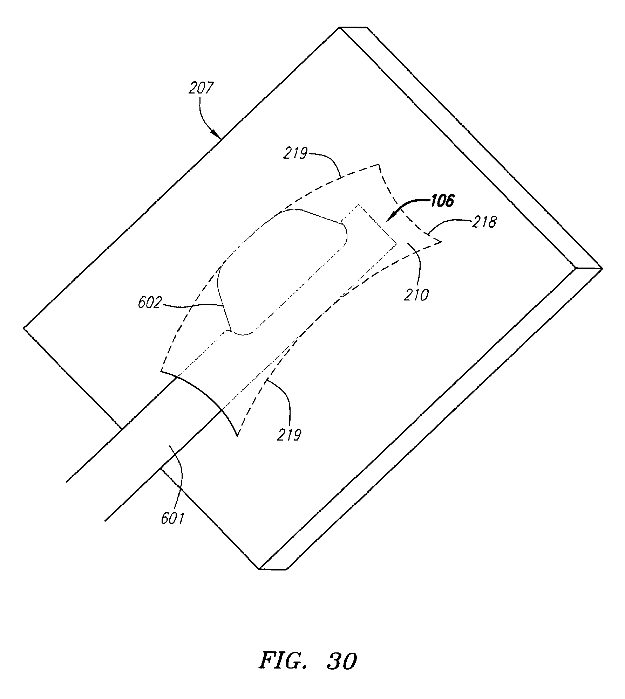

It should be noted that centering device 106 can include any number of one or more arms 602 for centering/positioning purposes. FIG. 30 is a schematic view depicting another exemplary embodiment of centering device 106 having one centering arm 602 extended within PFO tunnel 215. In this embodiment, PFO tunnel 215 is curved to one side and centering arm 602 is positioned on the opposite side. Centering arm 602 can then be extended a predetermined distance to position centering device 106 in the desired location.

In another exemplary embodiment, centering device 106 includes multiple arms 602 configured for use independently of each other to allow the user to have increased control over the position of centering device 106 within PFO tunnel 215. For instance, the user can adjust two opposing arms 602 to center device 106 between sidewalls 219 within tunnel 215, and then adjust a third arm 602 to position device 106 as desired relative to septum secundum 210 and septum primum 214. In another case, the user can use three or more arms 602 for centering based on the tunnel type or anatomy.

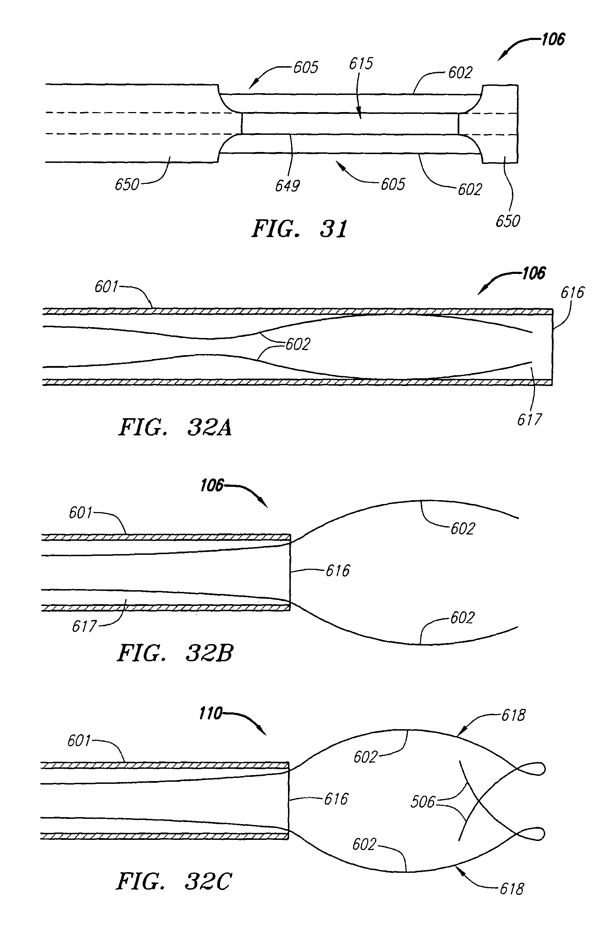

In some embodiments, it can be desirable to keep centering device 106 within PFO tunnel 215 while needle member 405 is advanced through septal wall 207. To reduce the risk that needle member 405 will contact centering device 106 during this procedure, support member 601 can be configured to deflect needle member 405. **FIG. 31** is a schematic view depicting an exemplary embodiment of centering device 106 where support member 601 is a rigid cylindrical member 649 having a smooth, or polished, surface 615 between lumen 603 and seat 604 (as shown in **FIG. 28A****),** which are formed in rigid extrusions 650 which are preferable metal and located on member 649. Here, if sharpened distal end 415 of needle member 405 comes into contact with support member 601, it is more likely to be deflected from rigid cylindrical member 649.

**FIGs. 32A-B** are cross-sectional views depicting additional exemplary embodiments of centering device 106 where support member 601 includes an open distal end 616 from which one or more pre-shaped centering arms 602 can be extended. Centering arms 602 are preferably pre-shaped to the extended position allowing elimination of seat 604 and recessed portion 605. Centering arms 602 are preferably deformable from a first configuration to allow housing within inner lumen 617 of support member 601 as depicted in **FIG. 32A****.** In **FIG. 32B****,** centering arms 602 are shown deployed from inner lumen 617 in their extended second configuration. Although in **FIGs. 32A-B,** centering arms 602 are shown as separate elements, the proximal end of the pre-shaped portion of each arm 602 can be coupled together on a common elongate shaft.

It should be noted that the functionality of the various embodiments described herein can be combined and integrated together to reduce the number of components in treatment system 100, simplify the design of treatment system 100 and so forth. For instance, **FIG. 32C** depicts an exemplary embodiment of treatment system 100 where the embodiments described with respect to **FIGs. 27A** and **32A-B** have been integrated together to form device 110. Here, centering arms 602, similar to that depicted in **FIGs. 32A-B** each include grasping element 506 of stabilization device 105, similar to that depicted in **FIG. 27A****,** located distal to the centering portion 618. Here, centering device 106 is used for centering and stabilization, allowing the elimination of a separate stabilization device 105 from system 100.

For stabilization and centering, support member 601 is preferably advanced through PFO exit 218. Once in left atrium 212, centering arms 602 can be advanced distally to deploy grasping elements 506 from the first, housed configuration, to the second and third configurations for catching and grasping septum primum 214. Once septum primum 214 is grasped, support member 601 can be retracted proximally with respect to centering arms 602 in order to deploy centering portions 618 of each arm 602. The centering portions 618 can then expand outwards and center device 106, thereby preferably also centering body member 101 1 and delivery device 104, while at the same time maintaining a grasp of septum primum 214.

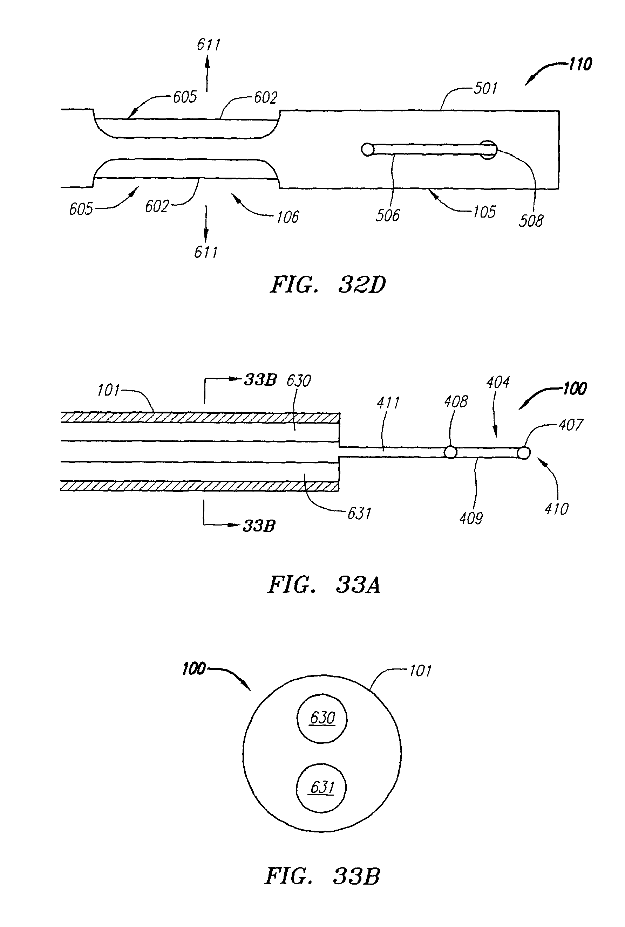

**FIG. 32D** is a schematic view depicting another exemplary embodiment of treatment system 100 where centering device 106 and stabilization device 105 have been integrated together. Here, stabilization member 501 includes two lumens 603 and seats 604 (not shown), and recessed portions 605 for use with centering arms 602. After stabilization with device 105, centering arms 602 can be extended in directions 611 to center or otherwise place combined device 110 in the desired position.

As discussed with respect to **FIG. 1****,** delivery device 104, stabilization device 105 and centering device 106 are each preferably used in conjunction with body member 101. Body member 101 can be configured in any manner desired in accordance with the needs of the application. **FIGs. 33A-B** are cross-sectional views depicting another exemplary embodiment of treatment system 100 where body member 101 includes two lumens 630 and 631. **FIG. 33A** is a longitudinal cross-sectional view and **FIG. 33B** is a radial cross-sectional view taken along line 33B-33B of **FIG. 33A****.** Preferably, lumen 630 is configured to slidably receive delivery device 104, while lumen 631 is configured to slidably receive either stabilization device 105 or an optional guidewire to facilitate routing body member 101 through the patient's vasculature. The guidewire can be placed in lumen 631 until body member 101 is in the desired position within the patient, at which time the guidewire can be removed and stabilization device 105 can be inserted. Also, centering device 106 is preferably integrated with stabilization device 105, such as in the embodiment described with respect to **FIG. 32D****,** in order to provide treatment system with both stabilization and centering capability. In order to prevent rotation of elongate body member 101 around stabilization device 105 during delivery, stabilization device is preferably fixably coupled with either body member 101 or delivery device 104.

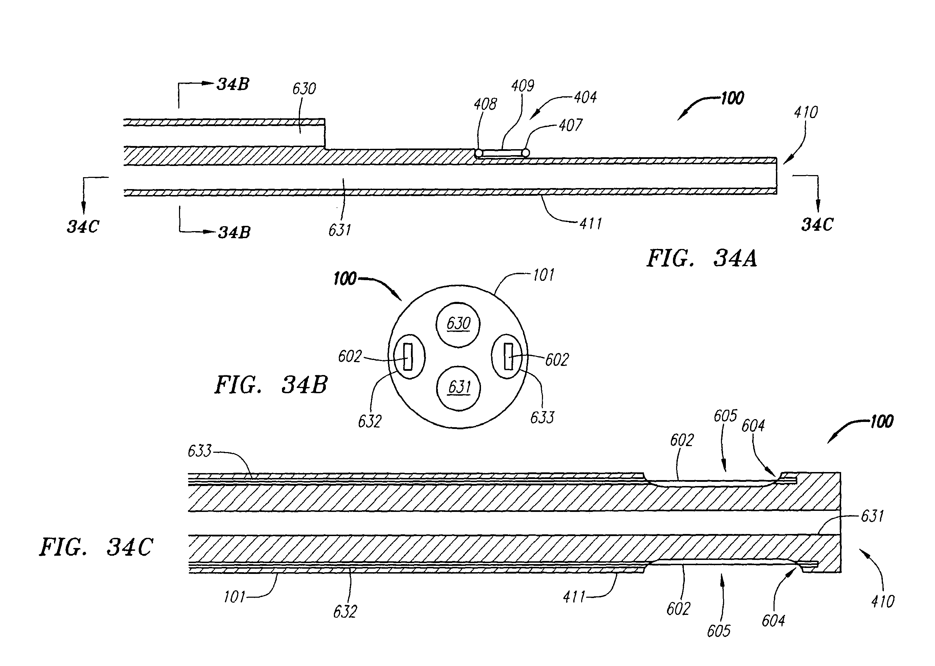

**FIGs. 34A-C** are cross-sectional views depicting another exemplary embodiment of treatment system 100 where body member 101 includes four lumens 630-633 as well as centering arms 602. Here, **FIG. 34A** is a first longitudinal cross-sectional view, **FIG. 34B** is a radial cross-sectional view taken along line 34B-34B of **FIG. 34A** and **FIG. 34C** is a second longitudinal cross-sectional view taken along line 34C-34C of **FIG. 34A****.** Preferably, lumen 630 is configured to slidably receive delivery device 104, while lumen 631 is configured for any purpose, including reception of stabilization device 105, a guidewire, dye infusion and the like. **FIG. 34B** depicts centering arms 602 within lumens 632-633 and **FIG. 34C** depicts centering arms 602 located within lumens 632-633, recessed portions 605 and seats 604. Here, recessed portions 605 and seats 604 are located distal to grasping device 404 on elongate support section 411. The distal portion of support section 411 can be placed within PFO tunnel 215 where centering arms 602 can be deflected for centering prior to deployment of implant 103 in left atrium.

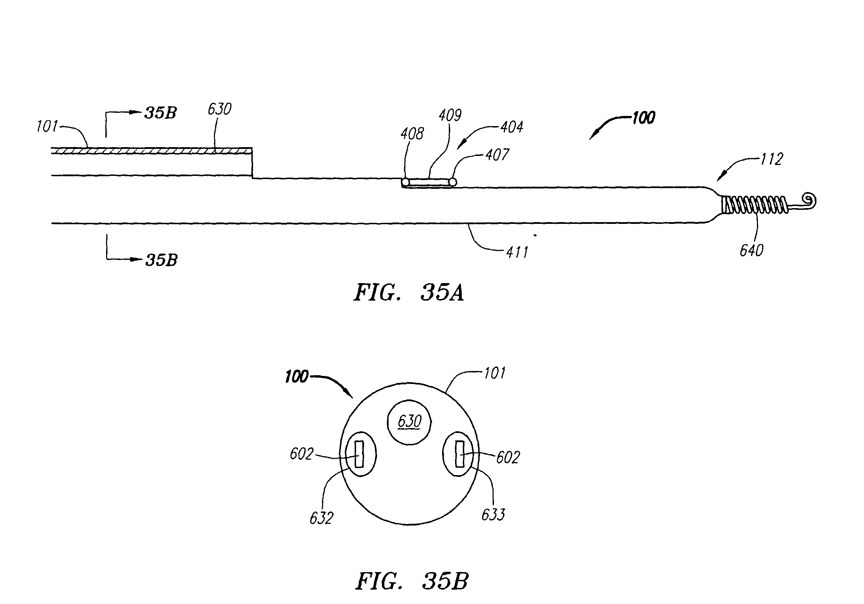

**FIGs. 35A-B** are cross-sectional views depicting another exemplary embodiment of treatment system 100 where body member 101 includes three lumens 630, 632 and 633 as well as centering arms 602. Here, **FIG. 35A** is a longitudinal cross-sectional view and **FIG. 35B** is a radial cross-sectional view taken along line 35B-35B of **FIG. 35A****.** In this embodiment, distal end 112 of body member 101 includes an atraumatic tip 640, which in this embodiment is a floppy tip. Here, with the aid of atraumatic tip 640, body member 101 is configured to be advanceable within the patient's vasculature without the aid of a guidewire. Accordingly, no additional lumen 631 is included for use with a guidewire. Also in this embodiment, stabilization device 105 has been optionally omitted, allowing body member 101 to achieve a relatively smaller radial cross-section size. In another exemplary embodiment, atraumatic tip 640 is omitted and body member 101 is configured to be slidably advanced through a tubular guide catheter placed within the patient's vasculature.

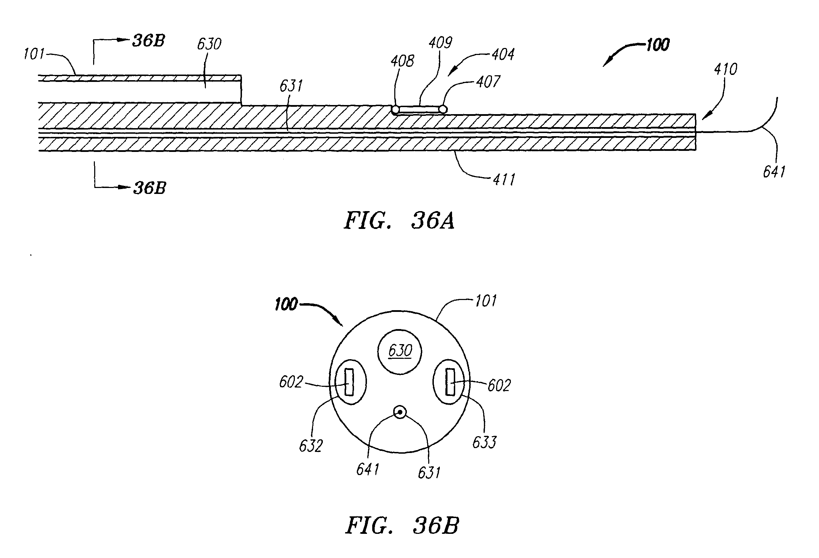

**FIGs. 36A-B** are cross-sectional views depicting another exemplary embodiment of treatment system 100 where body member 101 includes four lumens 630-633 as well as centering arms 602. Here, FIG. 36A is a longitudinal cross-sectional view and FIG. 36B is a radial cross-sectional view taken along line 36B-36B of FIG. 36A. This embodiment is similar to the embodiment described with respect to **FIGs. 34A-C** except here, lumen 631 is configured for use with guidewire 641 only, which can be the same size as or relatively thinner than stabilization device 105, allowing the radial cross-section size of lumen 631 and body member 101 to be reduced.

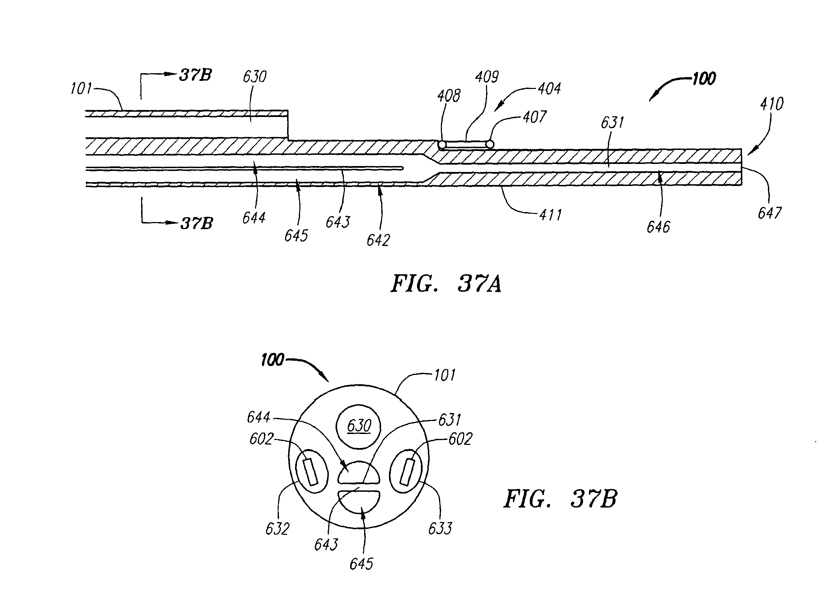

**FIGs. 37A-B** are cross-sectional views depicting another exemplary embodiment of treatment system 100 where body member 101 includes four lumens 630-633 as well as centering arms 602. Here, **FIG. 37A** is a longitudinal cross-sectional view and **FIG. 37B** is a radial cross-sectional view taken along line 37B-37B of FIG. 37A. This embodiment is similar to the embodiment described with respect to **FIGs. 35A-C** except here, lumen 631 is configured to facilitate exchange of stabilization device 105 and guidewire 641. Proximal portion 642 of lumen 631 includes a divider 643 to separate lumen 631 into a first portion 644 for stabilization device 105 and a second portion 645 for guidewire 641. Distal portion 646 of lumen 631 is preferably tapered to minimize the radial cross-section size of lumen 631. Exchange between stabilization device 105 and guidewire 641 is facilitated because both can reside within proximal portion 642 at the same time, with the desired one of stabilization device 105 or guidewire 641 being advanced distally through open distal end 647 for use.

It should be noted that in each of the embodiments described with respect to **FIGs. 33A-37B,** functionality can be added or removed as desired, while still remaining within the scope of treatment system 100. For instance, treatment system 100 can be further configured for dye infusion, pressure sensing, imaging, drug delivery, ablation, the use of occlusive devices such as balloons and stents, facilitating the implantation of coronary sinus pacing or defibrillation leads, the use of a stylet and the like. These and other additional types of functionality can be added in any manner, including, but not limited to the addition of one or more lumens 102, or the use of the existing lumens 102, integration directly into body member 101, or the addition of one or more extra body members 101.

In addition, treatment system 100 can include multiple delivery devices 104 for delivery of multiple implants 103, multiple stabilization devices 105 for stabilization on multiple tissue surfaces, multiple centering devices 106 and multiple body members 101 as desired. If treatment system 100 is used to access septal wall 207 via inferior vena cava 202, the maximum radial cross-section size of body member 101 is preferably 13 French or less, although it should be noted that any size body member 101 can be used in accordance with the needs of the application. Body member 101 can be constructed from any material as desired, but is preferably constructed from a flexible polymer such as polyethylene, polypropylene, nylon and the like.

Furthermore, it should be noted that any component or component portion within treatment system 100 can be configured to facilitate any type of imaging, including, but not limited to, internal and external ultrasound imaging, optical imaging, magnetic resonance imaging (MRI), and flouroscopy. For instance, radio-opaque portions can be used to increase the visibility in flouroscopic applications while echolucent coatings can be used to increase visibility in ultrasound applications. As an example, in one exemplary embodiment OA delivery member 401 can be entirely radio-opaque, or can include portions that are radio-opaque, such as on distal tip 430 of **FIG. 14A****.**

Also described herein are methods 700 and 800 of treating PFO tunnel 215, preferably by at least partially closing PFO tunnel 215. Methods 700 and 800 are preferably used with treatment system 100, but can be used with any medical system as desired. For ease of discussion, method 700 will be described with respect to treatment system 100 and method 800 will be described without reference to a particular treatment system, although it should be understood that methods 700 and 800 can be used with or without treatment system 100. Generally, the steps of methods 700 will vary, in part, on the actual configuration of implant 103, the number of implants 103 to be delivered, the location in which each implant 103 is to be delivered, the use of guidewire 641 or a guide catheter and the optional use of stabilization device 105 and/or centering device 106 or any combination thereof.

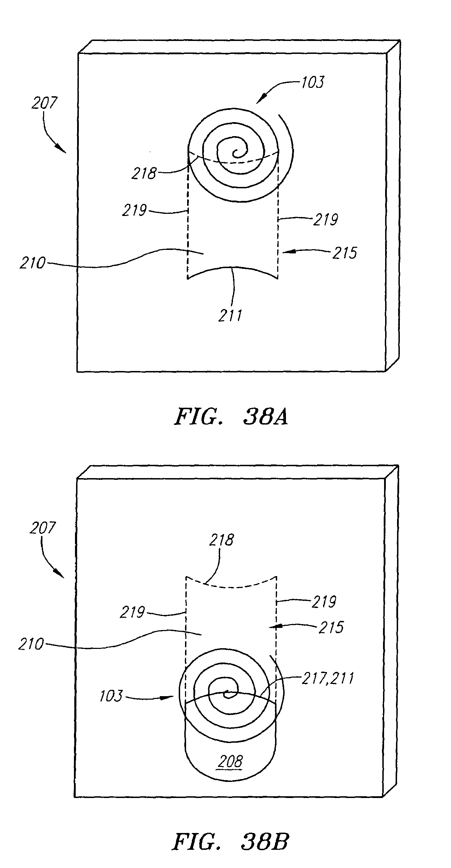

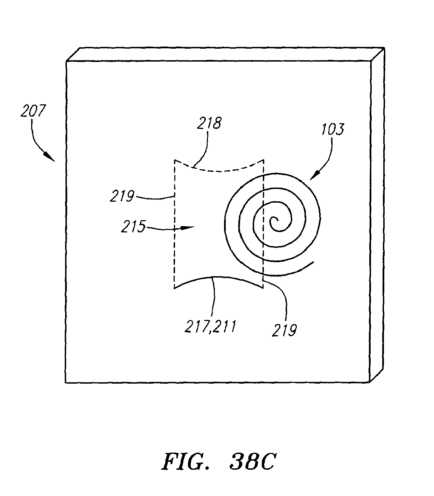

In **FIG. 4E****,** implant 103 is delivered through both septum primum 214 and septum secundum 210. It should be noted, however, that implant 103 can be delivered in any location desired. **FIGs. 38A-C** are cross-sectional views of septal wall 207 depicting exemplary embodiments of implant 103 in just several of the many alternate locations that can be used. In **FIG. 38A****,** implant 103 has been delivered through the upper portion of septum secundum 210 adjacent to PFO exit 218. In **FIG. 38B****,** implant 103 has been delivered through the lower portion of septum primum 214, adjacent to PFO entrance 217 and near (or in) fossa ovalis 208. In **FIG. 38C****,** implant 103 has been delivered through septal wall 207 adjacent to sidewall 219, septum primum 214 and septum secundum 210.

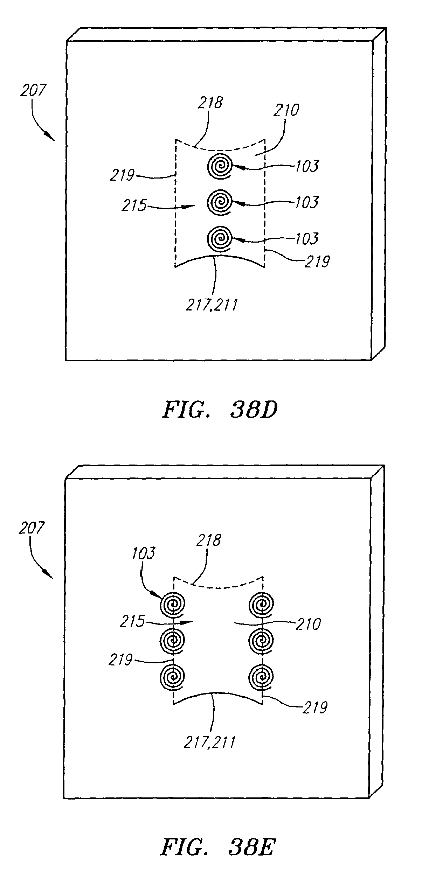

Also, as many implants 103 can be used in any arrangement as desired. **FIGs. 38D-E** are views of septal wall 207 depicting exemplary embodiments of multiple implants 103 in just several of the many alternate arrangements that can be used. In **FIG. 38D****,** three implants 103 have been delivered through both septum primum 214 and septum secundum 210. In **FIG. 38E****,** six implants 103 have been delivered through septal wall 207 adjacent to both sidewalls 219, septum primum 214 and septum secundum 210.

Although there are many different implementations and variations of method 700, for ease of discussion, method 700 will be described herein as using one implant 103, delivered through both septum primum 214 and septum secundum 210, using an exemplary embodiment of treatment system 100 similar to that described above with respect to **FIGs. 33A-B,** where body member 101 is configured for use with stabilization device 105 having centering device 106 integrated thereon.

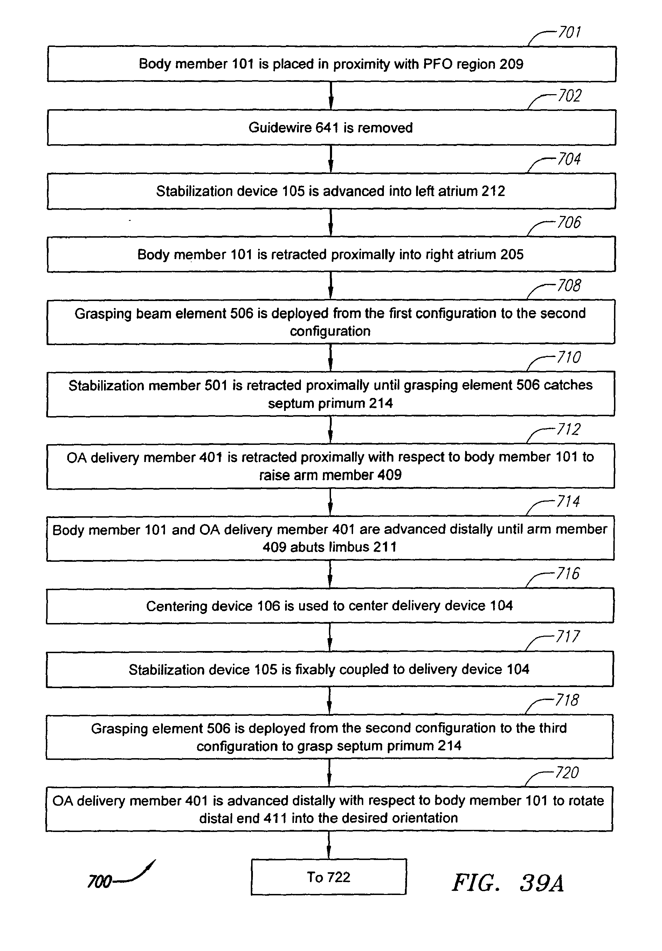

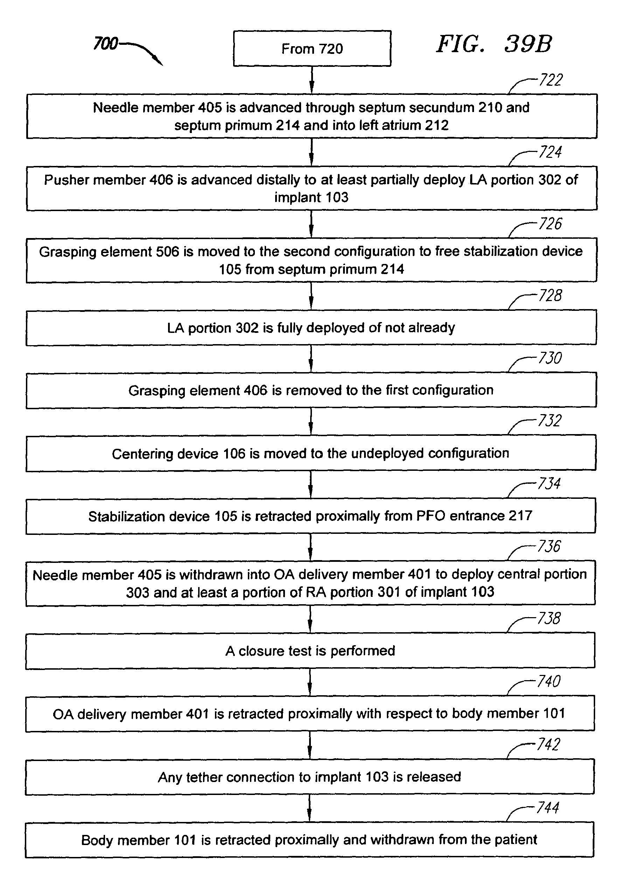

**FIGs. 39A-B** are flow diagrams depicting an example of method 700. First, at 701, body member 101 is placed in proximity with PFO region 209. As mentioned above, implant 103 can be delivered from left atrium 212 or right atrium 205. Preferably, implant 103 is placed into proximity with PFO region 209 by advancing body member 101 from the femoral vein to right atrium 205 in a conventional manner. For instance, in one example, a needle is inserted into the femoral vein and a guidewire is advanced through the needle into the femoral vein. The needle can then be removed and an access sheath can be routed over the guidewire, which can also then be removed. A J-tip guidewire, such as a 0.035"/0.038" guidewire, can be routed through the patient's vasculature into inferior vena cava 202 and right atrium 205. From there, the guidewire can be routed through PFO tunnel 215 and into left atrium 212. Next, an exchange sheath or multi-purpose guide can then be advanced over the J-tip guidewire into left atrium 212, at which point the J-tip guidewire can be removed. A relatively stiffer guidewire 641 can then be advanced through the exchange sheath or multi-purpose guide and into left atrium 212 and optionally the pulmonary vein, which can act as an anchor for the guidewire. Body member 101 can then be advanced over the guidewire 641 into proximity with PFO region 209, preferably through PFO tunnel 215 and into left atrium 212. In addition, a catheter or guidewire having a sizing device, such as a balloon, can be placed within PFO tunnel 215 to measure the size of PFO tunnel 215, for use in choosing a placement location, implant size, etc.

At 702, guidewire 641, if present, can be removed. At 704, stabilization device 105 is preferably advanced through lumen 631 and into left atrium 212. At 706, body member 101 can be retracted proximally into right atrium 205. Preferably, stabilization device 105 includes a stabilization member 501 and grasping device 502 with grasping element 506. At 708, grasping element 506 can be deployed from the first housed configuration to the second configuration for catching tissue, which, in this example, is preferably septum primum 214.

Next, at 710, stabilization member 501 is preferably moved distally until grasping element 506 catches septum primum 214. Then, at 712, OA delivery member 401 can be retracted proximally with respect to body member 101 to raise arm member 409. At 714, body member 101 and OA delivery member 401 are advanced distally until arm member 409 abuts limbus 211. At 716, centering device 106 can be used to center delivery device 104, preferably by deflecting centering arms 602. Once centered, if not already done so, at 717 stabilization device 105 can be fixably coupled to delivery device 104 (e.g., with a rotating hemostasis valve or Tuohy-Borst valve and the like). Next, at 718, grasping element 506 can be further deployed to the third configuration to grasp septum primum 214 and lock stabilization device 105 to septum primum 214. Alternatively, either 716, 717, 718 or any combination thereof can be implemented prior to 712. Also, 716-718 can be implemented in any order desired with respect to each other.

Once stabilized, centered and locked in place, OA delivery member 401 is preferably advanced distally with respect to body member 101 to rotate distal end 410 into the desired orientation with surface 320 of septum secundum 210. At 722, needle member 405 can be advanced through septum secundum 210 and septum primum 214 and into left atrium 212. Then, at 724, pusher member 406 can be advanced distally to at least partially deploy LA portion 302 of implant 103 from distal end 415 of needle member 405. In embodiments where centering arms 602 are in their deflected state for centering, it is possible for needle member 405 to pass between centering arms 602 and stabilization member 501 when inserted, based on needle insertion location 419. To avoid capture of implant 103 between centering arms 602 and stabilization member 501, centering arms 602 can be retracted proximally back into elongate body 101 thereby removing them from seats 604 and preventing implant 103 from being trapped between centering arms 602 and stabilization member 501. Next, at 726, grasping element 506 can be moved to the second configuration to free stabilization device 105 from septum primum 214. Alternatively, 726 can be performed before 724 if desired.

Then, at 728, LA portion 302 can be fully deployed if not already. At 730, grasping element 506 can be removed to the first configuration, housed within stabilization member 501. Next, at 732, centering device 106 can be moved to the undeployed configuration if not already, preferably by collapsing centering arms 602, after which stabilization device 105 can be retracted proximally from PFO entrance 217 at 734. At 736, needle member 405 can be withdrawn into OA delivery member 401 to deploy central portion 303 of implant 103 and at least a portion of RA portion 301. Here, at 738, an optional closure test can be performed to confirm at least partial closure, and preferably substantially complete closure, of PFO tunnel 215. Any desired closure test can be performed including, but not limited to, the introduction of gaseous bubbles simultaneously with imaging using contrast enhanced trans-cranial doppler (CE-TCD), intracardiac echocardiography (ICE) and the like, or the infusion of a radio-opaque dye imagable via flouroscopy. The test may be performed by pulling back OA delivery member 401 as far as necessary to deploy RA coil 301 and then test while device is at PFO entrance.

At 740, OA delivery member 401 can be retracted proximally with respect to body member 101 to complete deployment of RA portion 301, release limbus 211 and place OA delivery member 401 in the original position. If the desired degree of closure is confirmed, then any tether connection to implant 103 can be released at 742. Finally, at 744, body member 101 can be retracted distally and withdrawn from the patient.

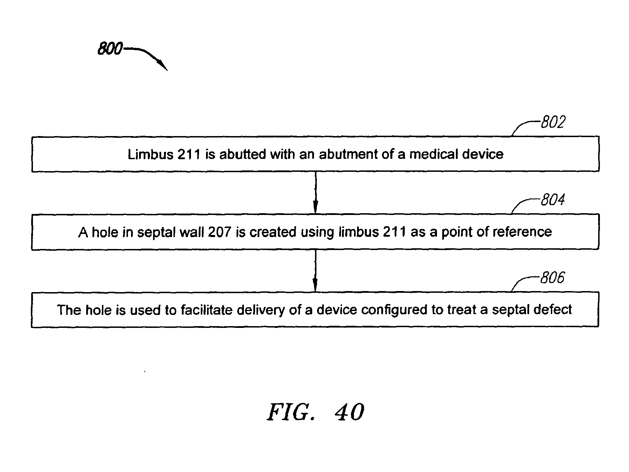

**FIG. 40** depicts another exemplary method 800 of treating a septal defect. At 802, limbus 211 is abutted with an abutment of a medical device. Preferably, limbus 211 is engaged with the medical device and optionally grasped such that the medical device is anchored to limbus 211. Then, at 804, a hole in septal wall 207, preferably in septum secundum 210, is created using limbus 211 as a point of reference. For instance, the hole can be created at a fixed or adjustable distance from limbus 211. At 806, the hole is used to facilitate delivery of a device configured to treat a septal defect. In one example, the device is deployed through the hole such that it causes at least partial closure of the septal defect. In this example of method 800, limbus 211 is abutted and used as a reference. In another example of method 800, the edge of septum primum 214 is abutted and used as a reference. In other examples of method 800, one or both sidewalls 219 and/or fossa ovalis 208 are abutted and used as points of reference.

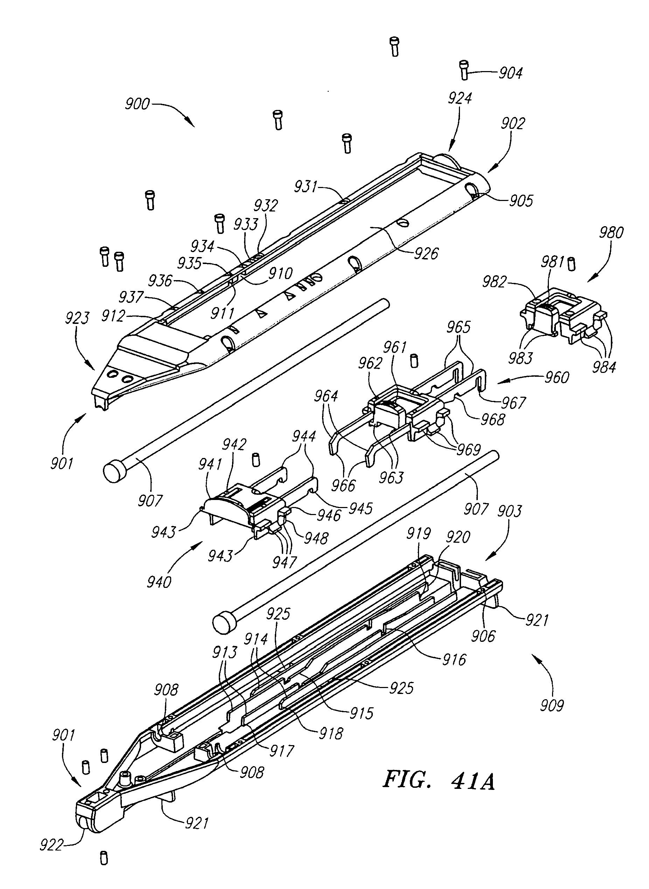

Control of system 100 can be accomplished with the use of a proximal control device, or proximal controller, 900. **FIG. 41A** is an exploded view depicting an exemplary embodiment of a proximal control device 900. In this embodiment, proximal controller 900 is preferably used to control delivery device 104 when configured for off-axis delivery, for example, in embodiments where delivery device 104 is configured in a manner similar to that described with respect to **FIGs. 14A-F.** Proximal controller is shown here in a preferred upright position. To facilitate description of the location of the various elements of controller 900, reference will be made to elements being above or beneath other elements, referring to their respective locations when controller 900 is oriented as shown in **FIG. 41A****.**

Although not limited to such, proximal controller 900 will be described in the context of use with an embodiment of body member 101 and delivery device 104 similar to that described with respect to **FIGs. 14A-F.** Like the embodiment described with respect to **FIGs. 14A-F,** delivery device 104 includes OA delivery device 401, needle member 405 and pusher member 406. However, this embodiment does not include stabilization device 105 or centering device 106, although proximal controller 900 can certainly be configured to control those devices as well.

In the embodiment depicted in **FIG. 41A****,** proximal controller 900 includes a housing 901 divided into two parts, an upper portion 902 and a lower portion 903, which are preferably coupled together. Portions 902 and 903 can be coupled together in any manner. Here, portions 902 and 903 are coupled together with a plurality of screws 904 that are routed through apertures 905 in upper portion 902 and interface with threaded chambers 906 within portion 903. Housing 901 also has a distal end 923 and a proximal end 924. Distal end 923 is preferably fixably coupled with body member 101.

Proximal controller 900 includes two guide rails 907 and a user interface 909 including three slidable actuators 940, 960, and 980 configured to slide along guide rails 907. Guide rails 907 are preferably rigid members with a smooth surface to allow for low surface frictional resistance to the movement of actuators 940, 960, and 980. When portions 902 and 903 are coupled together, guide rails 907 are preferably held in place by restraining seats 908 located in both portions 902 and 903 (seats 908 are obscured and not shown in upper portion 902). Also, actuators 940, 960, and 980 are maintained sequentially within housing 901 and can be controllably moved, or slid, along guide rails 907.

In this embodiment, control of each actuator 940, 960, 980 is accomplished by way of depressible buttons 941, 961 and 981, respectively. Access to actuators 940, 960 and 980 is achieved through opening 926 in upper housing portion 902. One of skill in the art will readily recognize that other forms of controlling actuators 940, 960, 980 can be used.

Each of actuators 940, 960, 980 is preferably coupled with a portion of delivery device 104. In this embodiment, actuator 940 is coupled with OA delivery member 401, actuator 960 is coupled with needle member 405 and actuator 980 is coupled with pusher member 406. To facilitate the description herein, actuator 940 will be referred to as OA actuator 940, actuator 960 will be referred to as needle actuator 960 and actuator 980 will be referred to as pusher actuator 980. Of course, any of actuators 940, 960, and 980 can be coupled with any portion of delivery device 104, or any other portion of system 100, as desired.

Preferably, proximal controller 900 is configured such that the movement of actuators 940, 960, and 980 with respect to each other can be controlled, or guided, at appropriate stages during an implantation procedure. At certain stages, movement of the various actuators 940, 960, and 980 is fully independent of the positions of one or more of the remaining actuators 940, 960, and 980. Conversely, at certain stages, movement of the various actuators 940, 960, and 980 is dependent on the positions of one or more of the remaining actuators 940, 960, and 980 and movement can be restricted to certain directions or prevented entirely. Also, controller 900 is preferably configured such that the movement of actuators 940, 960, 980 with respect to the anatomy of the subject can be controlled, or guided, at appropriate stages during the procedure. These features can reduce the risk that the user improperly operates system 100 while within the body of the subject, such as by prematurely releasing implant 103.

In this embodiment, control is also provided by a network of mechanical tabs, slots, abutments, surfaces and/or ribs which can act in conjunction to control and lock the movement of each actuator 940, 960 and 980. Before describing the operation of controller 900, each portion of controller 900 will be described in greater detail.

Upper housing portion 902 includes three slots 910, 911 and 912 (shown here partially obscured) located on both sides of opening 926. Housing portion 902 also includes multiple guide markings 931-937 which can correspond to one of guide markings 942, 962 and 982 located on each of actuators 940, 960 and 980, respectively. In this embodiment, guide markings 931-932 have a circular shape and correspond to circular marking 982 on pusher actuator 980, guide markings 935-936 have a triangular shape and correspond to triangular marking 962 on needle actuator 960, and guide markings 933, 934, and 937 have a rectangular shape and correspond to rectangular marking 942 on OA actuator 940.

Lower housing portion includes two sets of ribs, inner ribs 913 and outer ribs 914. Ribs 913-914 extend upwards from the base of lower housing portion 903. Inner ribs 913 each include two slots 915 and 916. The distal ends 917 of ribs 913 are located distal to the distal ends 918 of ribs 914. The proximal ends 919 of ribs 913 are also located distal to the proximal ends 920 of ribs 914. Located beneath and to the outside of ribs 914 are a set of abutments 925 for abutting OA actuator 940.

An aperture 922 is located at the distal end of lower housing portion 903 and is configured to allow routing of body member 101 therethrough. Lower housing portion 903 also includes a base 921 upon which it can rest and remain stable during the implantation procedure.

OA actuator 940 includes a set of outwardly extending tabs 943 located at the base of button 941. OA actuator 940 also includes two proximally located rails 944 each having two similarly shaped slots 945 and 946 (not shown) located therein. Slot 945 is located proximal to slot 946 and both are located in the bottom portion of rails 944. On both sides of OA actuator 940 are a set of guide rail abutments 947 that facilitate, or guide, the movement of OA actuator 940 along each guide rail 907. Below guide rail abutments 947 on each side is a proximally located tab 948 for abutting abutments 925.

Needle actuator 960 includes a set of outwardly extending tabs 963 located at the base of button 961. Needle actuator 960 also includes two distally located rails 964 and two proximally located rails 965. The distal end of each distal rail 964 includes a downwardly oriented chamfer 966, which can be used to force OA actuator 940 into a locked position in the case where the user has not fully done so. Distal rails 964 are spaced apart at a greater distance than proximal rails 944 (on OA actuator 940) to allow both sets of rails 944 and 964 to slide distally and proximally in a relatively unimpeded manner. OA proximal rails 944 are aligned with tabs 963 on needle actuator 960 and are configured to interact with tabs 963. Needle actuator 960 is configured to slide along rails 944 with tabs 963 in position to interact with slots 945-946. Likewise, OA actuator 940 is also configured to slide along needle actuator rails 964 and to abut chamfer 966 if needed.

Needle actuator proximal rails 965 each include two slots 967 and 968, both of which are located in the bottom portion of rails 965. The proximal surfaces of slots 967 extend further downwards than the other surfaces on rails 965 to provide a locking function that will be described in more detail below. On either side of needle actuator 960 are a set of guide rail abutments 969 that facilitate, or guide, the movement of needle actuator 960 along each guide rail 907.

Pusher actuator 980 includes a set of outwardly extending tabs 983 located at the base of button 981. Tabs 983 are aligned with needle proximal rails 965 and are configured to interact with slots 967-968. Pusher actuator 980 is also configured to slide over proximal rails 965 to allow the interaction of tabs 983 with slots 967-968. On either side of pusher actuator 980 are a set of guide rail abutments 984 that facilitate, or guide, the movement of pusher actuator 980 along each guide rail 907.

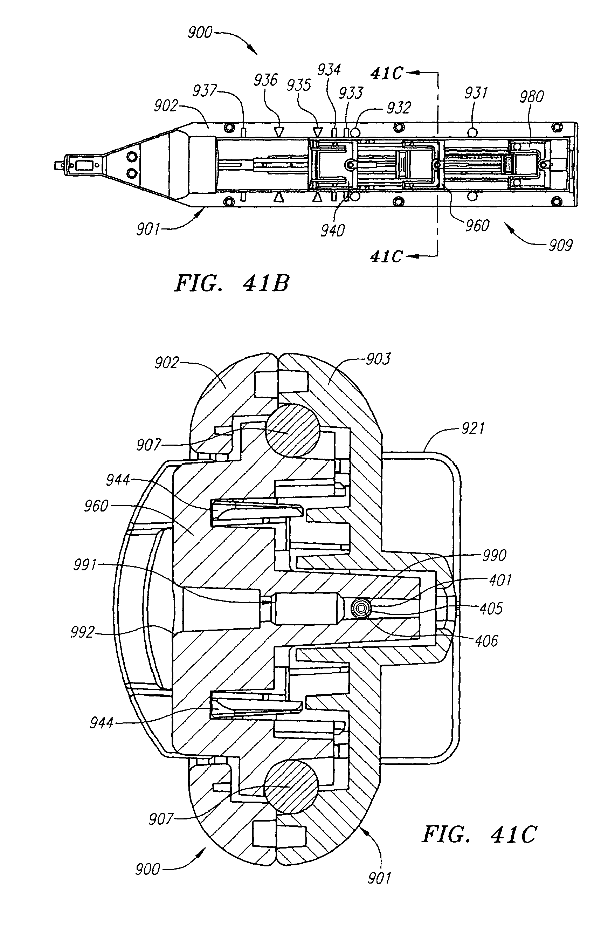

FIG. 41B is a top down view depicting this exemplary embodiment of controller 900 in an assembled state. Here, each actuator 940, 960 and 980 is shown in a position within housing 901. **FIG. 41C** is a cross-sectional view of controller 900 taken along line 41C-41C of **FIG. 41B****.** This cross-sectional view depicts needle actuator 960 within housing 901, in addition to needle member 405 with pusher member 406.

Here, needle member 405 is coupled with and surrounded by a sleeve 990, which is preferably formed of a rigid material, such as stainless steel and the like, and preferably smooth to decrease surface friction. A set screw 991 is adjustably located above sleeve 990 in a slot 992 within needle actuator 960. Set screw 991 is preferably adjusted and brought into contact with sleeve 990 to lock sleeve 990 in place within needle actuator 960. One of ordinary skill in the art will readily recognize that any technique can be used to lock sleeve 990 with needle member 405, or otherwise couple needle member 405 with needle actuator 960, including, but not limited to, bonding, welding, clamping, crimping, and the like.

Likewise, OA delivery member 401 and pusher member 406 are also both preferably coupled with their respective actuators 940 and 980, using similar sleeves in combination with set screws. One of skill in the art will readily recognize that numerous different techniques, including adhesives, welding, soldering, mechanical couplings and the like, can be used to lock each actuator 940, 960, and 980 with the respective component of system 100, in this case OA delivery member 401, needle member 405 and pusher member 406.

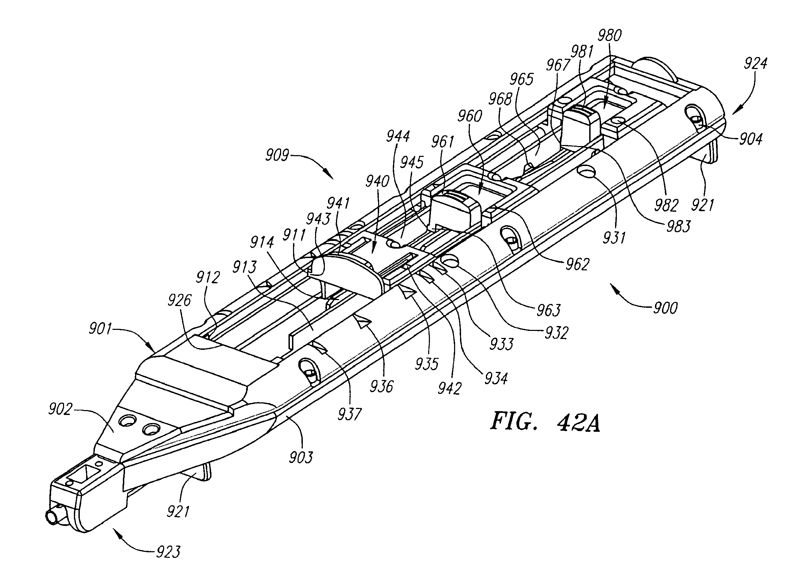

Turning now to the use of controller 900, an exemplary method of operating controller 900 is described with the aid of **FIGs. 42A-I.** **FIGs. 42A-I** are perspective views depicting an exemplary embodiment of controller 900 with actuators 940, 960 and 980 in various positions during the implantation procedure. Because various components of controller 900 can become obscured in the various views and because all components are labeled in **FIG. 41A****,** only visible components are labeled in **FIGs. 42A-I.**

In **FIG. 42A****,** each of actuators 940, 960, and 980 are shown in start positions, which are suitable positions to be maintained during advancement of body member 101 through the vasculature and into proximity with septal wall 207, preferably within right atrium 205. Here, guide marking 942 on OA actuator 940 is aligned with guide marking 934 on upper housing 902 and tabs 943 on OA actuator 940 are located within slots 911 in upper housing 902. When tabs 943 are located within any of slots 910-912 of upper housing 902, OA delivery device 401 is effectively locked in position with respect to body member 101, which is preferably fixably coupled with housing 901.

Also in this position, tabs 963 on needle actuator 960 are located within slots 945 within OA proximal rails 944. Depression of needle button 961 in this position is prevented by outer ribs 914, which abut tabs 963. This effectively locks actuator 960 in position with respect to OA actuator 940. With regards to pusher actuator 980, tabs 983 are located within slots 967 within needle proximal rails 965. Depression of needle button 981 in this position is prevented by inner ribs 913, which abut tabs 983, effectively locking pusher actuator 980 in position with respect to needle actuator 960, which in turn is locked in position with respect to OA actuator 940. Thus, here, the position of needle actuator 960 and pusher actuator 980 is locked with respect to OA actuator 940 and follows the movement of OA actuator 940.

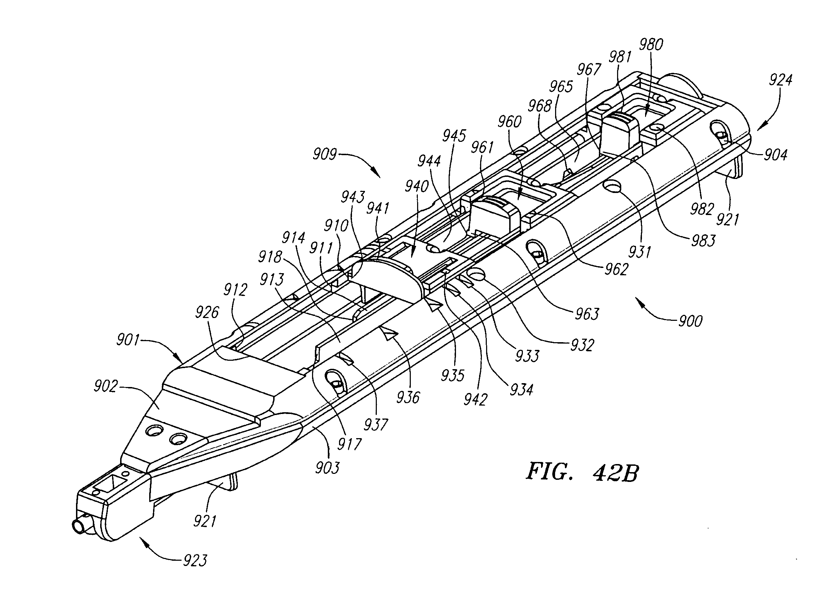

In **FIG. 42B****,** button 941 on OA actuator 940 has been depressed to disengage tabs 943 from slots 911 and allow the proximal transitioning of OA actuator 940 to the position depicted here, at which point button 941 has been released. This raises and proximally moves OA delivery member 401 to raise arm member 409 and place it in position to engage limbus 211, similar to the orientation depicted in **FIG. 14D****.** Here, OA guide marking 942 is aligned with guide marking 933 on housing 902 and OA tabs 943 are located within slots 910 in upper housing 902. OA button 941 remains depressible but the user is prevented from transitioning OA actuator 940 any further proximally than this position by the contact of tabs 948 with abutments 925 on housing portion 903.

Needle actuator 960 and pusher actuator 980 have been transitioned to positions slightly proximal that of the previous position, and remain locked in place with respect to OA actuator 940. Thus, the relative positions of needle member 405 and pusher member 406 have remained locked in place relative to OA delivery member 401, and both needle member 405 and pusher member 406 have been retracted within the subject's anatomy in lockstep fashion with OA delivery member 401. The device is then advanced distally to abut the limbus.

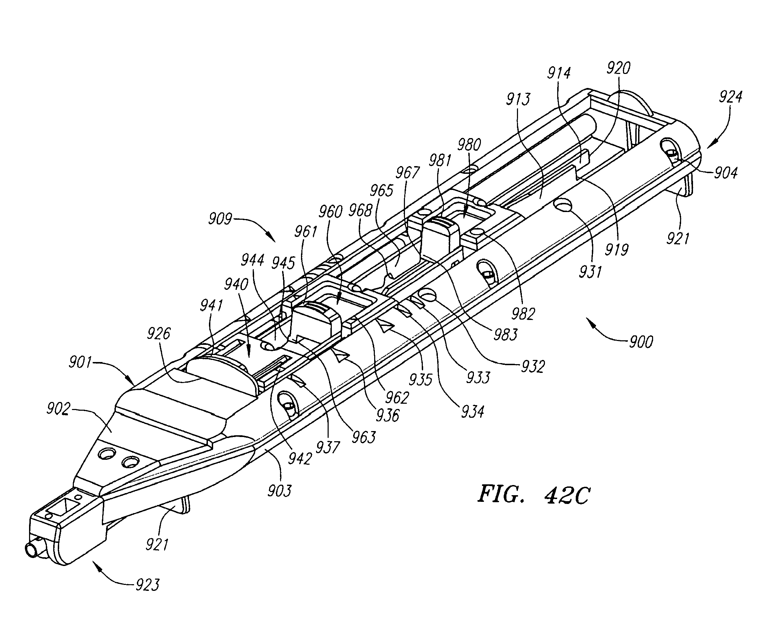

In FIG. 42C, OA actuator 940 has been transitioned distally to advance OA delivery member 401 into contact with septum secundum 210, causing arm member 409 to engage limbus 211 and positioning OA delivery member 401 into an off-axis delivery orientation, similar to the orientation depicted in FIG. 14F. At this point, body member 101 is preferably fixably coupled with the anatomy of the subject by way of grasping device 404. If, during this time, any of actuators 940, 960, and 980 are locked with respect to body member 101, for instance, by locking directly with housing 901 (e.g., OA tabs 943 in slots 910-912) or by locking with OA actuator 940 while locked with housing 901 (e.g., needle tabs 963 in OA slots 945 or pusher tabs 983 in needle slots 968 when needle actuator 960 is locked with respect to OA actuator 940), then that actuator 940, 960, and/or 980 also becomes locked with respect to the anatomy of the subject.

In the position of **FIG. 42C****,** OA guide marking 942 is aligned with guide marking 937 on upper housing 902 and OA tabs 943 are located within slots 912 in upper housing 902. OA button 941 remains depressible but the user is prevented from transitioning OA actuator 940 any further distally than this position by the contact of button 941 with the distal surface of opening 926 on housing portion 902.

Needle actuator 960 and pusher actuator 980 remain locked in position with respect to OA delivery member 401 and have been transitioned to positions distal that of the previous position. Needle button 961 is now depressible because tabs 963 are located distal to distal ends 918 of outer ribs 914. If the user depresses needle button 961, proximal travel of needle actuator 960 is prevented by the proximal surface of slot 945 (which extends further downwards than the distal surface of slot 945) and distal end 918 of outer rib 914, which abut tabs 963. Pusher actuator 980 remains locked in place with respect to OA actuator 940 and needle actuator 960. If a guidewire is being used, it is preferably removed prior to proceeding to the next step.

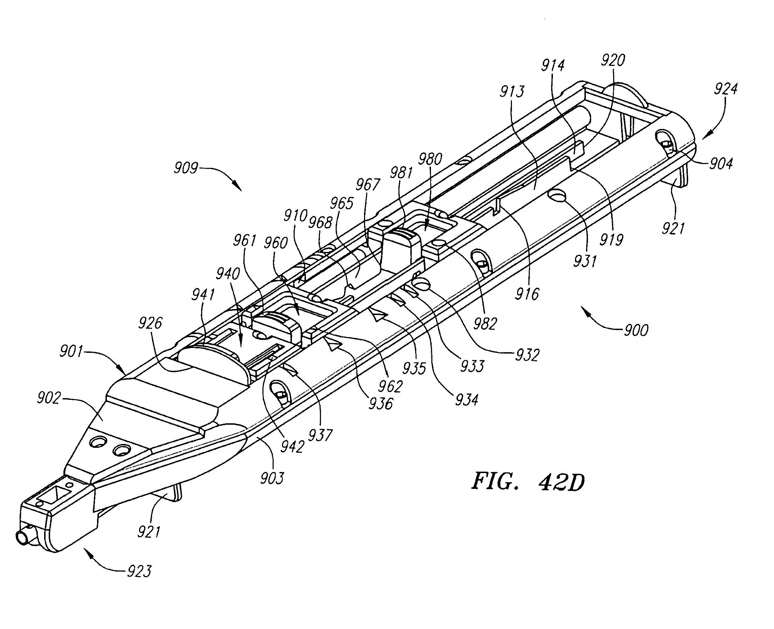

In **FIG. 42D****,** needle actuator 960 has been transitioned distally to advance needle member 405 out of OA delivery member 401 and through septal wall 207, preferably through both septum secundum 210 and septum primum 214. Here, needle guide marking 962 is aligned with guide marking 936 on upper housing 902 and needle tabs 963 are located within slots 946 in OA proximal rails 944. Needle button 961 remains depressible but the user is prevented from transitioning needle actuator 960 any further distally than this position by the presence of OA actuator 940, which remains in the same position as in **FIG. 42C****.** This prevents the user from inadvertently advancing needle member 405 too far into left atrium 212 and causing unwanted tissue damage. Needle distal rails 964 are now located beneath OA tabs 943 and prevent depression of OA button 941, preventing both distal and proximal movement and effectively locking OA actuator 940 in place.

It should be noted that proximal controller 900 can also be configured to automatically advance needle member 405 by the desired amount. For instance, needle member 405 can be spring loaded such that movement of needle actuator 960 to a certain position releases the spring, which provides force sufficient to advance needle member 405 through septal wall 207. Of course, one of skill in the art will readily recognize that other techniques for automatically advancing needle member 405 can be implemented and, accordingly, the systems and methods described herein are not limited to spring-based techniques.

Pusher actuator 980 has been transitioned with needle actuator 960 to a position distal that of the previous position. Specifically, pusher tabs 983 are now located over top of slot 915 in inner ribs 913, enabling the depression of pusher button 981. If the user depresses pusher button 981, proximal travel of pusher actuator 980 is prevented by the proximal surface of slot 967, which extends further downwards than the distal surface of slot 967. Preferably, button 981 is not depressible far enough to force tabs 983 below the bottommost portion of the proximal surface of slots 967, effectively preventing proximal movement of pusher actuator 980.

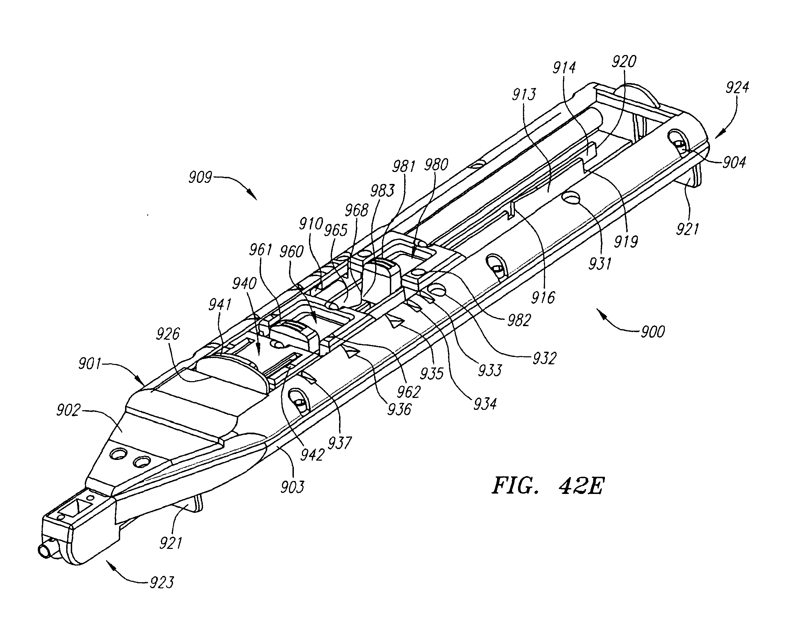

In FIG. 42E, pusher actuator 980 has been transitioned distally to advance LA portion 302 of implant 103 out of needle member 405, which, depending on the specific embodiment of implant 103, allows LA portion 302 to expand within left atrium 212. Here, pusher guide marking 982 is aligned with guide marking 932 on upper housing 902 and pusher tabs 963 have been advanced to the distal end of slots 915 within inner ribs 913 and into slots 968 in needle proximal rails 965. Pusher button 981 remains depressible but the user is prevented from transitioning pusher actuator 980 any further distally than this position by the pusher tabs 963 hitting distal surface of slots 915. As an additional safeguard, distal movement is also prevented by the distal surface of slot 968 in needle proximal rails 965. This distal surface acts in conjunction with inner ribs 913 to block tabs 983 from being advanced and prevent further distal movement of pusher actuator 980. OA actuator 940 and needle actuator 960 remain the same as described with respect to FIG. 42D.

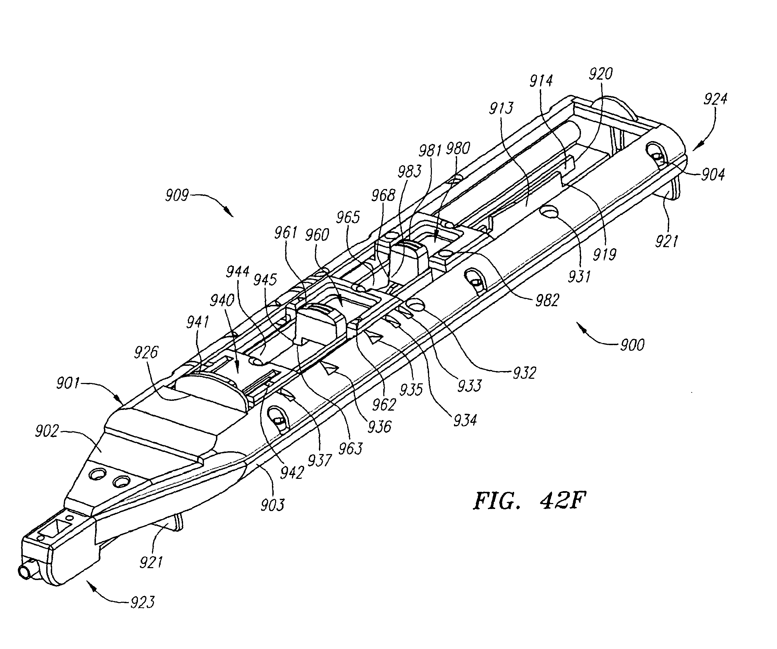

In FIG. 42F, needle actuator 960 has been transitioned proximally to retract needle member 405 from left atrium 212 and back into OA delivery member 401, which preferably pulls LA portion 302 of implant 103 into contact with septum primum 214. Here, needle guide marking 962 is aligned with guide marking 935 on upper housing 902 and needle tabs 963 are located within slots 945 in OA proximal rails 944. Needle button 961 remains depressible but the user is prevented from transitioning needle actuator 960 any further proximally by the proximal surface of slots 945 in OA proximal rails 944. Needle distal rails 964 are no longer beneath tabs 943 and OA button 941 is again depressible.

Pusher actuator 980 remains locked in place with respect to needle actuator 960 and has been transitioned with needle actuator 960 to a position proximal that of the previous position. Specifically, pusher tabs 983 remain within slots 968 but are now located over inner ribs 913 at a position proximal that of slots 915, preventing the depression of pusher button 981 and effectively locking pusher actuator 980 in place with respect to needle actuator 960.

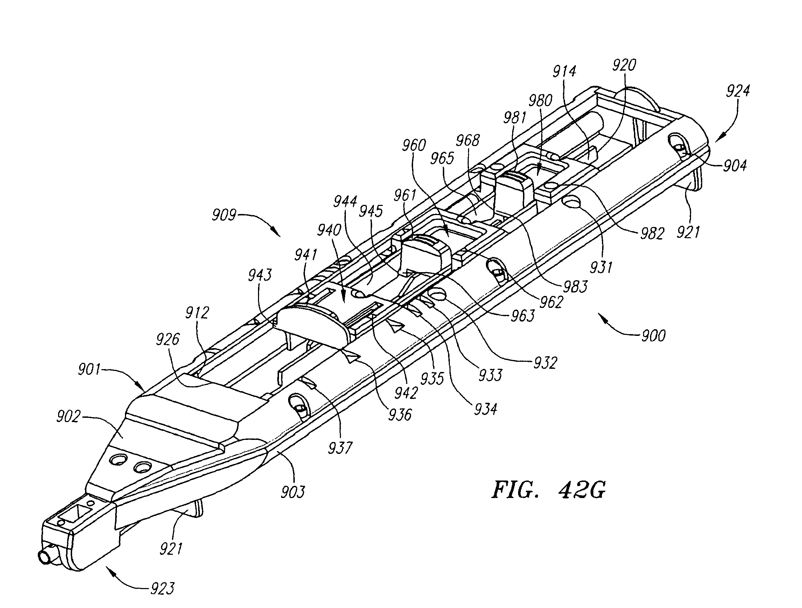

In **FIG. 42G****,** OA actuator 940 has been transitioned proximally to retract OA delivery member 401, removing OA delivery member 401 from the off-axis delivery orientation. Here, OA guide marking 942 is not aligned with any guide marking on upper housing 902 and OA tabs 943 have not yet become seated within any slots in upper housing 902, leaving OA button 941 held in a depressed position by the surface of upper housing 902. Needle actuator 960 and pusher actuator 980 both remain locked in position with respect to OA actuator 940 and move proximally with OA actuator 940 until tabs 983 on pusher actuator 980 contact the proximal surface of slot 916 in inner ribs 913.

In this embodiment, the proximal surface of slot 916 extends further upwards than any other surface on inner ribs 913 and acts to block further travel of actuators 940, 960, and 980. This creates a stopping point in the operation of the device immediately prior to full deployment of implant 103, which, among other things, can allow the user time to image the subject to ensure implant 103 is positioned as desired. Needle button 961 is not depressible at this point due to the presence of outer ribs 914, effectively locking tabs 963 in place within slots 945 on OA proximal rails 944. Pusher button 981 is depressible as tabs 983 are now located over slots 916 in inner ribs 913, although movement in the distal and proximal directions is prevented by the contact of tabs 983 with slots 916. Pusher guide marking 982 is preferably aligned with marking 931 on upper housing 902.

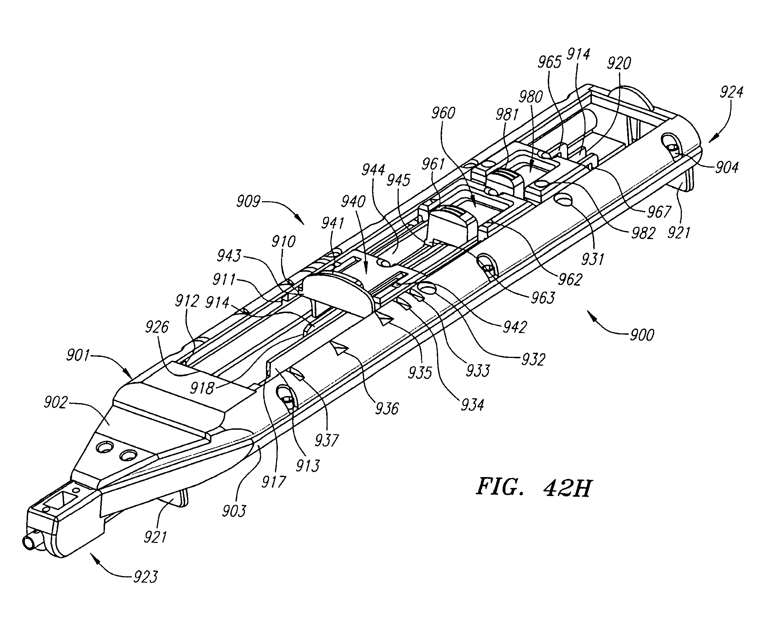

In **FIG. 42H****,** pusher button 981 has been depressed to unlock pusher actuator 980 from needle actuator 960, specifically to unlock tabs 983 from slots 968, allowing OA actuator 940 and needle actuator 960 to be transitioned further proximally. This retracts OA delivery member 401 and needle member 405 with respect to pusher member 406, causing OA delivery member 401 to raise up and disengaging arm member 409 from limbus 211. This also fully exposes implant 103 from within both needle member 405 and OA delivery member 401 and allows RA portion 301 to expand and engage septum secundum 210 (connection to implant 103 may be maintained via the use of a safety device such as a tether and the like).

In this position, OA guide marking 942 is aligned with guide marking 933 on upper housing 902 and OA tabs 943 are seated within slots 910 in upper housing 902. OA button 941 remains depressible but the user is prevented from transitioning OA actuator 940 any further proximally than this position by the contact of tabs 948 with abutments 925 on housing portion 903. Needle actuator 960 remains locked in position with respect to OA actuator 940 and moves proximally with OA actuator 940. Needle button 961 is not depressible due to the outer ribs 914 and is effectively locked in place within slots 945 of OA proximal rails 944. Pusher actuator 980 remains locked in the same position as that depicted in **FIG. 42G****,** although tabs 983 are now located distal to slots 968.

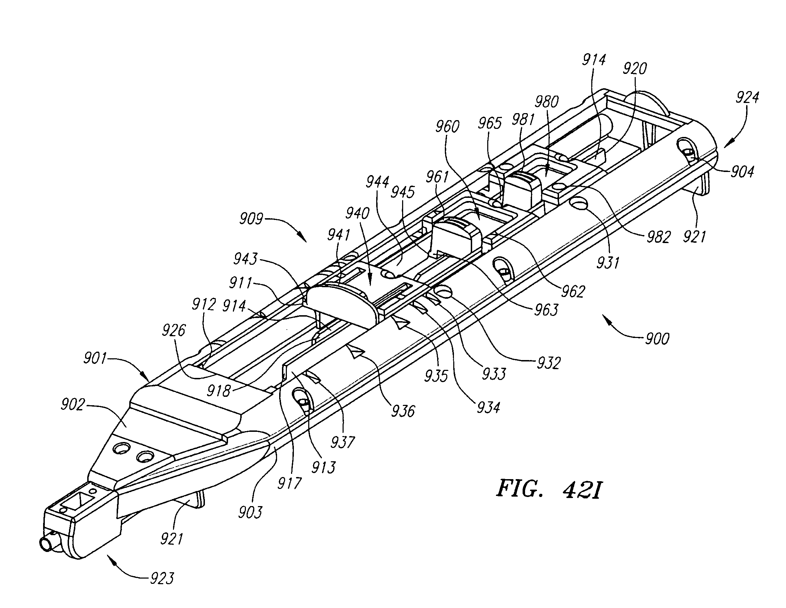

In **FIG. 42I****,** OA actuator 940 has been transitioned distally to lower OA delivery member 401 into the low profile configuration desired for removal of system 100 from within the subject. Before removing system 100, any connection maintained with implant 103 is preferably released. In this position, OA guide marking 942 is aligned with guide marking 934 on upper housing 902 and OA tabs 943 are seated within slots 911 in upper housing 902. OA button 941 remains depressible and movement of OA actuator 940 is not prevented in either direction. Needle actuator 960 remains locked in position with respect to OA actuator 940 and moves distally with OA actuator 940. Needle button 961 is not depressible due to the outer ribs 914 and is effectively locked in place within slots 945. Pusher actuator 980 remains locked in the same position as that depicted in **FIG. 42G****,** although tabs 983 are now located distal to slots 968.

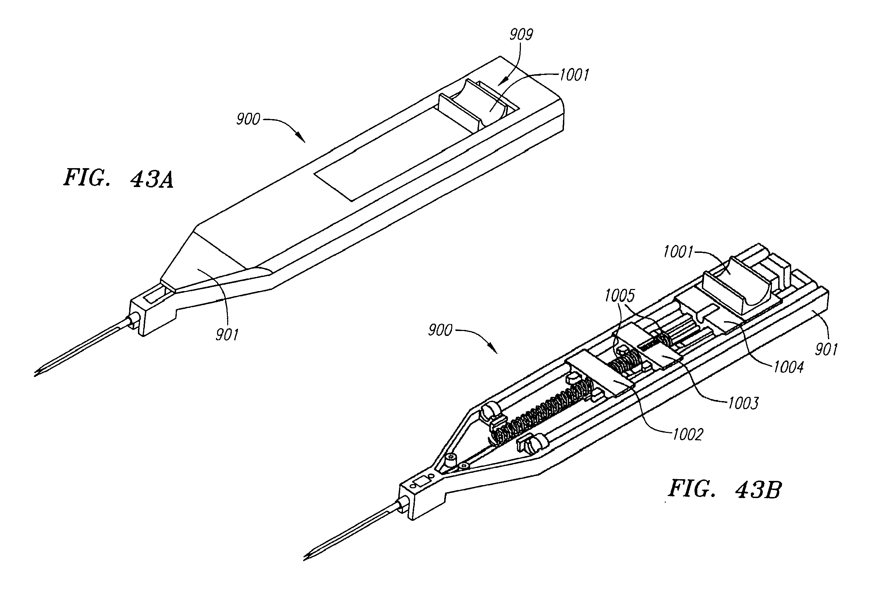

**FIGs. 41A-42I** depict exemplary embodiments of proximal controller 900 using slidable actuators 940, 960 and 980 for the various elements of system 100. It should be noted that other configurations of proximal controller 900 can also be used to control system 100. **FIGs. 43A-B** depict an exemplary embodiment of proximal controller 900 where each of the elements of system 100 are controlled via user interface 909 having one main slidable actuator 1001.

**FIG. 43A** is a perspective view depicting this embodiment fully housed, while **FIG. 43B** is an internal perspective view depicting this embodiment with a portion of the housing omitted. Here, it can be seen that the main slidable actuator 1001 controls sub-actuators 1002-1004, each coupled with one of OA delivery member 401, needle member 405 and pusher member 406. The order in which sub-actuators 1002-1004 are moved is controlled by multiple springs 1005, each having predetermined spring constants chosen to be different so that springs 1005 act together in a cascading manner to effectuate the desired order of movement of sub-actuators 1002-1004.

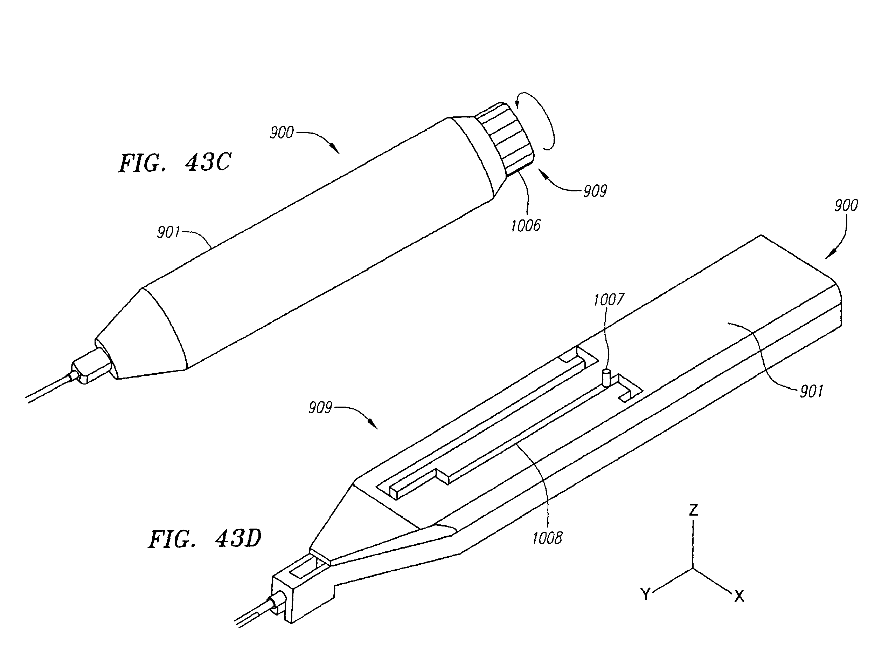

**FIG. 43C** is a perspective view depicting another exemplary embodiment of proximal controller 900 where control of the various elements of system 100 is accomplished via user interface 909 having a rotatable knob 1006 located on controller 900's proximal end. In this embodiment, rotation by a certain amount in a certain direction (clockwise or counterclockwise) can equate to movement of a specific element of system 100, such as OA delivery member 401, needle member 405 and pusher member 406, etc. Rotatable knob 1006 can also be depressible to alternate control between the various elements. For instance, each depression can select a different element, or, depression by variable amounts selects corresponding elements.

**FIG. 43D** is a perspective view depicting yet another exemplary embodiment of proximal controller 900. Here, user interface 909 includes a single lever-like actuator 1007 transitionable through a pathway 1008 to select and move the various elements of system 100. In this embodiment, movement in separate directions equates to different functions of controller 900. For instance, movement of actuator 1007 in the X direction selects a different element of system 100 while movement in the Y direction corresponds to actual movement of the selected element. Preferably, the layout of pathway 1008 is configured to effectuate the proper movement of each element of system 100 in the proper amount at the proper time. Thus, a user can simply continuously advance actuator 1007 through pathway 1008 in a single general direction to achieve proper delivery of implant 103.

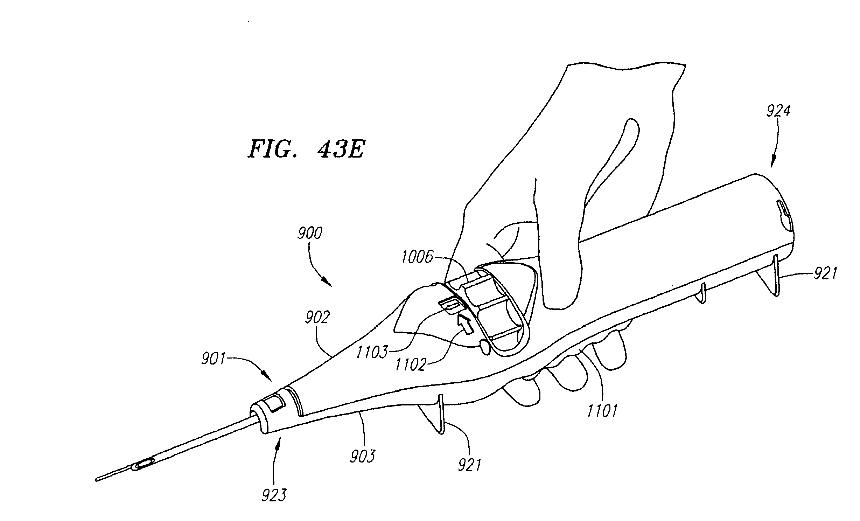

**FIG. 43E** is a perspective view depicting another exemplary embodiment of proximal controller 900 with rotatable knob 1006 during use by a user. Controller 900 has distal end 923 and proximal end 924 and includes housing 901, having upper and lower portions 902 and 903, respectively. Base 921 can be formed in lower housing 903 as shown. Here, knob 1006 is positioned distal to the grips on handle 1101 in a position such that a user can rotate knob 1006 in either direction (i.e., clockwise or counterclockwise) with his or her finger(s) or thumb. Handle 1101 can be grasped by hand and operated or can be rested on another surface (e.g., the user's leg or a table, etc.) and operated from that position. In this embodiment, the user preferably rotates knob 1006 in only the clockwise direction (from the user's perspective), as indicated by arrows 1102 displayed on device 900. Rotation in one direction increases the ease of operation for the user.

Adjacent to knob 1006 is information display 1103, which can be used to provide information to the user regarding any facet of device operation or the procedure. Display 1103 can have any configuration desired, including, but not limited to a mechanical and/or electronic display. In this embodiment, display 1103 is a window or opening in upper housing 902 through which an imprinted guide can be seen by the user, the guide changeable with rotation of knob 1006 and capable of displaying information regarding what step in the closure procedure the user is currently performing. Optionally, the window can be configured as a lens that magnifies the image for the user.

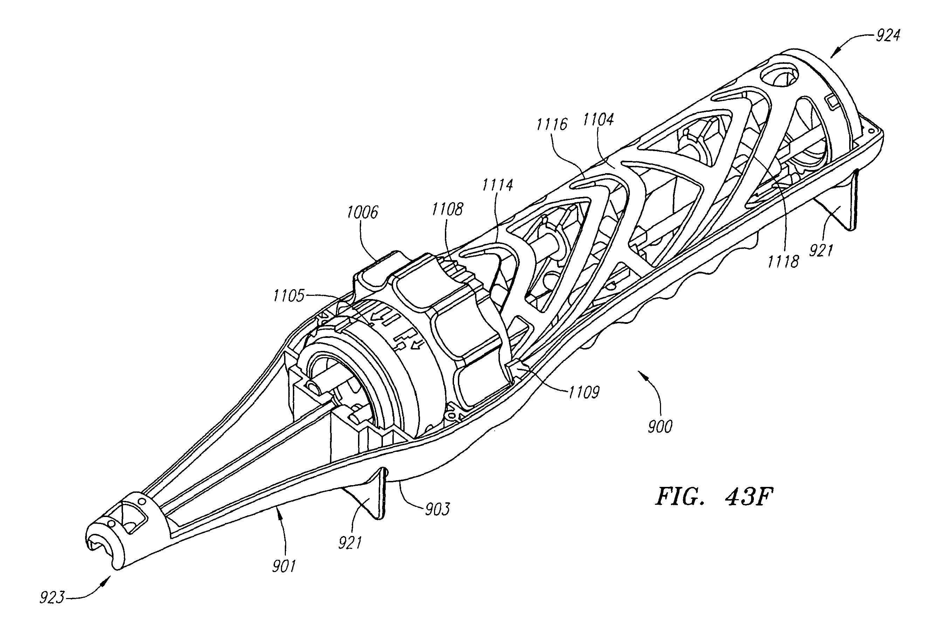

**FIG. 43F** is a perspective view depicting this embodiment of controller 900 with upper housing 902 removed and not shown. Here, a rotatable guide structure, referred to herein as cam 1104, is visible, which is preferably coupled with and moves in conjunction with rotatable knob 1006. Cam 1104 preferably includes three slots 1114, 1116 and 1118, the function of which will be described below. Also visible is a guide marking surface 1105, which includes the guides visible on display 1103 (shown in **FIG. 43E****).** Rotatable knob 1006 includes a plurality of ratchets 1108 configured to interface with deflectable abutment 1109.

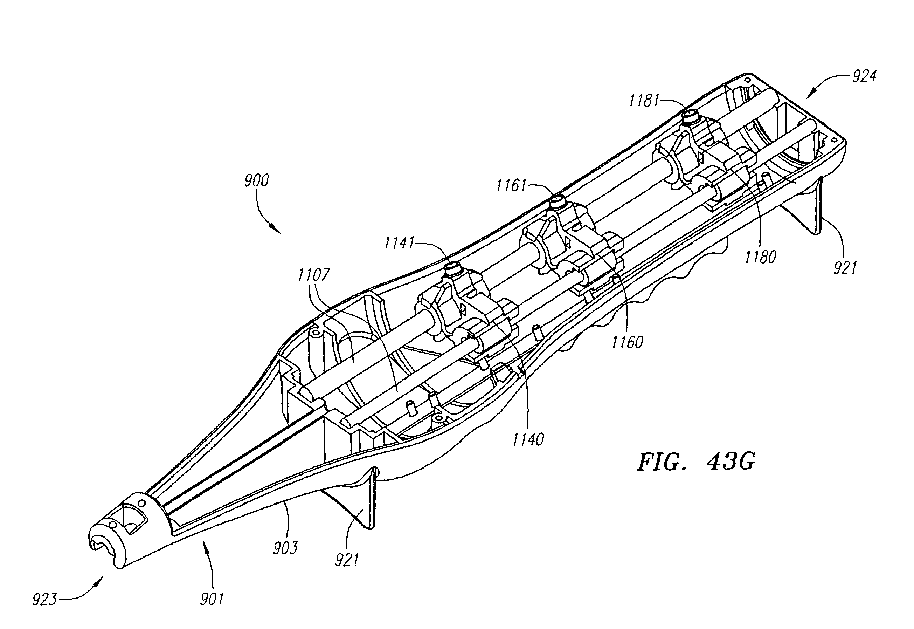

**FIG. 43G** is a perspective view depicting this embodiment with knob 1006 and rotatable cam 1104 removed from housing 901 and not shown. Here, an OA delivery member actuator 1140, a needle member actuator 1160, a pusher member actuator 1180 and guide rails 1107 can be seen. OA delivery member actuator 1140, needle member actuator 1160, and pusher member actuator 1180 are coupled with OA delivery member 401, needle 405 and pusher member 406, respectively (not shown), and configured to actuate longitudinal movement of members 401, 405 and 406 based on rotation of knob 1006.

Each actuator 1140, 1160 and 1180 can include an interface 1141, 1161 and 1181, respectively, that interfaces with one of the respective slots 1114, 1116 and 1118 (shown in **FIG. 43F****).** In this embodiment, interfaces 1141, 1161 and 1181 are rotatable wheels configured to ride along the surface of slots 1114, 1116 and 1118, respectively, causing each actuator 1140, 1160 and 1180 to slide proximally or distally over guide rails 1107. One of skill in the art will readily recognize that any low friction interface, such as rotatable wheels, ball bearings and the like, can be used to slide or otherwise move within slots 1114-1118. Rotatable cam 1104 can also include one or more reinforcing bridge member (not shown) coupled with cam 1104 at multiple positions along its length to prevent the rotational torque from causing the width of slots 1114, 1116 and 1118 to vary and increase friction on interfaces 1141, 1161 and/or 1181.

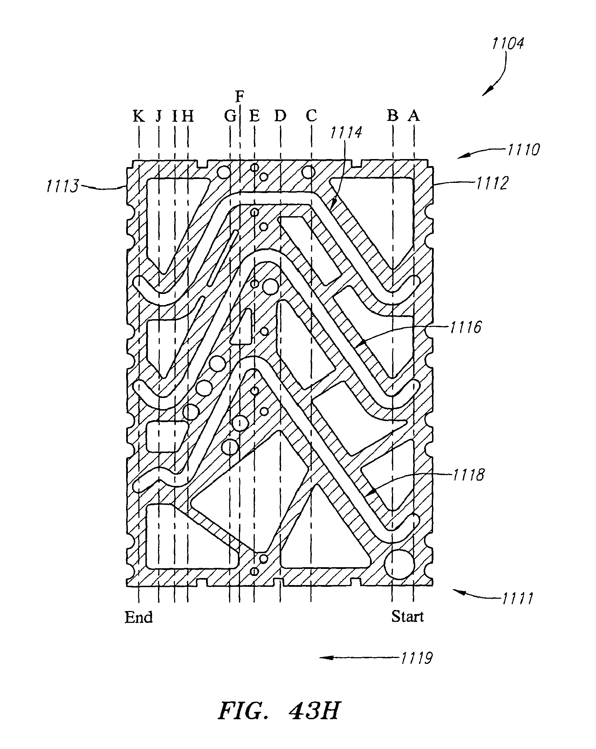

**FIG. 43H** is a schematic view of rotatable cam 1104, shown in a flat, unrolled perspective to more clearly illustrate the configuration of slots 1114, 1116 and 1118 and their relation to movement of actuators 1140, 1160 and 1180. As depicted here, cam 1104 has a distal end 1110, a proximal end 1111 and opposite sides 1112 and 1113, which are adjacent when cam 1104 is in a cylindrical configuration. As cam 1104 is rotated in a clockwise direction, interface wheels 1141, 1161 and 1181 travel in slots 1114, 1116 and 1118, respectively, in direction 1119.

Reference lines A-K extend longitudinally along cam 1104 and will be used to describe the position of actuators 1140, 1160 and 1180 with respect to the corresponding step in an exemplary embodiment of the closure procedure, making reference to portions of system 100 and the patient's anatomy that are not shown.

At the outset of the closure procedure, interface wheels 1141, 1161 and 1181 are all preferably located in their respective slots 1114-1118 at reference line A. These positions correspond to a low profile arrangement of members 401, 405 and 406 suitable to be maintained during advancement of body member 101 through the vasculature and into proximity with septal wall 207, preferably within right atrium 205. Once in proximity with septal wall 207, knob 1006 can be rotated to bring wheels 1141, 1161 and 1181 to a position along reference line B in the respective slots 1114-1118. These B positions are all proximal to the respective A positions. OA actuator 1140 has moved proximally and actuated the raising and proximal movement of OA delivery member 401 to raise arm member 409 and place it in position to engage limbus 211, similar to the orientation depicted in **FIG. 14D** (e.g., a secundum capture position).

Needle actuator 1160 and pusher actuator 1180 have moved proximally as well, such that all three members 401, 405 and 406 remain in the same positions with respect to each other. It should be noted that the use of actuators 1140, 1160 and 1180 interfacing with predefined slots 1114-1118 in the manner described here eliminates the need to lock each member 401, 405 or 406 with respect to another member, since the relative position of each member 401, 405 and 406 is controlled by the radial position of knob 1006 (and cam 1104).

After body member 101 has been advanced distally such that arm member 409 abuts limbus 211, knob 1006 is preferably rotated to the position of reference line C. This rotation transitions OA actuator 1140 distally causing OA member 401 to enter an off-axis delivery orientation, similar to the orientation depicted in **FIG. 14F****.** Based on the length and shape of arm member 409 and the thickness of limbus 211, it is possible for grasping device 404 to clamp down and capture limbus 211 at a position after position B but prior to position C. In such a case, continued rotation to position C does not cause additional downward movement of arm member 409, but does cause OA member 401 to continue into the off axis delivery orientation. Again, needle actuator 1160 and pusher actuator 1180 have moved distally with OA member 401, but by a slightly greater amount such that members 405 and 406 remain in the same positions with respect to each other but both have advanced within OA member 401, preferably to a point where needle 405 is just inside OA member 401's distal end 410.

One of skill in the art will readily recognize that the slope of slots 1114-1118 can determine the distal/proximal (i.e., longitudinal) rate of movement at which the respective member 401, 405 and 406 will move in relation to the rate of rotation of knob 1006. A relatively more vertical slope corresponds to a relatively greater distance while a relatively more horizontal slope corresponds to a relatively shorter distance. The rate at which members 401, 405 and 406 are transitioned can be dependent upon the individual application.

Rotation of knob 1006 to reference line D causes needle actuator 1160 to transition distally to advance needle member 405 out of OA delivery member 401 and through septal wall 207, preferably through both septum secundum 210 and septum primum 214. As in other embodiments described herein, it should be noted that proximal controller 900 can also be configured to automatically advance needle member 405 by the desired amount. For instance, needle member 405 can be spring loaded such that movement of needle actuator 1160 to a certain position releases the spring, which provides force sufficient to advance needle member 405 through septal wall 207. Of course, one of skill in the art will readily recognize that other techniques for automatically advancing needle member 405 can be implemented and, accordingly, the systems and methods described herein are not limited to spring-based techniques.

At position D, pusher actuator 1180 has been transitioned with needle actuator 1160 to a position distal that of the previous position, such that the positions of needle 405 and pusher 406 with respect to each other are the same as in position C, although both have been transitioned distally together while OA member 401 has not moved. As can be seen in **FIG. 43H****,** this is because needle slot 1116 and pusher slot 1118 are sloped in a distal direction from position C to position D, while OA member slot 1114 remains horizontal. In this embodiment, rotation of knob 1006 to position D engages a ratchet 1108 on abutment 1109 (see **FIG. 43F****)** such that knob 1006 can no longer be rotated in the opposite direction as a safeguard measure. Preferably, ratchets 1108 are located, at least, in positions corresponding to positions D-J to provide additional safeguards throughout the procedure.

Rotation of knob 1006 to reference line E causes pusher actuator 1180 to transition distally causing pusher member 406 to advance LA portion 302 of implant 103 out of needle member 405, which, depending on the specific embodiment of implant 103, allows LA portion 302 to expand within left atrium 212. OA actuator 1140 remain in the same position as position D, while needle actuator 1160 is transitioned proximally by a relatively small amount to facilitate deployment of LA portion 302.

Rotation of knob 1006 to reference line F, first causes needle actuator 1160 to retract proximally while pusher actuator 1180 remains stationary, then causes pusher actuator 1180 to retract proximally as well. This sequential motion can first further deploy LA portion 302 and center portion 303, and then retracts implant 103 to cause LA portion 302 to contact septum primum 214. OA actuator 1140 remains stationary between positions E and F.

Rotation of knob 1006 from position F to position G causes needle actuator 1160 and pusher actuator 1180 to proximally retract, at least partially, into OA member 401. OA actuator 1140 is proximally retracted by a relatively smaller amount than actuators 1160 and 1180. In this embodiment, implant 103 is preferably coupled with pusher member 406 to prevent complete deployment until desired.

Rotation of knob 1006 from position G to position H and then on to position I causes OA actuator 1140, needle actuator 1160 and pusher actuator 1180 to proximally retract to transition OA delivery member proximally from the OA delivery orientation. Here, pusher 406 is retracted proximally by the greatest amount, while needle 405 is retracted proximally by a slightly less amount and OA member 401 is retracted proximally by a slightly less amount than needle 405. Needle 405 is preferably again fully housed within OA member 401. In this embodiment, central portion 303 of implant 103 is preferably flexible and allows implant 103 to bend prior to being released from pusher 406.

Rotation of knob 1006 from position I to position J causes pusher actuator 1180 to advance distally while OA actuator 1140 and needle actuator 1160 are retracted proximally and then held in a constant position. This can expose the distal end of pusher 406 and allow RA portion 301 of implant 103 to be released, thereby fully deploying implant 103 (with the exception of any safety devices, such as a tether, that still connect implant 103 to delivery device 104).

Rotation of knob 1006 from position J to position K distally advances OA actuator 1140 and needle actuator 1160 to positions similar to the start position A, placing OA member 401 in the low profile position suitable for withdrawal through the anatomy of the subject with needle 405 located within OA member 401. Pusher actuator 1180 has been proximally retracted to cause pusher 406 to retract into OA member 401 for withdrawal from the subject.

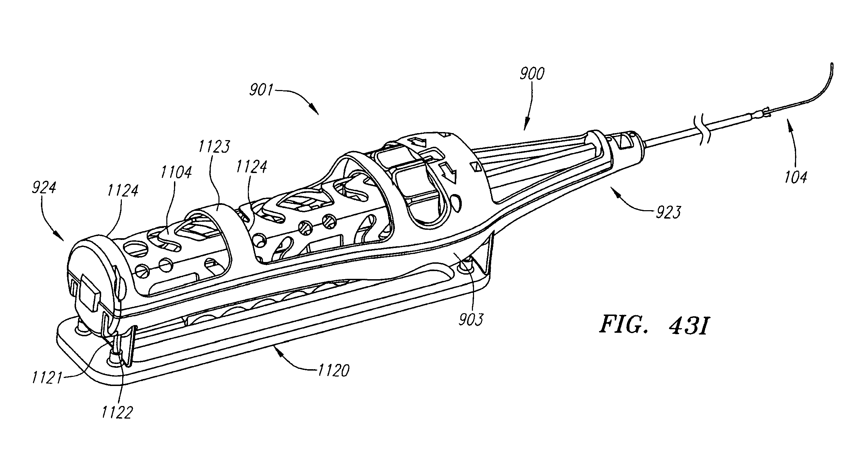

**FIG. 43I** is a perspective view depicting another exemplary embodiment of proximal controller 900 resting on a loading platform 1120 for use in loading implant 103 (not shown) prior to final assembly. Here, upper housing 902 has been replaced with a loading upper housing 1123 having open section 1124 to allow access to cam 1104. Loading platform 1120 is preferably used for loading implant 103 into delivery device 104 and engaging each actuator 1140, 1160 and 1180 with cam 1104. Loading platform 1120 can include one or more pegs 1121 configured to slide within corresponding apertures 1122 in lower housing 903 of controller 900. Pegs 1121 are preferably configured to contact and lift cam 1104 to disengage actuators 1140, 1160, and 1180. Once disengaged, actuators 1140, 1160 and 1180 can be freely moved within cam 1104 and delivery device 104 can be loaded with implant 103.

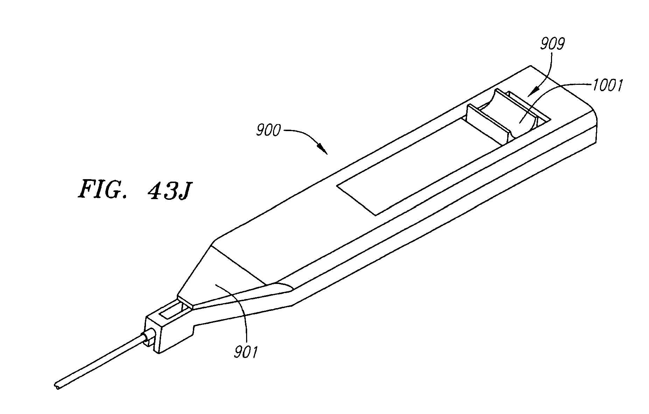

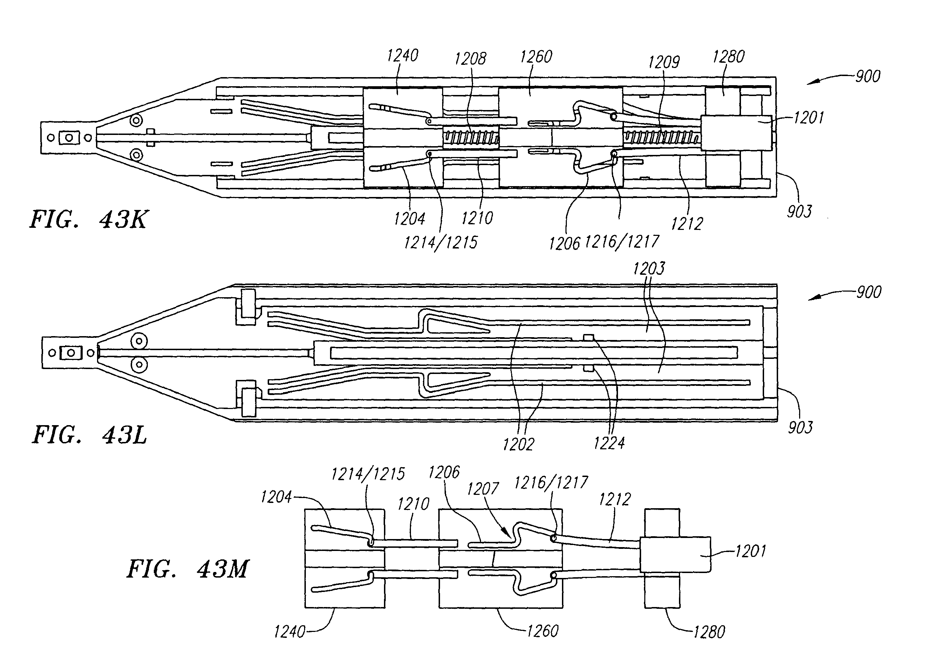

**FIG. 43J** is a top down view of another exemplary embodiment of proximal controller 900, similar to that described with reference to **FIGs. 43A-B.** In this embodiment, members 401, 405 and 406 (not shown) are controllable by way of a series of actuators that are translatable distally and proximally by distal and/or proximal movement of a single user interface 1201. **FIG. 43K** is a top down view of lower housing 903 with actuators 1240, 1260 and 1280 shown therein. Actuators 1240, 1260 and 1280 are coupled with OA member 401, needle member 405 and pusher member 406, respectively. User interface 1201 is coupled with pusher actuator 1280 which in turn is coupled with needle actuator 1260, which is in turn coupled with OA actuator 1240. Two bias members 1208 and 1209 are also shown. Bias member 1208, in this embodiment, is a spring-like member and is coupled between OA actuator 1240 and needle actuator 1260. Bias member 1209 is also a spring-like member and is coupled between needle actuator 1260 and pusher actuator 1280. It should be noted that any member configured to apply a bias can be used for bias members 1208 and 1209, not limited solely to spring-like members.

**FIG. 43L** is a top down view of lower housing 903 with actuators 1240-1280 removed and **FIG. 43M** is top down view of actuators 1240-1280. Preferably, actuators 1240 and 1260 each include slots 1204 and 1206, respectively. Pusher actuator 1280 preferably includes a deflectable strut 1212 configured to interface with slot 1206. The distal end of strut 1212 preferably includes an upward-facing abutment 1216 and a downward-facing abutment 1217 located opposite to abutment 1216 (here, abutment 1217 is obscured by strut 1212). Abutment 1216 is preferably configured to interface with slot 1206 of needle actuator 1260, while abutment 1217 is preferably configured to interface with track 1203 in lower housing 903. Likewise, needle actuator 1260 preferably includes a deflectable strut 1210 also having an upward-facing abutment 1214 and a downward-facing abutment 1215 (obscured). Upward-facing abutment 1214 is preferably configured to interface with slot 1204 in actuator 1240, while downward-facing abutment 1215 is preferably configured to interface with track 1203 in lower housing 903. In this embodiment, there are two of each of struts 1210-1212, slots 1204-1206, abutments 1214-1217 and tracks 1203, but it should be noted that more or less of said items can be used depending on the needs of the application.

In this configuration, movement of actuators 1240-1280 is dependent, in part, on the positions of abutments 1214 and 1216 within slots 1204 and 1206 respectively, as well as the position of abutments 1215 and 1217 within track 1203. In addition, bias members 1208 and 1209, depending on the relative bias strengths thereof, will also influence the order of movement of actuators 1240 and 1260, respectively.

Track 1203 and slots 1204 and 1206 are preferably laid out to provide an desired order of movement to each of actuators 1240-1280, either in unison or in relative motion with each other. To operate, a user preferably depresses interface button 1201 and advances user interface 1201, as well as pusher actuator 1280 which is coupled with interface 1201, in a distal direction. As with the other embodiments of controller 900 described herein, the movement of the actuators is dependent on the order of steps in the desired treatment or closure procedure.

In **FIG. 43K****,** actuators 1240-1280 are in positions suitable to place members 401, 405 and 406 in a low profile configuration suitable for advancement within the vasculature. Once in position within the heart, the user can commence the procedure by depressing interface 1201 and sliding it distally. It should be noted that guide markings can be placed on upper hosing 902 to guide the user in how far to advance interface 1201. Distal movement of interface 1201 causes pusher actuator 1280 to move distally, which also forces needle actuator 1260 to advance distally in lockstep fashion, since struts 1212 are prevented from deflecting outward and advancing in slots 1206 by the presence of rail 1202, which abuts downward-facing abutment 1217. Thus, struts 1212 do not move with respect to needle actuator 1260 and downward-facing abutment 1217 slides within track 1203. Conversely, OA actuator 1240 remains stationary because each track 1203 is coincidental with slot 1204 at this position, allowing struts 1210 to deflect and upward-facing abutment to slide forward within slot 1204.

The rate at which each actuator 1240-80 moves can be varied according to the slope of the respective slots and track. Additional abutments, such as abutments 1224 in lower housing 903 shown in **FIG. 43L****,** can be incorporated to prevent further distal motion of the actuators. As mentioned above, bias members 1208 and 1209 can be configured with different relative strengths, for instance, to allow actuators 1240 and 1260 to move in a desired sequence. Furthermore, bias members 1208 and/or 1209 can be configured to cause a particular actuator to move in a direction opposite that in which interface 1201 is being moved. For instance, slot 1206 has a middle section 1207 with a reversed slope that allows needle actuator 1260 to move proximally when the appropriate forces are applied by bias members 1208 and 1209.

Thus, as will be readily apparent to one of skill in the art based on the description herein, the layout of slots 1204-1206, track 1203 and the configuration of bias members 1208-1209 can allow numerous desired combinations of movement of actuators 1240-80 to be achieved. A wide variety of different procedures can be performed with the embodiments of proximal controller described herein, including, but not limited to those in the heart.

It should be noted that proximal controller 900 is not limited to the exemplary embodiments described with respect to **FIGs. 41A-43M****.** Each of these embodiments can be likewise implemented using automated electronic techniques, for instance, such as a rotatable cam controlled by one or more electronic push buttons. These and other techniques that can be used include, but are not limited to, automatic actuation, electronic actuation, robotic actuation, infrared sensor actuation, and other types of manual actuation using levers, depressible buttons, rotatable knobs and dials, switches and the like.

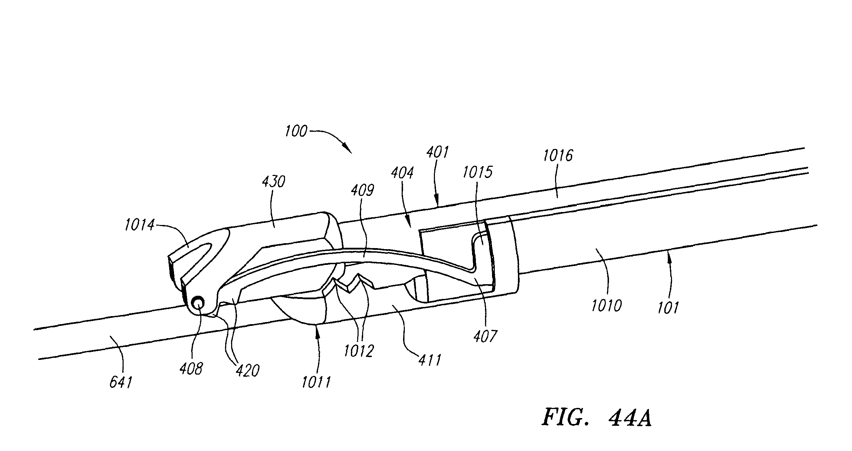

Referring back to configuration of the distal portion of system 100, **FIG. 44A** is a perspective view depicting another exemplary embodiment of system 100 without inclusion of stabilization device 105 and centering device 106. Here, body member 101 includes tubular body 1010 coupled with distal end tip 1011, which includes elongate support section 411. Guidewire 641 is shown routed through distal end tip 1011. OA delivery member includes distal cap 430 coupled with tubular body 1016.

Any portion of system 100 can be configured to increase the surface friction with septal wall 207. Here, elongate support section 411 of body member 101 includes multiple abutments, or teeth 1012 to aid in engaging the inner wall of tunnel 215, such as the wall of secundum 210. In this embodiment, teeth 1012 are triangularly configured although one of skill in the art will readily recognize that any configuration of teeth 1012 can be used. Also, any surface of system 100 can be configured to increase the surface friction with septal wall 207, such as by the use of abrasive coatings or textures formed without coatings. For instance, a polymeric sheet can be coupled between arm members 409 such that it extends across the gap between arm members 409 and thereby increases the surface friction with septal wall 207 as well as stabilizes the position of each arm member 409 with respect to the other. Any polymeric sheet or strands of polymeric material can be used including (but not limited) to polyester fabrics and the like.

Also in this embodiment, distal cap 430 of OA delivery member 401 is configured to be atraumatic. This reduces the risk of damaging bodily tissue during the implantation procedure or while routing OA delivery member 401 within the subject's vasculature. Here, the portion of distal cap opposite elongate support section 411 has an atraumatic beveled distal surface 1014.

In this embodiment, grasping device 404 includes two arm members 409 having a generally curved shape to accommodate limbus 211. The underside of each arm member 409 includes abutments 420 configured as teeth to aid in engaging septal wall 207. Here, hinge 408 is a swivel-type hinge that allows distal cap 430 of OA delivery member 401 to swivel, or rotate, about arm member 409. Hinge 407 is formed by the intersection of arm member 409 with a base portion 1015. Arm member 409 is configured to flex at this intersection from the at-rest state depicted here. This allows OA delivery member 401 to be raised up and away from body member 101 when proximal force is applied, but also biases OA delivery member 401 to return to the at-rest state, both facilitating engagement with limbus 211 and return of OA delivery member 401 to this low-profile configuration prior to withdrawal from the subject.

If desired, the angle at which OA delivery member 401 is oriented with respect to body member 101 after advancement of OA delivery member 401 into the off-axis position, can be adjusted by varying the lengths of each arm member 409. For instance, if an arm member 409 on the left side were relatively longer than arm member 409 on the right side, when deployed into the off axis configuration OA delivery member 401 would tilt to the left. One of skill in the art will readily recognize that by varying the degree to which the arm members 409 differ in length, one can vary the amount of tilt introduced into OA delivery member 401. This tilt can be used to cause needle 405 to penetrate septal wall 207 at any angle desired or needed for the particular application.

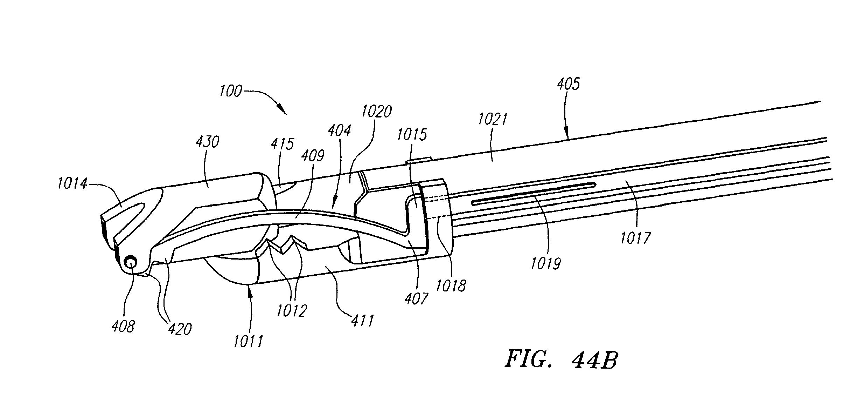

**FIG. 44B** is a perspective view depicting this exemplary embodiment of system 100 without guidewire 641, tubular body 1010 of body member 101, and tubular body 1016 of OA delivery member 401 in order to facilitate description of system 100. Visible within OA delivery member 401 is needle member 405 having a rigid distal end portion 1020 and a tubular body 1021. Rigid distal end portion 1020 includes sharp distal tip 415 and is preferably composed of a rigid material such as stainless steel, NITINOL and the like.

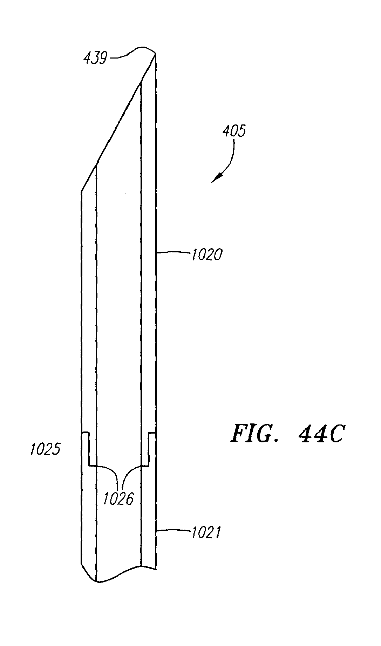

**FIG. 44C** is a cross-sectional view depicting an exemplary embodiment of needle member 405 with rigid distal end portion 1020 and tubular body 1021. Here, the interface region 1025 between portion 1020 and tubular body 1021 is configured to be overlapping. This can increase the strength of the coupling between each portion of needle member 405. In this embodiment, the thickness of the part of portion 1020 and tubular body 1021 in interface region is tapered, in this case in a stepped fashion, such that each portion is complementary to the other. As one of ordinary skill in the art will readily recognize, the stepped interface region 1025 can be reversed such that the most proximal part of portion 1020 is located on the outside of the most distal part of tubular body 1021.

Although not shown, interface 1025 can be further strengthened with the use of a tubular support member surrounding interface 1025. For instance, in one exemplary embodiment, a polymeric tube (e.g., polyester, polyethylene and the like) can be heat shrunk or bonded around the relatively rigid interface 1025 to provide strain relief.

It should be noted that the location of interface region 1025 along the longitudinal axis of needle member 405 can be chosen as desired. In one embodiment, the location of interface region 1025 is close enough to distal tip 415 to have a minimal effect on the flexibility of needle member 405, while at the same time being far enough from distal tip 439 to minimize the risk of any portion of implant 103 or pusher member 406 catching on surface junction 1026 during delivery. The actual location of interface region 1025 is dependent on the size of implant 103, the length of needle member 405 that enters a curved state during delivery, the angle of the sharp beveled surface of needle member 405, as well as other factors.

Referring back to **FIG. 44B****,** also visible is an elongate support portion 1017 and base portion 1015 of grasping device 404. Elongate support portion 1017 is configured to fit within a lumen of body member 101, preferably within tubular body 1010 (not shown). Elongate support portion 1017 provides support and leverage to arm members 409 during use. Elongate support portion 1017 is preferably coupled with tubular body 1010. In this embodiment, elongate support portion 1017 can be adhesively coupled with tubular body 1010 and can include one or more apertures 1019 configured to improve the strength of the adhesive bond and to facilitate the manufacturing process. Preferably, apertures 1019 are configured such that the adhesive, which can be introduced through one or more side ports or slits in tubular body 1010, can distribute within each aperture 1019 during the bonding process. This allows for a stronger bond between section 1017 and tubular body 1010 and also allows for an outlet for any excess adhesive applied during the manufacturing process.

Elongate support section 1017 can routed through a lumen 1018 (shown to be obscured with dashed lines) in distal end tip 1011. This allows the coupling of elongate support section 1017 with body member 101 to further strengthen the coupling of distal end tip 1011 with the remainder of body member 101. It should be noted that any technique, other than ones using adhesives, can be utilized to couple arm members 409 with body member 101.

The various tubular bodies used in system 100, such as tubular body 1010, 1016, and 1021, are preferably composed of flexible, durable, bio-compatible materials including, but not limited to, NITINOL, stainless steel, and polymers such as PEBAX, polyester, polyvinylchloride (PVC), polyethylene, polyetheretherketone (PEEK), polyimide (PI), nylon (with or without reinforcing materials such as braided or coiled stainless steel, kevlar, carbon fiber and the like). Some materials, such as PEEK, can be manufactured with a curve in a desired direction. Preferably, system 100 is manufactured so that the curve of the outer sheath is aligned in a predetermined manner to be consistent with any curved path the respective outer sheath is designed to follow. For instance, needle tubular body 1020, if manufactured from a material displaying a curve, it is preferably aligned such that the curve is oriented similarly to the curved path needle member 405 follows in the exemplary embodiment described with respect to **FIG. 18B****.** Also, needle distal end portion 1020 is preferably coupled with tubular body 1021 such that needle distal tip 439 (not shown in **FIG. 44B****)** is oriented as desired (e.g., on the inside of the curved portion of needle member 405).

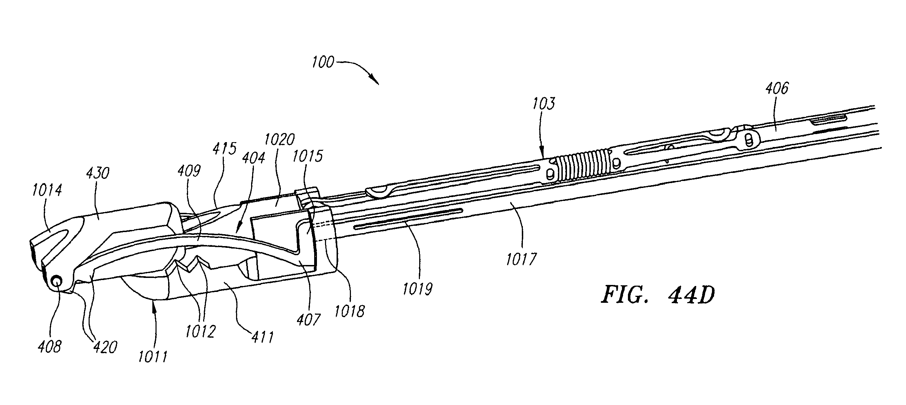

**FIG. 44D** is a perspective view of the exemplary embodiment of **FIG. 44B** but without tubular body 1020 of needle member 405. Here, implant 103 and pusher member 406 are both visible. Implant 103 is configured as a clip, similar to the embodiments described in the incorporated application "Clip-based Systems and Methods for Treating Septal Defects," which is referenced above, and also similar to the embodiments described in U.S. Patent Application Serial No. _(attorney docket 15997.4018) entitled "Systems and Methods for Accommodating Anatomical Characteristics in the Treatment of Septal Defects" filed May 5, 2007, which is fully incorporated by reference herein.

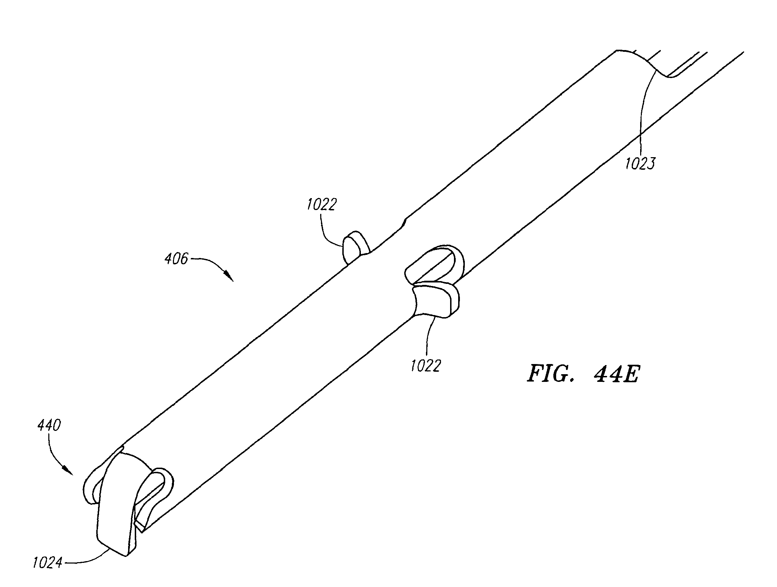

**FIG. 44E** is a perspective view depicting the distal portion of pusher member 406 in greater detail. Here, pusher member 406 includes tabs 1022 for engaging with apertures on clip 103 and one or more apertures 1023 which increase the flexibility of pusher member 406. The location of apertures 1023 also controls the direction in which pusher member 406 is relatively more flexible. Pusher member 406 also includes a closed distal end 440, which is closed by way of a deflected tab 1024, which also extends past the end of pusher member 406. This allows pusher member 406 to remain configured in a generally tubular manner, but reduces the risk of an open distal end 440 sliding over a portion of implant 103 or of distal end 440 sliding into an open central portion 303 of implant 103, whether configured as a coil, clip or otherwise. Deflected tab 1024 can be used as an alternative to, or in addition to, a blocking member included within central portion 303 of implant 103. A blocking member within implant 103, or at distal end 440 of pusher member 406, can also be a deflected tab, a radiopaque rod, and the like.

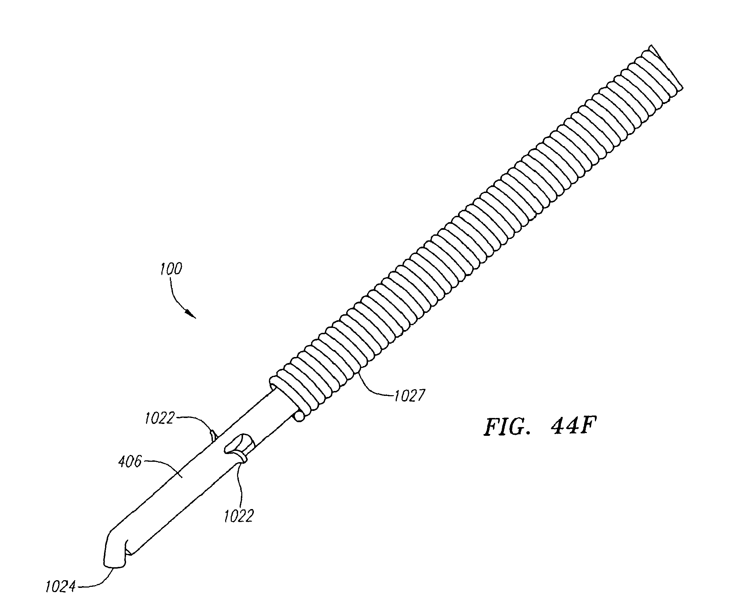

**FIG. 44F** is a perspective view depicting another exemplary embodiment of system 100 where pusher member 406 is located within an intermediate sheath 1027. Here, intermediate sheath 1027 is configured to reduce the risk of buckling or kinking, by occupying the space between the outer diameter of pusher member 406 and the inner diameter of needle member 405. Intermediate sheath 1027 is preferably flexible and, as depicted here, can be configured in a coil-like manner.

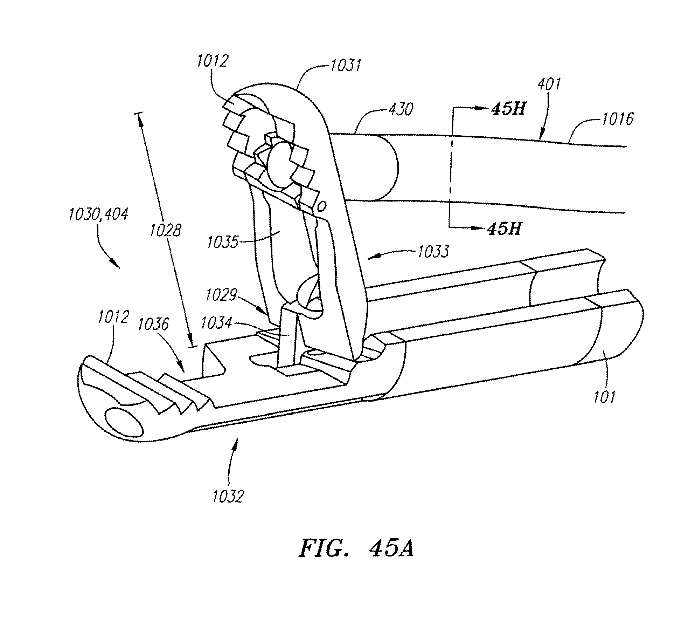

**FIG. 45A** is a perspective view depicting another exemplary embodiment of system 100. As with all other embodiments described herein, it should be noted that the elements, features and characteristics of this embodiment can be used with any other embodiments described herein. Shown here is OA delivery member 401 having outer sheath 1016. OA delivery member 401 is coupled with distal end tip 430 which in turn is pivotably coupled with distal end section 1030 of body member 101. Here, distal end section 1030 functions as tissue engagement device 404. Distal tips 1031 of distal end section 1030 have a rounded, preferably spherical radius, to maximize the atraumatic characteristics of the device.

Distal end section 1030 includes a lower portion 1032 pivotably coupled with an upper portion 1033. Both portions 1032 and 1033 can include one or more teeth 1012. In the instance where a plurality of teeth 1012 are present, as shown here, teeth 1012 on upper portion 1033 are preferably located in positions complimentary to teeth 1012 located on lower portion 1032 to allow for a greater interface between the two portions 1032-33 and a smaller overall profile. Portions 1032 and 1033 can be constructed from any desired material, including but not limited to NITINOL, stainless steel, polymeric materials or combinations thereof. For instance, in one exemplary embodiment, portions 1032 and 1033 are each constructed from a rigid polymeric material while teeth 1012 are constructed from stainless steel.

Lower portion 1032 and upper potion 1033 can be pivotably coupled together in any manner desired, including use of a living hinge or a hole and rod/strut mechanism (as shown here). Here, the hinge is formed through a single strut 1034 on upper portion 1033, although any number of struts 1034 can be used, as one of skill in the art will recognize the number and placement of struts 1034 can result in increased stability.

In this embodiment, distal tip 430 is also pivotably coupled with upper portion 1033 by way of a hinge (although, again, one of skill in the art will readily recognize the multiple manners in which distal tip 430 can be pivotably coupled with upper portion 1033). Here, distal tip 430 also includes teeth 1012 to provide increased friction with body tissue. Upper portion 1033 includes an open region 1035 in which distal tip 430 preferably partially resides. This allows distal tip 430 to be disposed proximal to distal tip 1031 thereby allowing a greater surface of body tissue to be engaged by distal end section 1030. Also of note is that lower portion 1032 is configured to provide an open region 1036. Open region 1036 is positioned adjacent distal tip 430 and allows needle member 405 (not shown) to pass distal end section 1030. **FIG. 45A** depicts system 100 with distal end section 1030 in an open position ready to engage body tissue, preferably septum secundum 210 (not shown).

The placement of distal tip 430 in a position proximal to distal end 1031 allows the height of upper portion 1033 in the capture position to be increased, making it more difficult for distal end section 1030 to inadvertently pass into the PFO tunnel. For instance, the distance from base 1029 of upper portion 1033 to the furthest point on the opposite end of upper portion 1033 that engages tissue can be referred to as the clamp distance 1028 of the device. If clamp distance 1028 is too short, distal end section 1030 may not be able to properly engage secundum 210. For instance, the limbus may be too thick to allow any grasping to occur or, alternatively, distal end section 1030 may be able to grasp the limbus, but not with enough force and surface friction to maintain an effective and reliable "lock" on the septum secundum during the course of the procedure. An adequate clamp distance 1028 preferably allows the user to maintain an effective lock on the secundum 210 to prevent non-negligible slippage during the procedure. This is also dependent on the configuration of the surfaces of upper portion 1033 and lower portion 1032, i.e., whether teeth 1012 or some other friction increasing structure, coating or texture is present, and the degree to which surface friction is thereby increased by said friction increasing means.

Preferably, device 404 is configured to achieve a puncture distance, i.e., the distance from the edge of the limbus to the point on the outer surface ot the secundum where the needle penetrates, of at least 3 millimeters (mm) in instances where the limbus is relatively thin. Clamp distance 1028 is preferably greater than the puncture distance to allow for adequate secundum tissue to be engaged. In one exemplary embodiment, device 404 is configured to achieve a puncture distance is in the range of 3-7 mm and preferably 3-5 mm. In another exemplary embodiment, device 404 is configured to achieve a puncture distance of approximately 4 mm. Clamp distance 1028 is preferably less than 15 mm. It should be noted that these distances are merely exemplary embodiments, and, in instances where no length is recited in the claims, in no way should the embodiments described herein be construed as limited to any particular length.

Also, upper portion 1033 can be made to extend relatively further distally than lower portion 1032 such that distal tip 430 is located distal to the distal tip 1031 of lower portion 1032. This can facilitate the motion of needle member 405 past lower portion 1032 and allow easier penetration and left atrial access.

It should be noted that upper portion 1033 and lower portion 1032 can be pivoted with respect to each other, or opened, by any amount in accordance with the needs of the application including amounts greater than or equal to 90 degrees. A mechanical stop is preferably included to prevent travel of the upper portion 1033 past the desired position. A stop is also preferably included between distal tip 430 and upper portion 1033 that prevents rotation of distal tip 430 too far forward in a distal direction and thereby maintains the desired orientation with the body tissue.

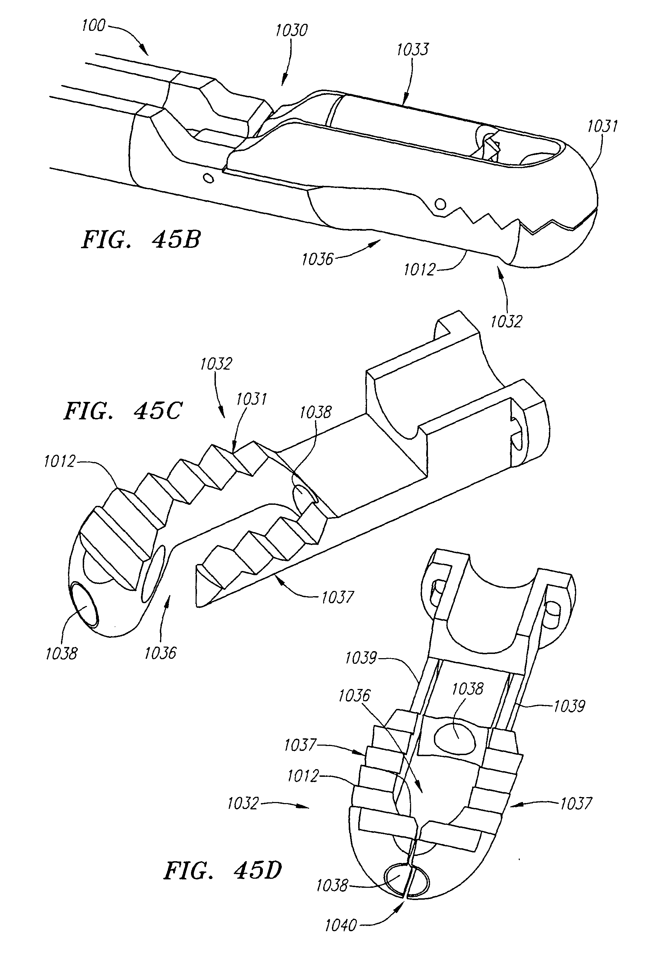

**FIG. 45B** is another perspective view depicting system 100, this time with distal end section 1030 in a closed configuration, such as that which would be used while advancing the device through the body vasculature (body member 101, distal end tip 430 and OA delivery member 401 are not shown for clarity).

**FIG. 45C** is a perspective view depicting another exemplary embodiment of lower portion 1032. In this embodiment, open region 1036 has a bent L shape and teeth 1012 are present on each of two side sections 1037 of lower portion 1032. Open region 1036 allows the passage of needle 405 and the escape of closure device 103 (not shown) after deployment.

**FIG. 45D** is a perspective view depicting another exemplary embodiment of lower portion 1032. Here, open region 1036 is almost entirely encompassed by side sections 1037 except for a distal escape slit 1040. Side sections 1037 are configured to deflect outwards away from each other thereby opening escape slit 1040 and providing a path through which closure device 103 can pass. Side sections 1037 are made deflectable, in this embodiment, by living hinges 1039.

In both **FIGs. 45C** and **45D****,** apertures 1038 are visible. Apertures 1038 can be used for passage of other devices, not limited to a guidewire and the like. Preferably, a guidewire is present in the PFO tunnel before attempting to engage the limbus. Aperture 1038 can be offset from center to allow needle 405 to pass by any guidewire that may be present. Although not shown in **FIG. 45A-D,** distal end section 1030 also preferably includes a bias member 413 that applies a closure bias between lower portion 1032 and upper portion 1033. This bias member 413 can be any member configured to apply pressure between portions 1032 and 1033 such as a spring, a bent nitinol wire, and the like. In one exemplary embodiment, the rod used as part of the hinge between upper portion 1033 and lower portion 1032 can be configured to allow pivoting motion while at the same time entering a torsioned state upon flexation thereby acting as both a hinge and a bias member 412.

Preferably, lower portion 1032 is configured to minimize surface friction to tissue as lower portion 1032 is advanced into PFO tunnel 215. For instance, one or more of teeth 1012 are preferably angled to have a relatively higher degree of surface friction against tissue when teeth 1012 are translated proximally than when translated distally. This allows lower portion 1032 to be easily advanced into PFO tunnel 215 while at the same time adequately engaging secundum 210 once properly positioned within tunnel 215.

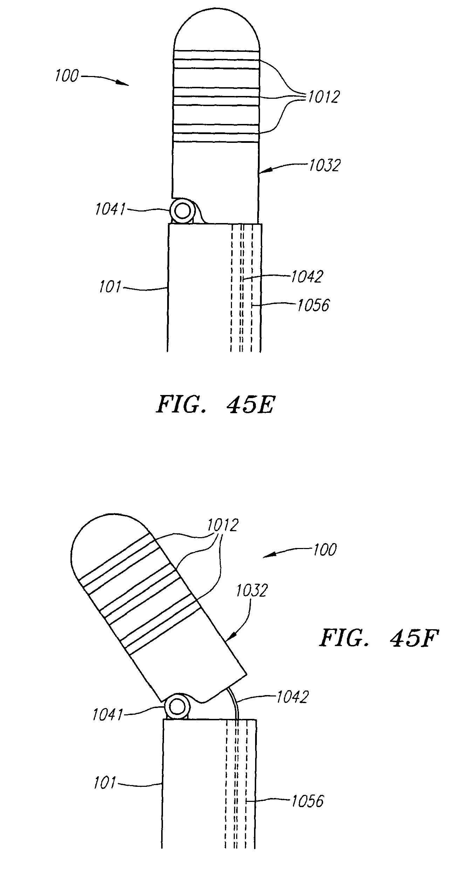

**FIG. 45E** is a top down view depicting an exemplary embodiment of system 100 having a deflectable lower portion 1032. This deflectable lower portion 1032 can be used instead of open portion 1036 to allow passage of needle member 405 and closure device 103. Here lower portion 1032 is pivotably coupled with body member 101 by way of hinge 1041 which is depicted on the left side of this figure. A push/pull wire 1042, slidably located within lumen 1056, is coupled with lower portion 1032 and allows the user to exert control over the position of lower portion 1032. **FIG. 45E** depicts lower portion 1032 in an undeflected state, while **FIG. 45F** depicts lower portion 1032 after it has been deflected about hinge 1041 by exerting a distal force on push/pull wire 1042. A stop (not shown) can be included to stop deflection of portion 1032 at the desired position. Push/pull wire 1042 can also reside external to body member 101 instead of within lumen 1056 in body member 101.

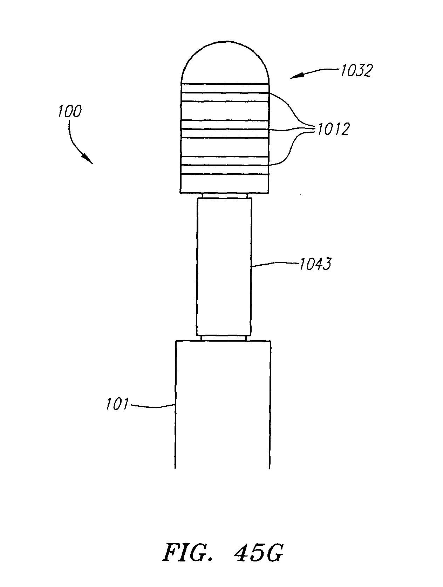

**FIG. 45G** is a top down view depicting lower portion 1032 in an impact-resistant configuration. In this embodiment, the configuration is achieved through the use of a rotatable outer covering, preferably composed of nitinol, stainless steel, or the like. This rotatable portion 1043 is preferably configured to rotate, or spin, if needle member 405 (not shown) were to come into contact with it. In an alternative embodiment, the low friction configuration can be achieved by the use of a static, generally cylindrical, highly polished or otherwise smoothed metallic section in a similar position on lower portion 1032.

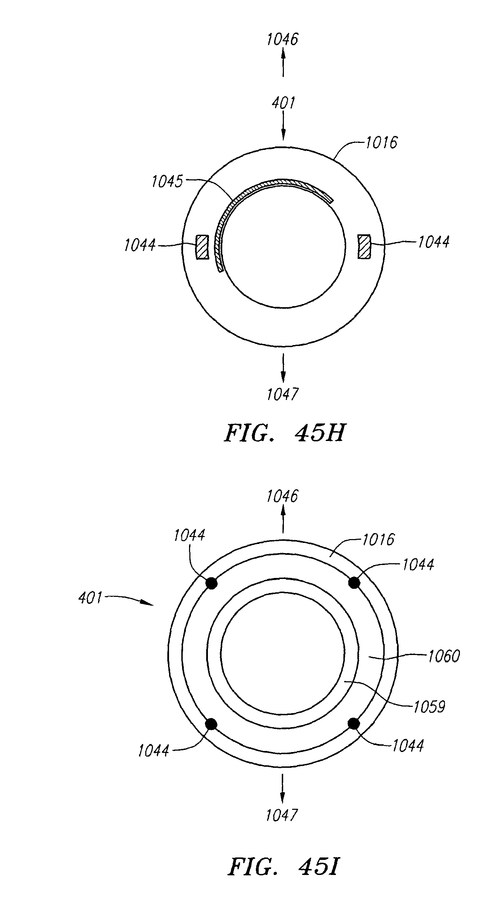

**FIG. 45H** is a radial cross-sectional view taken along lines 45H-45H of **FIG. 45A****.** Shown here is outer tubular sheath 1016 of OA delivery member 401 (the other members of system 100 are not shown for clarity). In this embodiment, outer sheath 1016 includes two reinforcement members 1044 which are disposed longitudinally along the length of sheath 1016, preferably at orientations generally 180 degrees apart. **FIG. 45H** also shows a segment of coil reinforcement 1045. Coil reinforcement 1045 is preferably disposed within sheath 1016 (as shown) or along an inner or outer surface of sheath 1016 and extends in a coiled fashion around the central axis of OA delivery member 401.

Both reinforcement members 1044 and coil reinforcement 1045 can extend along any length of OA delivery member 401 including the entire length, or any portion of the length in which additional reinforcement is desired. Reinforcement members 1044 and coil reinforcement 1045 can be used together or each individually as desired. In addition, any number of one or more reinforcement members 1044 can be used and any number of one or more coil reinforcements 1045 can be used. Reinforcement members 1044 and coil reinforcement 1045 can be made of any desired reinforcing material such as nitinol, stainless steel, cobalt-chrome alloys and the like. Reinforcement can decrease the tendency of sheath 1016 to stretch, can prevent buckling, kinking or other radial distortion when OA delivery member 401 is bent or deflected (such as during off axis delivery), and can provide a high radiopacity.

Also, use of reinforcement members 1044 can increase the tendency of sheath 1016 to deflect in a given direction. For instance, if reinforcement members 1044 are disposed at opposite sides of sheath 1016 as depicted here, sheath 1016 will be more likely to deflect up or down in directions 1046 and 1047 as shown. This can provide benefit during the delivery procedure by increasing the likelihood of OA delivery member 401 to deflect in a desired direction. Furthermore, sheath 1016, if fabricated from certain polymeric materials recognized by those of skill in the art, can exhibit a natural tendency to deflect in a given direction and this natural tendency can be used with reinforcement members 1044 to provide deflection in a desired direction. In addition, some manufacturing processes (e.g., extrusion and the like) can be used to orient the polymeric chains of sheath 1016 advantageously to provide the desired directionality. Furthermore, a relatively thinner portion of sheath 1016, which extends along the length of sheath 1016 in the desired region, can improve the tendency of sheath 1016 to deflect in a particular direction.

**FIG. 45I** is a cross-sectional view depicting another exemplary embodiment of OA delivery member 401. Here, OA deliver member 401 can include at least two, preferably three layers. An inner layer 1059 can be composed of nylon (e.g., nylon 6, nylon 12, etc.) or another friction reducing material (e.g., teflon, polyethylene, etc.). A mid-layer 1060 is preferably configured to resist kinking. In this embodiment, mid-layer 1060 is a braided stainless steel material, although other materials can be used. One exemplary braid is a sixteen wire braid of ribbon or round wire. The braid density can be approximately eighty wire crossovers per inch (PPI), sometimes referred to as the "pic" count. Here, four reinforcement members 1044 are located between layer 1060 and outer sheath 1016. Outer sheath 1016 can be composed of nylon, teflon, polyethylene or the like. It should be noted that if reinforcement members 1044 are placed between layers 1059 and 1060, layer 1016 can be eliminated.

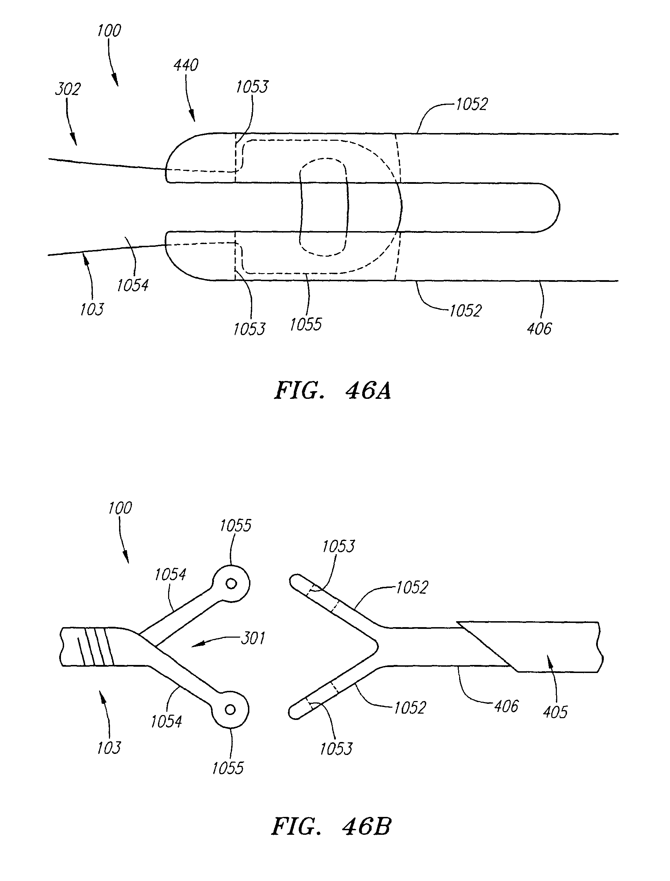

**FIG. 46A** is a side view depicting another exemplary embodiment of system 100. Here, pusher member 406 is shown with closure element 103. Pusher member 406 includes two deflectable members 1052 located on its distal end 440. Deflectable members 1052 are each biased to deflect away from each other. Members 1052 each include an aperture 1053 in which implant 103 is configured to interface. In this embodiment, pusher member 406 is configured to operate with a clip-like embodiment of implant 103, although pusher member 406 is not limited to such. This embodiment of implant 103 includes one or more deflectable arm-like members 1054 on RA portion 301 having relatively larger distal ends 1055. Here, distal ends 1055, apertures 1053 and a portion of arm-like members 1054 are shown with dotted lines to indicate obscurement by members 1052. When located within needle member 405 (not shown), deflectable members 1052 are restrained and maintained in the position shown in **FIG. 46A****.**

**FIG. 46B** is a perspective view depicting pusher member 406 after advancement from needle 405. Here, needle 405 no longer restrains members 1052, which then enter the deflected state shown. Upon deflection, members 1054 of implant 103 are free to enter a deflected state configured to engage the septal wall (not shown). Although not shown, an additional tether can be coupled with implant 103 and used to retrieve implant 103 should such retrieval become desirable at a later stage. In order to maintain a high degree of correspondence between motion of pusher 406 and implant 103, apertures 1053 are preferably configured to engage distal ends 1055 with a relatively snug fit, i.e., the amount of free space between distal ends 1055 and the walls of members 1052 around apertures 1053 is preferably minimized.

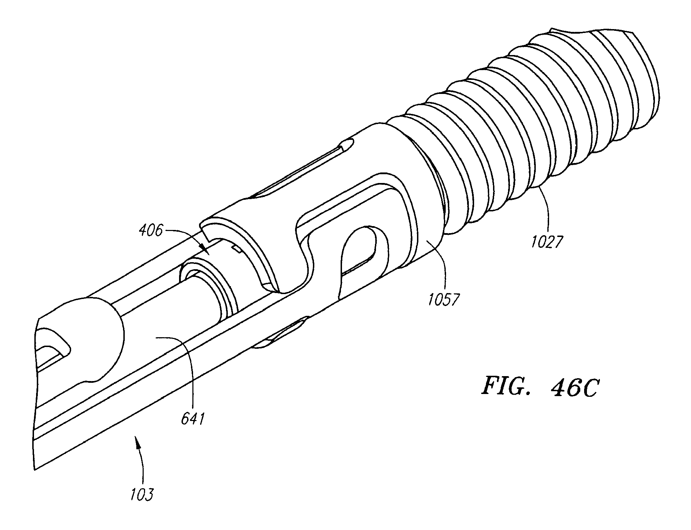

FIG. **46C** is a perspective view depicting another exemplary embodiment of system 100 with pusher member 406 and clip-like implant 103. Here, pusher member 406 includes an interface portion 1057 that is configured to interface with clip 103. Portion 1057 is preferably welded or otherwise fixably coupled with the tube-like body of pusher member 406. Portion 1057 can also be part of a solid wire body of pusher 406. The outer diameter of portion 1057 is preferably sized to fit snugly within the inner diameter of needle 405 (not shown). As can be seen, pusher 406 is configured to engage implant 103 while within needle 405 and can be used to advance implant 103 distally and retract implant 103 proximally as desired, similar to the embodiments described with respect to **FIGs. 20A-B, 44E-F** and **46A-B.**

It should be noted that any feature, function, method or component of any embodiment described with respect to **FIGs. 1-46C** can be used in combination with any other embodiment, whether or not described herein. As one of skill in the art will readily recognize, treatment system 100 and the methods for treating a septal defect can be configured or altered in an almost limitless number of ways, the many combinations and variations of which cannot be practically described herein.

The devices and methods herein may be used in any part of the body, in order to treat a variety of disease states. Of particular interest are applications within hollow organs including but not limited to the heart and blood vessels (arterial and venous), lungs and air passageways, digestive organs (esophagus, stomach, intestines, biliary tree, etc.). The devices and methods will also find use within the genitourinary tract in such areas as the bladder, urethra, ureters, and other areas.

Furthermore, the off-axis delivery systems may be used to pierce tissue and deliver medication, fillers, toxins, and the like in order to offer benefit to a patient. For instance, the device could be used to deliver bulking agent such as collagen, pyrolytic carbon beads, and/or various polymers to the urethra to treat urinary incontinence and other urologic conditions or to the lower esophagus/upper stomach to treat gastroesophageal reflux disease. Alternatively, the devices could be used to deliver drug or other agent to a preferred location or preferred depth within an organ. For example, various medications could be administered into the superficial or deeper areas of the esophagus to treat Barrett's esophagus, or into the heart to promote angiogenesis or myogenesis. Alternatively, the off-axis system can be useful in taking biopsies, both within the lumen and deep into the lumen. For example, the system could be used to take bronchoscopic biopsy specimens of lymph nodes that are located outside of the bronchial tree or flexible endoscopic biopsy specimens that are located outside the gastrointestinal tract. The above list is not meant to limit the scope of the invention.

In some embodiments, the off-axis delivery system is used with an anchoring means in order to anchor the device to a location within the body prior to rotation of the off-axis system. This anchoring means may involve the use of a tissue grasper or forceps. It should be noted that any device or set of devices can be advanced within the lumen of the off-axis delivery system, including but not limited to needles, biopsy forceps, aspiration catheters, drug infusion devices, brushes, stents, balloon catheters, drainage catheters, and the like.

While the invention is susceptible to various modifications and alternative forms, a specific example thereof has been shown in the drawings and is herein described in detail. It should be understood, however, that the invention is not to be limited to the particular form disclosed, but to the contrary, the invention is to cover all modifications, equivalents, and alternatives falling within the spirit of the disclosure.

## Claims

1. A system for treating a septal defect, comprising:
a proximal control device configured to be directly accessible by a user and configured to control each of an elongate delivery member, an elongate needle member configured to penetrate a septal wall and an elongate pusher member configured to abut an implantable septal defect closure device, the proximal control device comprising:
a rotatable element; and
a rotatable guide structure coupled with the rotatable element, the rotatable guide structure configured to guide movement of at least one of the delivery member, needle member and pusher members.

2. The system of claim 1, wherein the rotatable guide structure is configured to guide movement of each of the delivery member, needle member and pusher member.

3. The system of claim 2, wherein the proximal controller is configured to translate rotation of the rotatable element in a first direction into movement of the delivery member, needle member and pusher member.

4. The system of claim 3, wherein the rotatable element is rotatable in the first direction and a second direction opposite the first.

5. The system of claim 4, wherein the proximal controller is configured such that rotation of the rotatable element in the first direction by a first amount engages a safeguard device configured to prevent rotation of the rotatable element in the second direction.

6. The system of claim 5, wherein the safeguard device comprises a ratchet and an abutment configured to interface with the ratchet.

7. The system of claim 3, wherein rotation of the rotatable element in only the first direction is configured to move the delivery member, needle member and pusher member through the steps for at least partial deployment of the implantable septal defect closure device.

8. The system of claim 2, wherein the proximal control device further comprises:
a delivery member actuator coupled with a proximal end of the delivery member;
a needle member actuator coupled with a proximal end of the needle member; and
a pusher member actuator coupled with a proximal end of the pusher member, wherein each of the actuators is coupled with the rotatable guide structure.

9. The system of claim 8, wherein each of the delivery member actuator, needle member actuator and pusher member actuator are slidably coupled with a guide rail.

10. The system of claim 8, wherein the delivery member actuator, needle member actuator and pusher member actuator each comprise an interface configured to interface with a slot in the rotatable guide structure.

11. The system of claim 10, wherein each interface is configured as a rotatable wheel or bearing configured to rotate along an edge of the slot in the rotatable guide structure.

12. The system of claim 10, wherein the rotatable guide structure comprises a separate slot corresponding to each of the delivery member actuator, needle member actuator and pusher member actuators.

13. The system of claim 12, wherein the slots are oriented such that rotation of the rotatable guide structure in a first direction allows proximal and distal movement of at least one of the actuators.

14. The system of claim 12, wherein the controller is configured such that rotation of the rotatable guide structure forces the interface of each actuator to move within the corresponding slot, the movement of the interface corresponding to longitudinal movement of the respective actuator.

15. The system of claim 14, wherein the slots are oriented such that rotation of the rotatable guide structure in the first direction moves at least one actuator while not moving at least one, different actuator.

16. The system of claim 14, wherein the slots are oriented such that rotation of the rotatable guide structure in the first direction moves at least one actuator by a first longitudinal distance and moves a second actuator by a second longitudinal distance different than the first longitudinal distance.

17. The system of claim 8, further comprising a housing, wherein the rotatable guide structure and actuators are contained within the housing.

18. The system of claim 2, wherein the rotatable element is configured to be rotated manually by the user.

19. The system of claim 2, wherein the rotatable element is configured to be rotated automatically.
